# EUROPEAN PATENT SPECIFICATION

(11) **EP 2 080 140 B1**
(45) Date of publication and mention of the grant of the patent: **24.04.2013**
(21) Application number: 07871360.9
(22) Date of filing: 03.11.2007
(51) Int. Cl.: G06F 19/00

(54) **DIAGNOSIS OF METASTATIC MELANOMA AND MONITORING INDICATORS OF IMMUNOSUPPRESSION THROUGH BLOOD LEUKOCYTE MICROARRAY ANALYSIS**
DIAGNOSE VON METASTASIERENDEM MELANOM UND ÜBERWACHUNG VON IMMUNSUPPRESSIONSINDIKATOREN MITTTELS LEUKOZYTEN-MIKROARRAY
DIAGNOSTIC DE MELANOME METASTATIQUE ET SURVEILLANCE D'INDICATEURS D'IMMUNOSUPPRESSION PAR ANALYSE DE MICRORESEAUX DE LEUCOCYTES SANGUINS

(30) Priority: 03.11.2006 US 856406 P
(43) Date of publication of application: 22.07.2009
(60) Divisional application: 12152482.1
(73) Proprietor: Baylor Research Institute, Dallas, TX 75204 (US)
(72) Inventor: PALUCKA, Anna Karolina, Dallas, TX 75204 (US); BANCHEREAU, Jacques F., Dallas, TX 75230 (US); CHAUSSABEL, Damien, Richardson, TX 75082 (US)
(74) Representative: Sonn & Partner Patentanwälte
(86) International application number: PCT/US2007/083555
(87) International publication number: WO 2008/100352

(56) References cited:
- US-A1- 2005 222 029
- PAVEY SANDRA ET AL: "Microarray expression profiling in melanoma reveals a BRAF mutation signature", ONCOGENE, vol. 23, no. 23, 20 May 2004 (2004-05-20), pages 4060-4067, XP002644753, ISSN: 0950-9232
- ZHOU YOUWEN ET AL: "Osteopontin expression correlates with melanoma invasion", JOURNAL OF INVESTIGATIVE DERMATOLOGY, vol. 124, no. 5, May 2005 (2005-05), pages 1044-1052, XP002644754, ISSN: 0022-202X
- BITTNER M ET AL: "MOLECULAR CLASSIFICATION OF CUTANEOUS MALIGNANT MELANOMA BY GENE EXPRESSION PROFILING", NATURE, NATURE PUBLISHING GROUP, LONDON, GB, vol. 406, no. 6795, 3 August 2000 (2000-08-03), pages 536-540, XP000990000, ISSN: 0028-0836, DOI: DOI:10.1038/35020115
- CARR ET AL.: 'Gene-expression profiling in human cutaneous melanoma' ONCOGENE vol. 22, 2003, pages 3073 - 3080, XP008109734
- DAVIES ET AL.: 'Mutations of the BRAF gene in human cancer' NATURE vol. 417, June 2002, pages 949 - 954, XP001188240
- SHU ET AL.: 'RNCP1, an RNA-binding protein and a target of the p53 family, is required for maintaining the stability of the basal and stress-induced p21 transcript' GENES AND DEVELOPMENT vol. 20, 2006, pages 2961 - 2972, XP008109736
- ZHANG ET AL.: 'Identification of Direct Serum-response Factor Gene Targets during Me2So-induced P19 Cardiac Cell Differentiation' JOURNAL OF BIOLOGICAL CHEMISTRY vol. 280, May 2005, pages 19115 - 19126, XP008109738

## Description

### TECHNICAL FIELD OF THE INVENTION

The present invention relates in general to the field of diagnostic for monitoring indicators of metastatic melanoma and/or immunosuppression, and more particularly, to a system, method and apparatus for the diagnosis, prognosis and tracking of metastatic melanoma and monitoring indicators of immunosuppression associated with transplant recipients (e.g., liver).

### BACKGROUND OF THE INVENTION

Pharmacological immunosuppression has been instrumental in transplantation, transforming a last resort experimental procedure into a routinely successful one. Cyclosporin and tacrolimus/FK506 currently constitute the mainstay for transplant recipients. Activated T cells have been considered the main cellular targets for immunosuppressive treatments, but recent reports have also noted a marked effect of these drugs on antigen presenting cells, probably contributing further to the establishment of a generalized state of immune unresponsiveness (Lee et al., 2005b; Woltman et al., 2003). While severe immunosuppression generated by pharmacological treatment is necessary for the maintenance of graft survival and function, it also exposes the recipient to life-threatening infections and malignancies. Skin cancer is a well-established complication in organ transplant recipients (Gerlini et al., 2005). A recent epidemiological study showed that nearly 20% of 1115 renal transplant patients developed skin malignancies in Europe (Bordea et al., 2004), with much higher incidences being observed in less temperate climates, e.g., as high as 28% in Australia (Carroll et al., 2003). In addition to occurring more often, skin malignancies tend to take a more aggressive clinical course in transplanted patients, with a higher propensity to metastasize distantly and lead to a fatal outcome (Barrett et al., 1993).

Tumors maintain their survival by compromising the immune system. Different mechanisms have been identified including secretion of immunosuppressive factors such as cytokines (e.g., IL-10, TGF-beta), hormones (e.g., Prostaglandin E2), and others (e.g., MIA: Melanoma inhibitory activity, Tenascin C)(Jachimczak et al., 2005; Puente Navazo et al., 2001). Furthermore, tumors might promote development of suppressor T cells (Liyanage et al., 2002; Viguier et al., 2004), possibly via modulating dendritic cells (Gabrilovich, 2004; Lee et al., 2005a; Monti et al., 2004).

Thus, immunosuppression, whether from tumors or pharmacological treatments, has been linked to cancer progression. Therefore, a common molecular signature could be identified by profiling genome-wide transcriptional activity in peripheral blood mononuclear cell ("PBMC") samples obtained from immunosuppressed patients with either metastatic melanoma or liver allografts. The analysis of Leukocyte transcriptional profiles generated in the context of the present study supports this notion and identifies a blood signature of immunosuppression.

In Pavey S et al. (Oncogene 23(2004): 4060-4067) a microarray is described which can be used to predict the presence of BRAF mutations in a panel of 61 melanoma cell lines.

Zhou Y et al. (J Invest Dermatol 124(2005): 1044-1052) describe the overexpression of over 190 genes in metastatic melanomas. Thereby they noted that one of the most abundantly expressed genes in metastatic melanoma modules is osteopontin.

In Bittner M et al. (Nature 406(2000): 536-540) gene expression profiling was used to clarify cutaneous malignant melanomas.

### SUMMARY OF THE INVENTION

Genomic research is facing significant challenges with the analysis of transcriptional data that are notoriously noisy, difficult to interpret and do not compare well across laboratories and platforms. The present inventors have developed an analytical strategy emphasizing the selection of biologically relevant genes at an early stage of the analysis, which are consolidated into analytical modules that overcome the inconsistencies among microarray platforms. The transcriptional modules developed may be used for the analysis of large gene expression datasets. The results derived from this analysis are easily interpretable and particularly robust, as demonstrated by the high degree of reproducibility observed across commercial microarray platforms.

Applications for this analytical process are illustrated through the mining of a large set of PBMC transcriptional profiles. Twenty-eight transcriptional modules regrouping 4,742 genes were identified. Using the present invention is it possible to demonstrate that diseases are uniquely characterized by combinations of transcriptional changes in, e.g., blood leukocytes, measured at the modular level. Indeed, module-level changes in blood leukocytes transcriptional levels constitute the molecular fingerprint of a disease or sample.

This invention has a broad range of applications. It can be used to characterize modular transcriptional components of any biological system (e.g., peripheral blood mononuclear cells (PBMCs), blood cells, fecal cells, peritoneal cells, solid organ biopsies, resected tumors, primary cells, cells lines, cell clones, etc.). Modular PBMC transcriptional data generated through this approach can be used for molecular diagnostic, prognostic, assessment of disease severity, response to drug treatment, drug toxicity, etc. Other data processed using this approach can be employed for instance in mechanistic studies, or screening of drug compounds. In fact, the data analysis strategy and mining algorithm can be implemented in generic gene expression data analysis software and may even be used to discover, develop and test new, disease- or condition-specific modules. The present invention may also be used in conjunction with pharmacogenomics, molecular diagnostic, bioinformatics and the like, wherein in-depth expression data may be used to improve the results (e.g., by improving or sub-selecting from within the sample population) that mat be obtained during clinical trails.

More particularly, the present invention includes methods for diagnosing a disease or condition by obtaining the transcriptome of a patient; analyzing the transcriptome based on one or more transcriptional modules that are indicative of a disease or condition; and determining the patient's disease or condition based on the presence, absence or level of expression of genes within the transcriptome in the one or more transcriptional modules. The transcriptional modules may be obtained by: iteratively selecting gene expression values for one or more transcriptional modules by: selecting for the module the genes from each cluster that match in every disease or condition; removing the selected genes from the analysis; and repeating the process of gene expression value selection for genes that cluster in a sub-fraction of the diseases or conditions; and iteratively repeating the generation of modules for each clusters until all gene clusters are exhausted.

Examples of clusters selected for use with the present invention include, but are not limited to, expression value clusters, keyword clusters, metabolic clusters, disease clusters, infection clusters, transplantation clusters, signaling clusters, transcriptional clusters, replication clusters, cell-cycle clusters, siRNA clusters, miRNA clusters, mitochondrial clusters, T cell clusters, B cell clusters, cytokine clusters, lymphokine clusters, heat shock clusters and combinations thereof. Examples of diseases or conditions for analysis using the present invention include, e.g., autoimmune disease, a viral infection a bacterial infection, cancer and transplant rejection. More particularly, diseases for analysis may be selected from one or more of the following conditions: systemic juvenile idiopathic arthritis, systemic lupus erythematosus, type I diabetes, liver transplant recipients, melanoma patients, and patients bacterial infections such as *Escherichia coli, Staphylococcus aureus,* viral infections such as influenza A, and combinations thereof. Specific array may even be made that detect specific diseases or conditions associated with a bioterror agent.

Cells that may be analyzed using the present invention, include, e.g., peripheral blood mononuclear cells (PBMCs), blood cells, fetal cells, peritoneal cells, solid organ biopsies, resected tumors, primary cells, cells lines, cell clones and combinations thereof. The cells may be single cells, a collection of cells, tissue, cell culture, cells in bodily fluid, e.g., blood. Cells may be obtained from a tissue biopsy, one or more sorted cell populations, cell culture, cell clones, transformed cells, biopies or a single cell. The types of cells may be, e.g., brain, liver, heart, kidney, lung, spleen, retina, bone, neural, lymph node, endocrine gland, reproductive organ, blood, nerve, vascular tissue, and olfactory epithelium cells. After cells are isolated, these mRNA from these cells is obtained and individual gene expression level analysis is performed using, e.g., a probe array, PCR, quantitative PCR, bead-based assays and combinations thereof. The individual gene expression level analysis may even be performed using hybridization of nucleic acids on a solid support using cDNA made from mRNA collected from the cells as a template for reverse transcriptase.

Pharmacological immunosuppression promotes graft survival in transplant recipients. Endogenous immunosuppression promotes tumor survival in cancer-bearing patients. Leukocytes from patients with metastatic melanoma display an endogenous immunosuppression signature common with liver transplant recipients under pharmacological immunosuppression. Blood microarray analyses were carried out in 25 healthy volunteers, 35 patients with metastatic melanoma, and 39 liver transplant recipients. Disease signatures were identified, and confirmed in an independent dataset, in comparison to healthy controls. Analysis of a set of 69 transcripts over-expressed preferentially in melanoma and transplant groups in comparison to six other diseases revealed remarkable functional convergence, including several repressors of interleukin-2 transcription, powerful inhibitors of NF-kappaB and MAPK pathways as well as antiproliferative molecules. Thus, patients with metastatic melanoma display an endogenous transcriptional signature of immunosuppression. This signature may now be used to identify patients at the high risk of metastatic melanoma progression.

The present invention includes a system and a method to analyze samples for the prognosis and diagnosis of metastatic melanoma and/or monitoring indicators of immunosuppression associated with transplant recipients (e.g., liver) using multiple variable gene expression analysis. The gene expression differences that remain can be attributed with a high degree of confidence to the unmatched variation. The gene expression differences thus identified can be used, for example, to diagnose disease, identify physiological state, design drugs and monitor therapies.

The sample may be screened by quantitating the mRNA, protein or both mRNA and protein level of the expression vector. When the screening is for mRNA levels, it may be quantitated by a method selected from polymerase chain reaction, real time polymerase chain reaction, reverse transcriptase polymerase chain reaction, hybridization, probe hybridization, and gene expression array. The screening method may also include detection of polymorphisms in the biomarker. Alternatively, the screening step may be accomplished using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing. For use with the present invention the sample may be any of a number of immune cells, e.g., leukocytes or sub-components thereof.

The present invention relates to a method of identifying a patient with melanoma by examining the phenotype a sample for combinations of six, seven, eight, ten, fifteen, twenty, twenty-five or more genes selected from modules M1.1 and M1.2 of Table 12.

In one aspect, the present invention includes a method of identifying gene expression response to pharmacological immunosuppression in transplant recipients by examining the phenotype a sample for combinations of six, seven, eight, ten, fifteen, twenty, twenty-five or more genes selected from modules M1.1 and M1.2 of Table 13.

The sample may be screened by quantitating the mRNA, protein or both mRNA and protein level of the expression vector. When mRNA level is examined, it may be quantitated by a method selected from polymerase chain reaction, real time polymerase chain reaction, reverse transcriptase polymerase chain reaction, hybridization, probe hybridization, and gene expression array. The screening method may also include detection of polymorphisms in the biomarker. Alternatively, the screening step may be accomplished using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing. For use with the present invention the sample may be any of a number of immune cells, e.g., leukocytes or sub-components thereof.

The expression vector may be screened by quantitating the mRNA, protein or both mRNA and protein level of the expression vector. When the expression vector is mRNA level, it may be quantitated by a method selected from polymerase chain reaction, real time polymerase chain reaction, reverse transcriptase polymerase chain reaction, hybridization, probe hybridization, and gene expression array. The screening method may also include detection of polymorphisms in the biomarker. Alternatively, the screening step may be accomplished using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing. For use with the present invention the sample may be any of a number of immune cells, e.g., leukocytes or sub-components thereof.

For example, a method of identifying a subject with melanoma by determining a database that includes the level of expression of one or more metastatic melanoma expression vectors. Another embodiment of the present invention includes a computer implemented method for determining the genotype of a sample by obtaining a plurality of sample probe intensities. Diagnosing metastatic melanoma is based upon the sample probe intensities and calculating a linear correlation coefficient between the sample probe intensities and reference probe intensities.

The present invention also includes a computer readable medium including computer-executable instructions for performing the method of determining the genotype of a sample. The method of determining the phenotype includes obtaining a plurality of sample probe intensities and diagnosing melanoma based upon the sample probe intensities for two or more metastatic melanoma expression vectors selected from those listed in modules M1.1 and M1.2 of Table 12 into a dataset; and calculating a linear correlation coefficient between the sample probe intensities and a reference probe intensity. The tentative phenotype is accepted as the phenotype of the sample if the linear correlation coefficient is greater than a threshold value. In addition, the present invention includes a microarray for identifying a human subject with melanoma. A microarray includes the detection of expression of two or more metastatic melanoma genes listed in modules M1.1 and M1.2 of Table 12 into a dataset. The present invention provides a method of distinguishing between metastatic melanoma and immunosuppression associated with transplants by determining the level of expression of one or more genes, and calculating one or more gene expression vectors. The melanoma-specific transcriptome-expression vectors may include values for the upregulation or downregulation of six or more genes listed in modules M1.1 and M1.2 of Table 12. The present invention provides a method of identifying a subject with immunosuppression associated with transplants by determining the level of expression of one or more immunosuppression associated expression vectors. The immunosuppression-specific transcriptome-expression vectors may include values for the upregulation or downregulation of six or more genes listed in modules M1.1 and M1.2 of Table 13.

A computer readable medium is also included that has computer-executable instructions for performing the method for determining the phenotype of a sample. The method for determining the phenotype of a sample includes obtaining a plurality of sample probe intensities and diagnosing immunosuppression based upon the sample probe intensities for two or more immunosuppression associated expression vectors. A linear correlation coefficient is calculated between the sample probe intensities and a reference probe intensity and a tentative phenotype is accepted as the phenotype of the sample if the linear correlation coefficient is greater than a threshold value. The present invention also includes a system for diagnosing immunosuppression including an expression level detector for determining the expression level of two or more immunosuppression expression vectors selected from the 1, 2, 3, 4, 5, 6, 8, 10, 15, 20, 25 or more genes. The melanoma-specific transcriptome-expression vectors that are used to generate expression data for each gene, which is saved into a dataset, may include values for the upregulation or downregulation of six or more genes listed in modules M1.1 and M1.2 of Table 12. The immunosuppression-specific transcriptome-expression vectors may include values in a dataset that includes the upregulation or downregulation of six or more genes listed in modules M1.1 and M1.2 of Table 13. The presence of melanoma or immunosuppression is determined based on the presence of two or more positive indicators in a gene expression vector dataset.

The arrays, methods and systems of the present invention may even be used to select patients for a clinical trial by obtaining the transcriptome of a prospective patient; comparing the transcriptome to one or more transcriptional modules that are indicative of a disease or condition that is to be treated in the clinical trial; and determining the likelihood that a patient is a good candidate for the clinical trial based on the presence, absence or level of one or more genes that are expressed in the patient's transcriptome within one or more transcriptional modules that are correlated with success in a clinical trial. Generally, for each module a vector that correlates with a sum of the proportion of transcripts in a sample may be used, e.g., when each module includes a vector and wherein one or more diseases or conditions is associated with the one or more vectors. Therefore, each module may include a vector that correlates to the expression level of one or more genes within each module.

Herein arrays, e.g., custom microarrays are described that include nucleic acid probes immobilized on a solid support that includes sufficient probes from one or more expression vectors to provide a sufficient proportion of differentially expressed genes to distinguish between one or more diseases. For example, an array of nucleic acid probes immobilized on a solid support, in which the array includes at least two sets of probe modules, wherein the probes in the first probe set have one or more interrogation positions respectively corresponding to one or more diseases. The array may have between 100 and 100,000 probes, and each probe may be, e.g., 9-21 nucleotides long. When separated into organized probe sets, these may be interrogated separately.

One or more nucleic acid probes immobilized on a solid support to form a module array are also described that includes at least one pair of first and second probe groups, each group having one or more probes as defined by Table 1. The probe groups are selected to provide a composite transcriptional marker vector that is consistent across microarray platforms. In fact, the probe groups may even be used to provide a composite transcriptional marker vector that is consistent across microarray platforms and displayed in a summary for regulatory approval. The skilled artisan will appreciate that using the modules of the present invention it is possible to rapidly develop one or more disease specific arrays that may be used to rapidly diagnose or distinguish between different disease and/or conditions.

A method for displaying transcriptome vector data from a transcriptome vector dataset by separating one or more genes into one or more modules to visually display an aggregate gene expression vector value for each of the modules; and displaying the aggregate gene expression vector value for overexpression, underexpression or equal expression of the aggregate gene expression vector value in each module is also described. In one example, overexpression is identified with a first identifier and underexpression is identified with a second identifier. Examples of identifiers include colors, shapes, patterns, light/dark, on/off, symbols and combinations thereof. For example, overexpression is identified with a first identifier and underexpression is identified with a second identifier, wherein the first identifier is a first color and the second identifier is a second color, and wherein first and second identifiers are superimposed to provide a combined color.

### BRIEF DESCRIPTION OF THE DRAWINGS

For a more complete understanding of the features and advantages of the present invention, reference is now made to the detailed description of the invention along with the accompanying figures and in which:
FIGURES 1A to 1C show the basic microarray data mining strategy steps involved in accepted gene-level microarray data analysis (FIGURE 1A), the modular mining strategy of the present invention FIGURE 1B and a full size representation of the module extraction algorithm FIGURE 1C to generate on or more datasets used to create the expression vectors;
FIGURE 2 is a graph representing transcriptional profiles showing levels of modular gene expression profiles across an independent group of samples;
FIGURE 3 is a distribution of keyword occurrence in the literature obtained for four sets of coordinately expressed genes;
FIGURE 4 illustrates a modular microarray analysis strategy for characterization of the transcriptional system;
FIGURE 5 is an analysis of patient blood leukocyte transcriptional profiles;
FIGURE 6 illustrates module maps of transcriptional changes caused by disease;
FIGURE 7 illustrates the identification of a blood leukocyte transcriptional signature in patients with metastatic melanoma;
FIGURE 8 illustrates the validation of microarray results in an independent set of samples;
FIGURE 9 illustrates the identification of a blood leukocyte transcriptional signature in transplant recipients under immunosuppressive drug therapy
FIGURE 10-13 illustrate detailed results of the module-level analysis;
FIGURE 14 illustrates the module-level analysis for distinctive transcriptional signatures in blood from patients with metastatic melanoma and from liver transplant recipients;
FIGURE 15 illustrates the mapping transcriptional changes in patient blood leukocytes at the module level;
FIGURE 16 illustrates the module-level analysis for common transcriptional signatures in blood from patients with metastatic melanoma and from liver transplant recipients;
FIGURE 17 illustrates the analysis of significance patterns with genes expressed at higher levels in both melanoma and liver transplant patients compared to healthy volunteers;
FIGURE 18 illustrates the modular distribution of ubiquitous and specific gene signatures common to melanoma and transplant groups;
FIGURE 19 illustrates the transcriptional signature of immunosuppression;
FIGURE 20 shows a statistical group comparison between patients and their respective controls;
FIGURE 21 shows the analysis of significance patterns for genes over-expressed in SLE patients but not in patients with acute Influenza A infection;
FIGURE 22 shows the patterns of significance for genes common to Influenza A and SLE; and
FIGURE 23 is a functional analysis of genes shared by patients with Influenza infection and Lupus grouped according to significance patterns.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims. Unless defined otherwise, all technical and scientific terms used herein have the meaning commonly understood by a person skilled in the art to which this invention belongs. The following references provide one of skill with a general definition of many of the terms used in this invention: Singleton, et al., Dictionary Of Microbiology And Molecular Biology (2d ed. 1994); The Cambridge Dictionary Of Science And Technology (Walker ed., 1988); The Glossary Of Genetics, 5th Ed., R. Rieger et al. (eds.), Springer Verlag (1991); and Hale & Marham, The Harper Collins Dictionary Of Biology (1991).

Various biochemical and molecular biology methods are well known in the art. For example, methods of isolation and purification of nucleic acids are described in detail in WO 97/10365, WO 97/27317, Chapter 3 of Laboratory Techniques in Biochemistry and Molecular Biology: Hybridization With Nucleic Acid Probes, Part I. Theory and Nucleic Acid Preparation, (P. Tijssen, ed.) Elsevier, N.Y. (1993); Chapter 3 of Laboratory Techniques in Biochemistry and Molecular Biology: Hybridization With Nucleic Acid Probes, Part 1. Theory and Nucleic Acid Preparation, (P. Tijssen, ed.) Elsevier, N.Y. (1993); and Sambrook et al., Molecular Cloning: A Laboratory Manual, Cold Spring Harbor Press, N.Y., (1989); and Current Protocols in Molecular Biology, (Ausubel, F. M. et al., eds.) John Wiley & Sons, Inc., New York (1987-1999), including supplements such as supplement 46 (April 1999).

### BIOINFORMATICS DEFINITIONS

As used herein, an "object" refers to any item or information of interest (generally textual, including noun, verb, adjective, adverb, phrase, sentence, symbol, numeric characters, etc.). Therefore, an object is anything that can form a relationship and anything that can be obtained, identified, and/or searched from a source. "Objects" include, but are not limited to, an entity of interest such as gene, protein, disease, phenotype, mechanism, drug, etc. In some aspects, an object may be data, as further described below.

As used herein, a "relationship" refers to the co-occurrence of objects within the same unit (e.g., a phrase, sentence, two or more lines of text, a paragraph, a section of a webpage, a page, a magazine, paper, book, etc.). It may be text, symbols, numbers and combinations, thereof

As used herein, "meta data content" refers to information as to the organization of text in a data source. Meta data can comprise standard metadata such as Dublin Core metadata or can be collection-specific. Examples of metadata formats include, but are not limited to, Machine Readable Catalog (MARC) records used for library catalogs, Resource Description Format (RDF) and the Extensible Markup Language (XML). Meta objects may be generated manually or through automated information extraction algorithms.

As used herein, an "engine" refers to a program that performs a core or essential function for other programs. For example, an engine may be a central program in an operating system or application program that coordinates the overall operation of other programs. The term "engine" may also refer to a program containing an algorithm that can be changed. For example, a knowledge discovery engine may be designed so that its approach to identifying relationships can be changed to reflect new rules of identifying and ranking relationships.

As used herein, "semantic analysis" refers to the identification of relationships between words that represent similar concepts, e.g., though suffix removal or stemming or by employing a thesaurus. "Statistical analysis" refers to a technique based on counting the number of occurrences of each term (word, word root, word stem, n-gram, phrase, etc.). In collections unrestricted as to subject, the same phrase used in different contexts may represent different concepts. Statistical analysis of phrase co-occurrence can help to resolve word sense ambiguity. "Syntactic analysis" can be used to further decrease ambiguity by part-of-speech analysis. As used herein, one or more of such analyses are referred to more generally as "lexical analysis." "Artificial intelligence (AI)" refers to methods by which a non-human device, such as a computer, performs tasks that humans would deem noteworthy or "intelligent." Examples include identifying pictures, understanding spoken words or written text, and solving problems.

As used herein, the term "database" or "dataset" refer to repositories for raw or compiled data, even if various informational facets can be found within the data fields. A database is typically organized so its contents can be accessed, managed, and updated (e.g., the database is dynamic). The term "database" and "source" are also used interchangeably in the present invention, because primary sources of data and information are databases. However, a "source database" or "source data" refers in general to data, e.g., unstructured text and/or structured data, which are input into the system for identifying objects and determining relationships. A source database may or may not be a relational database. However, a system database usually includes a relational database or some equivalent type of database or dataset which stores or includes stored values relating to relationships between objects.

As used herein, a "system database" and "relational database" are used interchangeably and refer to one or more collections of data organized as a set of tables containing data fitted into predefined categories. For example, a database table may comprise one or more categories defined by columns (e.g. attributes), while rows of the database may contain a unique object for the categories defined by the columns. Thus, an object such as the identity of a gene might have columns for its presence, absence and/or level of expression of the gene. A row of a relational database may also be referred to as a "set" and is generally defined by the values of its columns. A "domain" in the context of a relational database is a range of valid values a field such as a column may include.

As used herein, a "domain of knowledge" refers to an area of study over which the system is operative, for example, all biomedical data. It should be pointed out that there is advantage to combining data from several domains, for example, biomedical data and engineering data, for this diverse data can sometimes link things that cannot be put together for a normal person that is only familiar with one area or research/study (one domain). A "distributed database" refers to a database that may be dispersed or replicated among different points in a network.

Terms such "data" and "information" are often used interchangeably, as are "information" and "knowledge." As used herein, "data" is the most fundamental unit that is an empirical measurement or set of measurements. Data is compiled to contribute to information, but it is fundamentally independent of it. Information, by contrast, is derived from interests, e.g., data (the unit) may be gathered on ethnicity, gender, height, weight and diet for the purpose of finding variables correlated with risk of cardiovascular disease. However, the same data could be used to develop a formula or to create "information" about dietary preferences, i.e., likelihood that certain products in a supermarket have a higher likelihood of selling.

As used herein, "information" refers to a data set that may include numbers, letters, sets of numbers, sets of letters, or conclusions resulting or derived from a set of data. "Data" is then a measurement or statistic and the fundamental unit of information. "Information" may also include other types of data such as words, symbols, text, such as unstructured free text, code, etc. "Knowledge" is loosely defined as a set of information that gives sufficient understanding of a system to model cause and effect. To extend the previous example, information on demographics, gender and prior purchases may be used to develop a regional marketing strategy for food sales while information on nationality could be used by buyers as a guideline for importation of products. It is important to note that there are no strict boundaries between data, information, and knowledge; the three terms are, at times, considered to be equivalent. In general, data comes from examining, information comes from correlating, and knowledge comes from modeling.

As used herein, "a program" or "computer program" refers generally to a syntactic unit that conforms to the rules of a particular programming language and that is composed of declarations and statements or instructions, divisible into, "code segments" needed to solve or execute a certain function, task, or problem. A programming language is generally an artificial language for expressing programs.

As used herein, a "system" or a "computer system" generally refers to one or more computers, peripheral equipment, and software that perform data processing. A "user" or "system operator" in general includes a person, that uses a computer network accessed through a "user device" (e.g., a computer, a wireless device, etc) for the purpose of data processing and information exchange. A "computer" is generally a functional unit that can perform substantial computations, including numerous arithmetic operations and logic operations without human intervention.

As used herein, "application software" or an "application program" refers generally to software or a program that is specific to the solution of an application problem. An "application problem" is generally a problem submitted by an end user and requiring information processing for its solution.

As used herein, a "natural language" refers to a language whose rules are based on current usage without being specifically prescribed, e.g., English, Spanish or Chinese. As used herein, an "artificial language" refers to a language whose rules are explicitly established prior to its use, e.g., computer-programming languages such as C, C++, Java, BASIC, FORTRAN, or COBOL.

As used herein, "statistical relevance" refers to using one or more of the ranking schemes (O/E ratio, strength, etc.), where a relationship is determined to be statistically relevant if it occurs significantly more frequently than would be expected by random chance.

As used herein, the terms "coordinately regulated genes" or "transcriptional modules" are used interchangeably to refer to grouped, gene expression profiles (e.g., signal values associated with a specific gene sequence) of specific genes. Each transcriptional module correlates two key pieces of data, a literature search portion and actual empirical gene expression value data obtained from a gene microarray. The set of genes that is selected into a transcriptional module based on the analysis of gene expression data (using the module extraction algorithm described above). Additional steps are taught by Chaussabel, D. & Sher, A. Mining microarray expression data by literature profiling. Genome Biol 3, RESEARCH0055 (2002), (http://genomebiology.com/2002/3/10/research/0055) and expression data obtained from a disease or condition of interest, e.g., Systemic Lupus erythematosus, arthritis, lymphoma, carcinoma, melanoma, acute infection, autoimmune disorders, autoinflammatory disorders, etc.).

The Table below lists examples of keywords that were used to develop the literature search portion or contribution to the transcription modules. The skilled artisan will recognize that other terms may easily be selected for other conditions, e.g., specific cancers, specific infectious disease, transplantation, etc. For example, genes and signals for those genes associated with T cell activation are described hereinbelow as Module ID "M 2.8" in which certain keywords (e.g., Lymphoma, T-cell, CD4, CD8, TCR, Thymus, Lymphoid, IL2) were used to identify key T-cell associated genes, e.g., T-cell surface markers (CD5, CD6, CD7, CD26, CD28, CD96); molecules expressed by lymphoid lineage cells (lymphotoxin beta, IL2-inducible T-cell kinase, TCF7; and T-cell differentiation protein mal, GATA3, STAT5B). Next, the complete module is developed by correlating data from a patient population for these genes (regardless of platform, presence/absence and/or up or downregulation) to generate the transcriptional module. In some cases, the gene profile does not match (at this time) any particular clustering of genes for these disease conditions and data, however, certain physiological pathways (e.g., cAMP signaling, zinc-finger proteins, cell surface markers, etc.) are found within the "Underdetermined" modules. In fact, the gene expression data set may be used to extract genes that have coordinated expression prior to matching to the keyword search, i.e., either data set may be correlated prior to cross-referencing with the second data set.

**Table 1. Examples of Genes within Distinct Modules**

| **Module I.D.** | **Number of probe sets** | **Keyword selection** | **Assessment** |
|---|---|---|---|
| M 1.1 | 76 | Ig, Immunoglobulin, Bone, Marrow, PreB, IgM,Mu. | Plasma cells. Includes genes coding for Immunoglobulin chains (e.g. IGHM, IGJ, IGLL1, IGKC, IGHD) and the plasma cell marker CD38. |
| M 1.2 | 130 | Platelet, Adhesion, Aggregation, Endothelial, Vascular | Platelets. Includes genes coding for platelet glycoproteins (ITGA2B, ITGB3, GP6, GP1A/B), and platelet-derived immune mediators such as PPPB (pro-platelet basic protein) and PF4 (platelet factor 4). |
| M 1.3 | 80 | Immunoreceptor, BCR, B-cell, IgG | B-cells. Includes genes coding for B-cell surface markers (CD72, CD79A/B, CD19, CD22) and other B-cell associated molecules: Early B-cell factor (EBF), B-cell linker (BLNK) and B lymphoid tyrosine kinase (BLK). |
| M 1.4 | 132 | Replication, Repression, Repair, CREB, Lymphoid, TNF-alpha | Undetermined. This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | 142 | Monocytes, Dendritic, MHC, Costimulatory, TLR4, MYD88 | Myeloid lineage. Includes molecules expressed by cells of the myeloid lineage (CD86, CD163, FCGR2A), some of which being involved in pathogen recognition (CD14, TLR2, MYD88). This set also includes TNF family members (TNFR2, BAFF). |
| M 1.6 | 141 | Zinc, Finger, P53, RAS | Undetermined. This set includes genes coding for signaling molecules, e.g. the zinc finger containing inhibitor of activated STAT (PIAS1 and PIAS2), or the nuclear factor of activated T-cells NFATC3. |
| M 1.7 | 129 | Ribosome, Translational, 40S, 60S, HLA | MHC/Ribosomal proteins. Almost exclusively formed by genes coding MHC class I molecules (HLA-A,B,C,G,E)+ Beta 2-microglobulin (B2M) or Ribosomal proteins (RPLs, RPSs). |
| M 1.8 | 154 | Metabolism, Biosynthesis, Replication, Helicase | Undetermined. Includes genes encoding metabolic enzymes (GLS, NSF1, NAT1) and factors involved in DNA replication (PURA, TERF2, EIF2S1). |
| M 2.1 | 95 | NK, Killer, Cytolytic, CD8, Cell-mediated, T-cell, CTL, IFN-g | Cytotoxic cells. Includes cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | 49 | Granulocytes, Neutrophils, Defense, Myeloid, Marrow | Neutrophils. This set includes innate molecules that are found in neutrophil granules (Lactotransferrin: LTF, defensin: DEAF1, Bacterial Permeability Increasing protein: BPI, Cathelicidin antimicrobial protein: CAMP...). |
| M 2.3 | 148 | Erythrocytes, Red, Anemia, Globin, Hemoglobin | Erythrocytes. Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF). |
| M 2.4 | 133 | Ribonucleoprotein, 60S, nucleolus, Assembly, Elongation | Ribosomal proteins. Including genes encoding ribosomal proteins (RPLs, RPSs), Eukaryotic Translation Elongation factor family members (EEFs) and Nucleolar proteins (NPM1, NOAL2, NAP1L1). |
| M 2.5 | 315 | Adenoma, Interstitial, Mesenchyme, Dendrite, Motor | Undetermined. This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | 165 | Granulocytes, Monocytes, Myeloid, ERK, Necrosis | Myeloid lineage. Includes genes expressed in myeloid lineage cells (IGTB2/CD18, Lymphotoxin beta receptor, Myeloid related proteins 8/14 Formyl peptide receptor 1), such as Monocytes and Neutrophils. |
| M 2.7 | 71 | No keywords extracted. | Undetermined. This module is largely composed of transcripts with no known function. Only 20 genes associated with literature, including a member of the chemokine-like factor superfamily (CKLFSF8). |
| M 2.8 | 141 | Lymphoma, T-cell, CD4, CD8, TCR, Thymus, Lymphoid, IL2 | T-cells. Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | 159 | ERK, Transactivation, Cytoskeletal, MAPK, JNK | Undetermined. Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M2.10 | 106 | Myeloid, Macrophage, Dendritic, Inflammatory, Interleukin | Undetermined. Includes genes encoding for Immune-related cell surface molecules (CD36, CD86, LILRB), cytokines (IL15) and molecules involved in signaling pathways (FYB, TICAM2-Toll-like receptor pathway). |
| M 2.11 | 176 | Replication, Repress, RAS, Autophosphorylation, Oncogenic | Undetermined. Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | 122 | ISRE, Influenza, Antiviral, IFN-gamma, IFN-alpha, Interferon | Interferon-inducible. This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | 322 | TGF-beta, TNF, | Inflammation I. Includes genes encoding molecules involved |
| | | Inflammatory, Apoptotic, Lipopolysaccharide | in inflammatory processes (e.g. IL8, ICAM1, C5R1, CD44, PLAUR, IL1A, CXCL16), and regulators of apoptosis (MCL1, FOX03A, RARA, BCL3/6/2A1, GADD45B). |
| M 3.3 | 276 | Inflammatory, Defense, Lysosomal, Oxidative, LPS | Inflammation II. Includes molecules inducing or inducible by inflammation (IL18, ALOX5, ANPEP, AOAH, HMOX1, SERPINB1), as well as lysosomal enzymes (PPT1, CTSB/S, NEU1, ASAH1, LAMP2, CAST). |
| M 3.4 | 325 | Ligase, Kinase, KIP1, Ubiquitin, Chaperone | Undetermined. Includes protein phosphatases (PPP1R12A, PTPRC, PPP1CB, PPM1B) and phosphoinositide 3-kinase (PI3K) family members (PIK3CA, PIK32A, PIP5K3). |
| M 3.5 | 22 | No keyword extracted | Undetermined. Composed of only a small number of transcripts. Includes hemoglobin genes (HBA1, HBA2, HBB). |
| M 3.6 | 288 | Ribosomal, T-cell, Beta-catenin | Undetermined. This set includes mitochondrial ribosomal proteins (MRPLs, MRPs), mitochondrial elongations factors (GFM1/2), Sortin Nexins (SN1/6/14) as well as lysosomal ATPases (ATP6V1C/D). |
| M 3.7 | 301 | Spliceosome, Methylation, Ubiquitin | Undetermined. Includes genes encoding proteasome subunits (PSMA2/5, PSMB5/8); ubiquitin protein ligases HIP2, STUB1, as well as components ofubiqutin ligase complexes (SUGT1). |
| M 3.8 | 284 | CDC, TCR, CREB, Glycosylase | Undetermined. Includes genes encoding enzymes: aminomethyltransferase, arginyltransferase, asparagines synthetase, diacylglycerol kinase, inositol phosphatases, methyltransferases, helicases... |
| M 3.9 | 260 | Chromatin, Checkpoint, Replication, Transactivation | Undetermined. Includes genes encoding kinases (IBTK, PRKRIR, PRKDC, PRKCI) and phosphatases (e.g. PTPLB, PPP2CB/3CB, PTPRC, MTM1, MTMR2). |

As used herein, the term "array" refers to a solid support or substrate with one or more peptides or nucleic acid probes attached to the support. Arrays typically have one or more different nucleic acid or peptide probes that are coupled to a surface of a substrate in different, known locations. These arrays, also described as "microarrays", "gene-chips" or DNA chips that may have 10,000; 20,000, 30,000; or 40,000 different identifiable genes based on the known genome, e.g., the human genome. These pan-arrays are used to detect the entire "transcriptome" or transcriptional pool of genes that are expressed or found in a sample, e.g., nucleic acids that are expressed as RNA, mRNA and the like that may be subjected to RT and/or RT-PCR to made a complementary set of DNA replicons. Arrays may be produced using mechanical synthesis methods, light directed synthesis methods and the like that incorporate a combination of non-lithographic and/or photolithographic methods and solid phase synthesis methods. Bead arrays that include 50-mer oligonucleotide probes attached to 3 micrometer beads may be used that are, e.g., lodged into microwells at the surface of a glass slide or are part of a liquid phase suspension arrays (e.g., Luminex or Illumina) that are digital beadarrays in liquid phase and uses "barcoded" glass rods for detection and identification.

Various techniques for the synthesis of these nucleic acid arrays have been described, e.g., fabricated on a surface of virtually any shape or even a multiplicity of surfaces. Arrays may be peptides or nucleic acids on beads, gels, polymeric surfaces, fibers such as fiber optics, glass or any other appropriate substrate. Arrays may be packaged in such a manner as to allow for diagnostics or other manipulation of an all inclusive device, see for example, U.S. Pat. No. 6,955,788.

### BIOLOGICAL DEFINITIONS

As used herein, the term "disease" refers to a physiological state of an organism with any abnormal biological state of a cell. Disease includes, but is not limited to, an interruption, cessation or disorder of cells, tissues, body functions, systems or organs that may be inherent, inherited, caused by an infection, caused by abnormal cell function, abnormal cell division and the like. A disease that leads to a "disease state" is generally detrimental to the biological system, that is, the host of the disease. With respect to the present invention, any biological state, such as an infection (e.g., viral, bacterial, fungal, helminthic, etc.), inflammation, autoinflammation, autoimmunity, anaphylaxis, allergies, premalignancy, malignancy, surgical, transplantation, physiological, and the like that is associated with a disease or disorder is considered to be a disease state. A pathological state is generally the equivalent of a disease state.

Disease states may also be categorized into different levels of disease state. As used herein, the level of a disease or disease state is an arbitrary measure reflecting the progression of a disease or disease state as well as the physiological response upon, during and after treatment. Generally, a disease or disease state will progress through levels or stages, wherein the affects of the disease become increasingly severe. The level of a disease state may be impacted by the physiological state of cells in the sample.

As used herein, the terms "therapy" or "therapeutic regimen" refer to those medical steps taken to alleviate or alter a disease state, e.g., a course of treatment intended to reduce or eliminate the affects or symptoms of a disease using pharmacological, surgical, dietary and/or other techniques. A therapeutic regimen may include a prescribed dosage of one or more drugs or surgery. Therapies will most often be beneficial and reduce the disease state but in many instances the effect of a therapy will have non-desirable or side-effects. The effect of therapy will also be impacted by the physiological state of the host, e.g., age, gender, genetics, weight, other disease conditions, etc.

As used herein, the term "pharmacological state" or "pharmacological status" refers to those samples that will be, are and/or were treated with one or more drugs, surgery and the like that may affect the pharmacological state of one or more nucleic acids in a sample, e.g., newly transcribed, stabilized and/or destabilized as a result of the pharmacological intervention. The pharmacological state of a sample relates to changes in the biological status before, during and/or after drug treatment and may serve a diagnostic or prognostic function, as taught herein. Some changes following drug treatment or surgery may be relevant to the disease state and/or may be unrelated side-effects of the therapy. Changes in the pharmacological state are the likely results of the duration of therapy, types and doses of drugs prescribed, degree of compliance with a given course of therapy, and/or un-prescribed drugs ingested.

As used herein, the term "biological state" refers to the state of the transcriptome (that is the entire collection of RNA transcripts) of the cellular sample isolated and purified for the analysis of changes in expression. The biological state reflects the physiological state of the cells in the sample by measuring the abundance and/or activity of cellular constituents, characterizing according to morphological phenotype or a combination of the methods for the detection of transcripts.

As used herein, the term "expression profile" refers to the relative abundance of RNA, DNA or protein abundances or activity levels. The expression profile can be a measurement for example of the transcriptional state or the translational state by any number of methods and using any of a number of gene-chips, gene arrays, beads, multiplex PCR, quantitiative PCR, run-on assays, Northern blot analysis, Western blot analysis, protein expression, fluorescence activated cell sorting (FACS), enzyme linked immunosorbent assays (ELISA), chemiluminescence studies, enzymatic assays, proliferation studies or any other method, apparatus and system for the determination and/or analysis of gene expression that are readily commercially available.

As used herein, the term "transcriptional state" of a sample includes the identities and relative abundances of the RNA species, especially mRNAs present in the sample. The entire transcriptional state of a sample, that is the combination of identity and abundance of RNA, is also referred to herein as the transcriptome. Generally, a substantial fraction of all the relative constituents of the entire set of RNA species in the sample are measured.

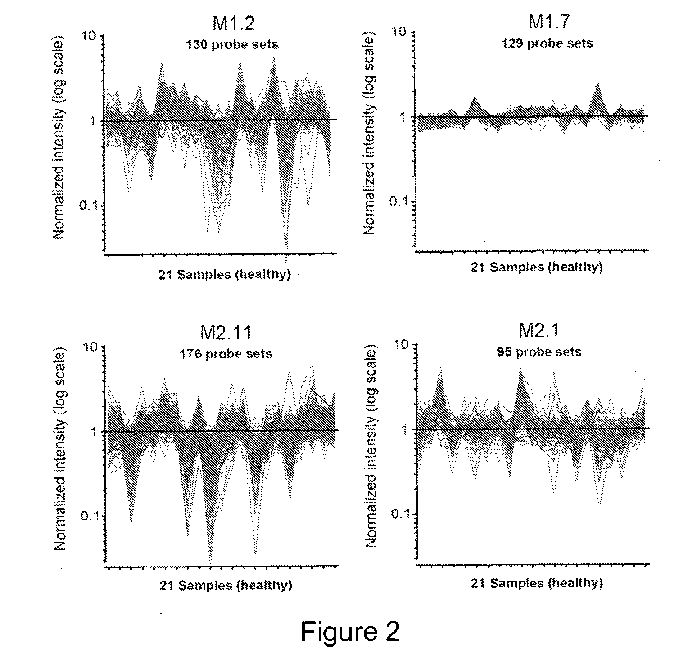

As used herein, the term "transcriptional vectors," "expression vectors," "genomic vectors" (used interchangeably) refers to transcriptional expression data that reflects the "proportion of differentially expressed genes." For example, for each module the proportion of transcripts differentially expressed between at least two groups (e.g., healthy subjects versus patients). This vector is derived from the comparison of two groups of samples. The first analytical step is used for the selection of disease-specific sets of transcripts within each module. Next, there is the "expression level." The group comparison for a given disease provides the list of differentially expressed transcripts for each module. It was found that different diseases yield different subsets of modular transcripts. With this expression level it is then possible to calculate vectors for each module(s) for a single sample by averaging expression values of disease-specific subsets of genes identified as being differentially expressed. This approach permits the generation of maps of modular expression vectors for a single sample, e.g., those described in the module maps disclosed herein. These vector module maps represent an averaged expression level for each module (instead of a proportion of differentially expressed genes) that can be derived for each sample. These composite "expression vectors" are formed through successive rounds of selection: 1) of the modules that were significantly changed across study groups and 2) of the genes within these modules which are significantly changed across study groups (Figure 2, *step II*). Expression levels are subsequently derived by averaging the values obtained for the subset of transcripts forming each vector (Figure 2, *step III*). Patient profiles can then be represented by plotting expression levels obtained for each of these vectors on a graph (e.g. on a radar plot). Therefore a set of vectors results from two round of selection, first at the module level, and then at the gene level. Vector expression values are composite by construction as they derive from the average expression values of the transcript forming the vector.

Using the present invention it is possible to identify and distinguish diseases not only at the module-level, but also at the gene-level; i.e., two diseases can have the same vector (identical proportion of differentially expressed transcripts, identical "polarity"), but the gene composition of the expression vector can still be disease-specific. This disease-specific customization permits the user to optimize the performance of a given set of markers by increasing its specificity.

Using modules as a foundation grounds expression vectors to coherent functional and transcriptional units containing minimized amounts of noise. Furthermore, the present invention takes advantage of composite transcriptional markers. As used herein, the term "composite transcriptional markers" refers to the average expression values of multiple genes (subsets of modules) as compared to using individual genes as markers (and the composition of these markers can be disease-specific). The composite transcriptional markers approach is unique because the user can develop multivariate microarray scores to assess disease severity in patients with, e.g., SLE, or to derive expression vectors disclosed herein. The fact that expression vectors are composite (i.e. formed by a combination of transcripts) further contributes to the stability of these markers. Most importantly, it has been found that using the composite modular transcriptional markers of the present invention the results found herein are reproducible across microarray platform, thereby providing greater reliability for regulatory approval. Indeed, vector expression values proved remarkably robust, as indicated by the excellent reproducibility obtained across microarray platforms; as well as the validation results obtained in an independent set of pediatric lupus patients. These results are of importance since improving the reliability of microarray data is a prerequisite for the widespread use of this technology in clinical practice.

Gene expression monitoring systems for use with the present invention may include customized gene arrays with a limited and/or basic number of genes that are specific and/or customized for the one or more target diseases. Unlike the general, pan-genome arrays that are in customary use, the present invention provides for not only the use of these general pan-arrays for retrospective gene and genome analysis without the need to use a specific platform, but more importantly, it provides for the development of customized arrays that provide an optimal gene set for analysis without the need for the thousands of other, non-relevant genes. One distinct advantage of the optimized arrays and modules of the present invention over the existing art is a reduction in the financial costs (e.g., cost per assay, materials, equipment, time, personnel, training, etc.), and more importantly, the environmental cost of manufacturing pan-arrays where the vast majority of the data is irrelevant. The modules of the present invention allow for the first time the design of simple, custom arrays that provide optimal data with the least number of probes while maximizing the signal to noise ratio. By eliminating the total number of genes for analysis, it is possible to, e.g., eliminate the need to manufacture thousands of expensive platinum masks for photolithography during the manufacture of pan-genetic chips that provide vast amounts of irrelevant data. Using the present invention it is possible to completely avoid the need for microarrays if the limited probe set(s) of the present invention are used with, e.g., digital optical chemistry arrays, ball bead arrays, beads (e.g., Luminex), multiplex PCR, quantitiative PCR, run-on assays, Northern blot analysis, or even, for protein analysis, e.g., Western blot analysis, 2-D and 3-D gel protein expression, MALDI, MALDI-TOF, fluorescence activated cell sorting (FACS) (cell surface or intracellular), enzyme linked immunosorbent assays (ELISA), chemiluminescence studies, enzymatic assays, proliferation studies or any other method, apparatus and system for the determination and/or analysis of gene expression that are readily commercially available.

The "molecular fingerprinting system" of the present invention may be used to facilitate and conduct a comparative analysis of expression in different cells or tissues, different subpopulations of the same cells or tissues, different physiological states of the same cells or tissue, different developmental stages of the same cells or tissue, or different cell populations of the same tissue against other diseases and/or normal cell controls. In some cases, the normal or wild-type expression data may be from samples analyzed at or about the same time or it may be expression data obtained or culled from existing gene array expression databases, e.g., public databases such as the NCBI Gene Expression Omnibus database.

As used herein, the term "differentially expressed" refers to the measurement of a cellular constituent (e.g., nucleic acid, protein, enzymatic activity and the like) that varies in two or more samples, e.g., between a disease sample and a normal sample. The cellular constituent may be on or off (present or absent), upregulated relative to a reference or downregulated relative to the reference. For use with gene-chips or gene-arrays, differential gene expression of nucleic acids, e.g., mRNA or other RNAs (miRNA, siRNA, hnRNA, rRNA, tRNA, etc.) may be used to distinguish between cell types or nucleic acids. Most commonly, the measurement of the transcriptional state of a cell is accomplished by quantitative reverse transcriptase (RT) and/or quantitative reverse transcriptase-polymerase chain reaction (RT-PCR), genomic expression analysis, post-translational analysis, modifications to genomic DNA, translocations, in situ hybridization and the like.

For some disease states it is possible to identify cellular or morphological differences, especially at early levels of the disease state. The present invention avoids the need to identify those specific mutations or one or more genes by looking at modules of genes of the cells themselves or, more importantly, of the cellular RNA expression of genes from immune effector cells that are acting within their regular physiologic context, that is, during immune activation, immune tolerance or even immune anergy. While a genetic mutation may result in a dramatic change in the expression levels of a group of genes, biological systems often compensate for changes by altering the expression of other genes. As a result of these internal compensation responses, many perturbations may have minimal effects on observable phenotypes of the system but profound effects to the composition of cellular constituents. Likewise, the actual copies of a gene transcript may not increase or decrease, however, the longevity or half-life of the transcript may be affected leading to greatly increases protein production. The present invention eliminates the need of detecting the actual message by, in one embodiment, looking at effector cells (e.g., leukocytes, lymphocytes and/or sub-populations thereof) rather than single messages and/or mutations.

The skilled artisan will appreciate readily that samples may be obtained from a variety of sources including, e.g., single cells, a collection of cells, tissue, cell culture and the like. In certain cases, it may even be possible to isolate sufficient RNA from cells found in, e.g., urine, blood, saliva, tissue or biopsy samples and the like. In certain circumstances, enough cells and/or RNA may be obtained from: mucosal secretion, feces, tears, blood plasma, peritoneal fluid, interstitial fluid, intradural, cerebrospinal fluid, sweat or other bodily fluids. The nucleic acid source, e.g., from tissue or cell sources, may include a tissue biopsy sample, one or more sorted cell populations, cell culture, cell clones, transformed cells, biopies or a single cell. The tissue source may include, e.g., brain, liver, heart, kidney, lung, spleen, retina, bone, neural, lymph node, endocrine gland, reproductive organ, blood, nerve, vascular tissue, and olfactory epithelium.

The present invention includes the following basic components, which may be used alone or in combination, namely, one or more data mining algorithms; one or more module-level analytical processes; the characterization of blood leukocyte transcriptional modules; the use of aggregated modular data in multivariate analyses for the molecular diagnostic/prognostic of human diseases; and/or visualization of module-level data and results. Using the present invention it is also possible to develop and analyze composite transcriptional markers, which may be further aggregated into a single multivariate score.

The present inventors have recognized that current microarray-based research is facing significant challenges with the analysis of data that are notoriously "noisy," that is, data that is difficult to interpret and does not compare well across laboratories and platforms. A widely accepted approach for the analysis of microarray data begins with the identification of subsets of genes differentially expressed between study groups. Next, the users try subsequently to "make sense" out of resulting gene lists using pattern discovery algorithms and existing scientific knowledge.

Rather than deal with the great variability across platforms, the present inventors have developed a strategy that emphasized the selection of biologically relevant genes at an early stage of the analysis. Briefly, the method includes the identification of the transcriptional components characterizing a given biological system for which an improved data mining algorithm was developed to analyze and extract groups of coordinately expressed genes, or transcriptional modules, from large collections of data.

The biomarker discovery strategy described herein is particularly well adapted for the exploitation of microarray data acquired on a global scale. Starting from ~44,000 transcripts a set of 28 modules was defined that are composed of nearly 5000 transcripts. Sets of disease-specific composite expression vectors were then derived. Vector expression values (expression vectors) proved remarkably robust, as indicated by the excellent reproducibility obtained across microarray platforms. This finding is notable, since improving the reliability of microarray data is a prerequisite for the widespread use of this technology in clinical practice. Finally, expression vectors can in turn be combined to obtain unique multivariate scores, therefore delivering results in a form that is compatible with mainstream clinical practice. Interestingly, multivariate scores recapitulate global patterns of change rather than changes in individual markers. The development of such "global biomarkers" can be used for both diagnostic and pharmacogenomics fields.

In one example, twenty-eight transcriptional modules regrouping 4742 probe sets were obtained from 239 blood leukocyte transcriptional profiles. Functional convergence among genes forming these modules was demonstrated through literature profiling. The second step consisted of studying perturbations of transcriptional systems on a modular basis. To illustrate this concept, leukocyte transcriptional profiles obtained from healthy volunteers and patients were obtained, compared and analyzed. Further validation of this gene fingerprinting strategy was obtained through the analysis of a published microarray dataset. Remarkably, the modular transcriptional apparatus, system and methods of the present invention using pre-existing data showed a high degree of reproducibility across two commercial microarray platforms.

The present invention includes the implementation of a widely applicable, two-step microarray data mining strategy designed for the modular analysis of transcriptional systems. This novel approach was used to characterize transcriptional signatures of blood leukocytes, which constitutes the most accessible source of clinically relevant information.

As demonstrated herein, it is possible to determine, differential and/or distinguish between two disease based on two vectors even if the vector is identical (+/+) for two diseases - e.g. M1.3 = 53% down for both SLE and FLU because the composition of each vector can still be used to differentiate them. For example, even though the proportion and polarity of differentially expressed transcripts is identical between the two diseases for M1.3, the gene composition can still be disease-specific. The combination of gene-level and module-level analysis considerably increases resolution. Furthermore, it is possible to use 2, 3, 4, 5, 10, 15, 20, 25, 28 or more modules to differentiate diseases.

The term "gene" refers to a nucleic acid (e.g., DNA) sequence that includes coding sequences necessary for the production of a polypeptide (e.g., ), precursor, or RNA (e.g., mRNA). The polypeptide may be encoded by a full length coding sequence or by any portion of the coding sequence so long as the desired activity or functional property (e.g., enzymatic activity, ligand binding, signal transduction, immunogenicity, etc.) of the full-length or fragment is retained. The term also encompasses the coding region of a structural gene and the sequences located adjacent to the coding region on both the 5' and 3' ends for a distance of about 2 kb or more on either end such that the gene corresponds to the length of the full-length mRNA and 5' regulatory sequences which influence the transcriptional properties of the gene. Sequences located 5' of the coding region and present on the mRNA are referred to as 5'-untranslated sequences. The 5'-untranslated sequences usually contain the regulatory sequences. Sequences located 3' or downstream of the coding region and present on the mRNA are referred to as 3'-untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "nucleic acid" refers to any nucleic acid containing molecule, including but not limited to, DNA, cDNA and RNA. In particular, the terms "a gene in Table X" refers to at least a portion or the full-length sequence listed in a particular table, as found hereinbelow. The gene may even be found or detected a genomic form, that is, it includes one or more intron(s). Genomic forms of a gene may also include sequences located on both the 5' and 3' end of the coding sequences that are present on the RNA transcript. These sequences are referred to as "flanking" sequences or regions. The 5' flanking region may contain regulatory sequences such as promoters and enhancers that control or influence the transcription of the gene. The 3' flanking region may contain sequences that influence the transcription termination, post-transcriptional cleavage, mRNA stability and polyadenylation.

As used herein, the term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified" or "mutant" refers to a gene or gene product that displays modifications in sequence and/or functional properties (i.e., altered characteristics) when compared to the wild-type gene or gene product. It is noted that naturally occurring mutants can be isolated; these are identified by the fact that they have altered characteristics (including altered nucleic acid sequences) when compared to the wild-type gene or gene product.

As used herein, the term "polymorphism" refers to the regular and simultaneous occurrence in a single interbreeding population of two or more alleles of a gene, where the frequency of the rarer alleles is greater than can be explained by recurrent mutation alone (typically greater than 1%).

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide protein) chain. The DNA sequence thus codes for the amino acid sequence.

As used herein, the terms "complementary" or "complementarity" are used in reference to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

As used herein, the term "hybridization" is used in reference to the pairing of complementary nucleic acids. Hybridization and the strength of hybridization (i.e., the strength of the association between the nucleic acids) is impacted by such factors as the degree of complementarity between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein the term "stringency" is used in reference to the conditions of temperature, ionic strength, and the presence of other compounds such as organic solvents, under which nucleic acid hybridizations are conducted. Under "low stringency conditions" a nucleic acid sequence of interest will hybridize to its exact complement, sequences with single base mismatches, closely related sequences (e.g., sequences with 90% or greater homology), and sequences having only partial homology (e.g., sequences with 50-90% homology). Under "medium stringency conditions," a nucleic acid sequence of interest will hybridize only to its exact complement, sequences with single base mismatches, and closely related sequences (e.g., 90% or greater homology). Under "high stringency conditions," a nucleic acid sequence of interest will hybridize only to its exact complement, and (depending on conditions such a temperature) sequences with single base mismatches. In other words, under conditions of high stringency the temperature can be raised so as to exclude hybridization to sequences with single base mismatches.

As used herein, the term "probe" refers to an oligonucleotide (i.e., a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular gene sequences. Any probe used in the present invention may be labeled with any "reporter molecule," so that it is detectable in any detection system, including, but not limited to enzyme (e.g., ELISA, as well as enzyme-based histochemical assays), fluorescent, radioactive, luminescent systems and the like. It is not intended that the present invention be limited to any particular detection system or label.

As used herein, the term "target," refers to the region of nucleic acid bounded by the primers. Thus, the "target" is sought to be sorted out from other nucleic acid sequences. A "segment" is defined as a region of nucleic acid within the target sequence.

As used herein, the term "Southern blot" refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (Sambrook et al., Molecular Cloning: A Laboratory Manual, Cold Spring Harbor Press, NY, pp 9.31-9.58, 1989).

As used herein, the term "Northern blot" refers to the analysis of RNA by electrophoresis of RNA on agarose gels, to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (Sambrook, et al., supra, pp 7.39-7.52, 1989).

As used herein, the term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabeled antibodies.

As used herein, the term "polymerase chain reaction" ("PCR") refers to the method of K. B. Mullis (U.S. Pat. Nos. 4,683,195 4,683,202, and 4,965,188), which describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "real time PCR" as used herein, refers to various PCR applications in which amplification is measured during as opposed to after completion of the reaction. Reagents suitable for use in real time PCR embodiments of the present invention include but are not limited to TaqMan probes, molecular beacons, Scorpions primers or double-stranded DNA binding dyes.

As used herein, the term "transcriptional upregulation" as used herein refers to an increase in synthesis of RNA, by RNA polymerases using a DNA template. For example, when used in reference to the methods of the present invention, the term "transcriptional upregulation" refers to an increase of least 1 to 2 fold, 2 to 3 fold, 3 to 10 fold, and even greater than 10 fold, in the quantity of mRNA corresponding to a gene of interest detected in a sample derived from an individual predisposed to SLE as compared to that detected in a sample derived from an individual who is not predisposed to SLE. However, the system and evaluation is sufficiently specific to require less that a 2 fold change in expression to be detected. Furthermore, the change in expression may be at the cellular level (change in expression within a single cell or cell populations) or may even be evaluated at a tissue level, where there is a change in the number of cells that are expressing the gene. Particularly useful differences are those that are statistically significant.

Conversely, the term "transcriptional downregulation" refers to a decrease in synthesis of RNA, by RNA polymerases using a DNA template. For example, when used in reference to the methods of the present invention, the term "transcriptional downregulation" refers to a decrease of least 2 fold, 2 to 3 fold, 3 to 10 fold, and even greater than 10 fold, in the quantity of mRNA corresponding to a gene of interest detected in a sample derived from an individual predisposed to SLE as compared to that detected in a sample derived from an individual who is not predisposed to such a condition or to a database of information for wild-type and/or normal control, e.g., fibromyalgia. Again, the system and evaluation is sufficiently specific to require less that a 2 fold change in expression to be detected. Particularly useful differences are those that are statistically significant.

Both transcriptional "upregulation"/overexpression and transcriptional "downregulation"/underexpression may also be indirectly monitored through measurement of the translation product or protein level corresponding to the gene of interest. The present invention is not limited to any given mechanism related to upregulation or downregulation of transcription.

The term "eukaryotic cell" as used herein refers to a cell or organism with membrane-bound, structurally discrete nucleus and other well-developed subcellular compartments. Eukaryotes include all organisms except viruses, bacteria, and bluegreen algae.

As used herein, the term "in vitro transcription" refers to a transcription reaction comprising a purified DNA template containing a promoter, ribonucleotide triphosphates, a buffer system that includes a reducing agent and cations, e.g., DTT and magnesium ions, and an appropriate RNA polymerase, which is performed outside of a living cell or organism.

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

As used herein, the term "diagnosis" refers to the determination of the nature of a case of disease. In some embodiments of the present invention, methods for making a diagnosis are provided which permit determination of SLE.

The present invention may be used alone or in combination with disease therapy to monitor disease progression and/or patient management. For example, a patient may be tested one or more times to determine the best course of treatment, determine if the treatment is having the intended medical effect, if the patient is not a candidate for that particular therapy and combinations thereof. The skilled artisan will recognize that one or more of the expression vectors may be indicative of one or more diseases and may be affected by other conditions, be they acute or chronic.

As used herein, the term "pharmacogenetic test" refers to an assay intended to study interindividual variations in DNA sequence related to, e.g., drug absorption and disposition (pharmacokinetics) or drug action (pharmacodynamics), which may include polymorphic variations in one or more genes that encode the functions of, e.g., transporters, metabolizing enzymes, receptors and other proteins.

As used herein, the term "pharmacogenomic test" refers to an assay used to study interindividual variations in whole-genome or candidate genes, e.g., single-nucleotide polymorphism (SNP) maps or haplotype markers, and the alteration of gene expression or inactivation that may be correlated with pharmacological function and therapeutic response.

As used herein, an "expression profile" refers to the measurement of the relative abundance of a plurality of cellular constituents. Such measurements may include, e.g., RNA or protein abundances or activity levels. The expression profile can be a measurement for example of the transcriptional state or the translational state. See U.S. Pat. Nos. 6,040,138, 5,800,992, 6,020,135, 6,033,860. The gene expression monitoring system, include nucleic acid probe arrays, membrane blot (such as used in hybridization analysis such as Northern, Southern, dot, and the like), or microwells, sample tubes, gels, beads or fibers (or any solid support comprising bound nucleic acids). See, e.g., U.S. Pat. Nos. 5,770,722, 5,874,219, 5,744,305, 5,677,195 and 5,445,934. The gene expression monitoring system may also comprise nucleic acid probes in solution.

The gene expression monitoring system according to the present invention may be used to facilitate a comparative analysis of expression in different cells or tissues, different subpopulations of the same cells or tissues, different physiological states of the same cells or tissue, different developmental stages of the same cells or tissue, or different cell populations of the same tissue.

As used herein, the term "differentially expressed: refers to the measurement of a cellular constituent varies in two or more samples. The cellular constituent can be either up-regulated in the test sample relative to the reference or down-regulated in the test sample relative to one or more references. Differential gene expression can also be used to distinguish between cell types or nucleic acids. See U.S. Pat. No. 5,800,992.

Therapy or Therapeutic Regimen: In order to alleviate or alter a disease state, a therapy or therapeutic regimen is often undertaken. A therapy or therapeutic regimen, as used herein, refers to a course of treatment intended to reduce or eliminate the affects or symptoms of a disease. A therapeutic regimen will typically comprise, but is not limited to, a prescribed dosage of one or more drugs or surgery. Therapies, ideally, will be beneficial and reduce the disease state but in many instances the effect of a therapy will have non-desirable effects as well. The effect of therapy will also be impacted by the physiological state of the sample.

Modules display distinct "transcriptional behavior". It is widely assumed that co-expressed genes are functionally linked. This concept of "guilt by association" is particularly compelling in cases where genes follow complex expression patterns across many samples. The present inventors discovered that transcriptional modules form coherent biological units and, therefore, predicted that the co-expression properties identified in the initial dataset would be conserved in an independent set of samples. Data were obtained for PBMCs isolated from the blood of twenty-one healthy volunteers. These samples were not used in the module selection process described above.

The present invention includes the following basic components, which may be used alone or in combination, namely, one or more data mining algorithms; one or more module-level analytical processes; the characterization of blood leukocyte transcriptional modules; the use of aggregated modular data in multivariate analyses for the molecular diagnostic/prognostic of human diseases; and/or visualization of module-level data and results. Using the present invention it is also possible to develop and analyze composite transcriptional markers, which may be further aggregated into a single multivariate score.

The present inventors have recognized that current microarray-based research is facing significant challenges with the analysis of data that are notoriously "noisy," that is, data that is difficult to interpret and does not compare well across laboratories and platforms. A widely accepted approach for the analysis of microarray data begins with the identification of subsets of genes differentially expressed between study groups. Next, the users try subsequently to "make sense" out of resulting gene lists using pattern discovery algorithms and existing scientific knowledge.

Rather than deal with the great variability across platforms, the present inventors have developed a strategy that emphasized the selection of biologically relevant genes at an early stage of the analysis. Briefly, the method includes the identification of the transcriptional components characterizing a given biological system for which an improved data mining algorithm was developed to analyze and extract groups of coordinately expressed genes, or transcriptional modules, from large collections of data.

The biomarker discovery strategy that we have developed is particularly well adapted for the exploitation of microarray data acquired on a global scale. Starting from ~44,000 transcripts the inventors defined 28 modules composed of nearly 5000 transcripts. Sets of disease-specific composite expression vectors were then derived. Vector expression values proved remarkably robust, as indicated by the excellent reproducibility obtained across microarray platforms. This finding is notable, since improving the reliability of microarray data is a prerequisite for the widespread use of this technology in clinical practice. Finally, vectors can in turn be combined to obtain unique multivariate scores, therefore delivering results in a form that is compatible with mainstream clinical practice. Interestingly, multivariate scores recapitulate global patterns of change rather than changes in individual markers. The development of such "global biomarkers" constitutes therefore a promising prospect for both diagnostic and pharmacogenomics fields.

In one example, twenty-eight transcriptional modules regrouping 4742 probe sets were obtained from 239 blood leukocyte transcriptional profiles. Functional convergence among genes forming these modules was demonstrated through literature profiling. The second step consisted of studying perturbations of transcriptional systems on a modular basis. To illustrate this concept, leukocyte transcriptional profiles obtained from healthy volunteers and patients were obtained, compared and analyzed. Further validation of this gene fingerprinting strategy was obtained through the analysis of a published microarray dataset. Remarkably, the modular transcriptional apparatus, system and methods of the present invention using pre-existing data showed a high degree of reproducibility across two commercial microarray platforms.

The present invention includes the implementation of a widely applicable, two-step microarray data mining strategy designed for the modular analysis of transcriptional systems. This novel approach was used to characterize transcriptional signatures of blood leukocytes, which constitutes the most accessible source of clinically relevant information.

As demonstrated herein, it is possible to determine, differential and/or distinguish between two disease based on two vectors even if the vector is identical (+/+) for two diseases - e.g. M1.3 = 53% down for both SLE and FLU because the composition of each vector can still be used to differentiate them. For example, even though the proportion and polarity of differentially expressed transcripts is identical between the two diseases for M1.3, the gene composition can still be disease-specific. The combination of gene-level and module-level analysis considerably increases resolution. Furthermore, it is possible to use 2, 3, 4, 5, 10, 15, 20, 25, 28 or more modules to differentiate diseases.

Material and methods. Processing of blood samples. All blood samples were collected in acid citrate dextrose tubes (BD Vacutainer) and immediately delivered at room temperature to the Baylor Institute for Immunology Research, Dallas, TX, for processing. Peripheral blood mononuclear cells (PBMCs) from 3-4 ml of blood were isolated *via* Ficoll gradient and immediately lysed in RLT reagent (Qiagen, Valencia, CA) with beta-mercaptoethanol (BME) and stored at -80°C prior to the RNA extraction step.

Microarray analysis. Total RNA was isolated using the RNeasy kit (Qiagen) according to the manufacturer's instructions and RNA integrity was assessed using an Agilent 2100 Bioanalyzer (Agilent, Palo Alto, CA).

Affymetrix GeneChips: These microarrays include short oligonucleotide probe sets synthesized *in situ* on a quartz wafer. Target labeling was performed according to the manufacturer's standard protocol (Affymetrix Inc., Santa Clara, CA). Biotinylated cRNA targets were purified and subsequently hybridized to Affymetrix HG-U133A and U133B GeneChips (>44,000 probe sets). Arrays were scanned using an Affymetrix confocal laser scanner. Microarray Suite, Version 5.0 (MAS 5.0; Affymetrix) software was used to assess fluorescent hybridization signals, to normalize signals, and to evaluate signal detection calls. Normalization of signal values per chip was achieved using the MAS 5.0 global method of scaling to the target intensity value of 500 per GeneChip. A gene expression analysis software program, GeneSpring, Version 7.1 (Agilent), was used to perform statistical analysis and hierarchical clustering.

Illumina BeadChips: These microarrays include 50mer oligonucleotide probes attached to 3µm beads, which are lodged into microwells at the surface of a glass slide. Samples were processed and acquired by Illumina Inc. (San Diego, CA) on the basis of a service contract. Targets were prepared using the Illumina RNA amplification kit (Ambion, Austin, TX). cRNA targets were hybridized to Sentrix HumanRef8 BeadChips (>25,000 probes), which were scanned on an Illumina BeadStation 500. Illumina's Beadstudio software was used to assess fluorescent hybridization signals.

Literature profiling. The literature profiling algorithm employed in this study has been previously described in detail (Chaussabel, D. & Sher, A. Mining microarray expression data by literature profiling. Genome Biol 3, RESEARCH0055 (2002)). This approach links genes sharing similar keywords. It uses hierarchical clustering, a popular unsupervised pattern discovery algorithm, to analyze patterns of term occurrence in literature abstracts. Step 1: A gene:literature index identifying pertinent publications for each gene is created. Step 2: Term occurrence frequencies were computed by a text processor. Step 3: Stringent filter criteria are used to select relevant keywords (i.e., eliminate terms with either high or low frequency across all genes and retain the few discerning terms characterized by a pattern of high occurrence for only a few genes). Step 4: Two-way hierarchical clustering groups of genes and relevant keywords based on occurrence patterns, providing a visual representation of functional relationships existing among a group of genes.

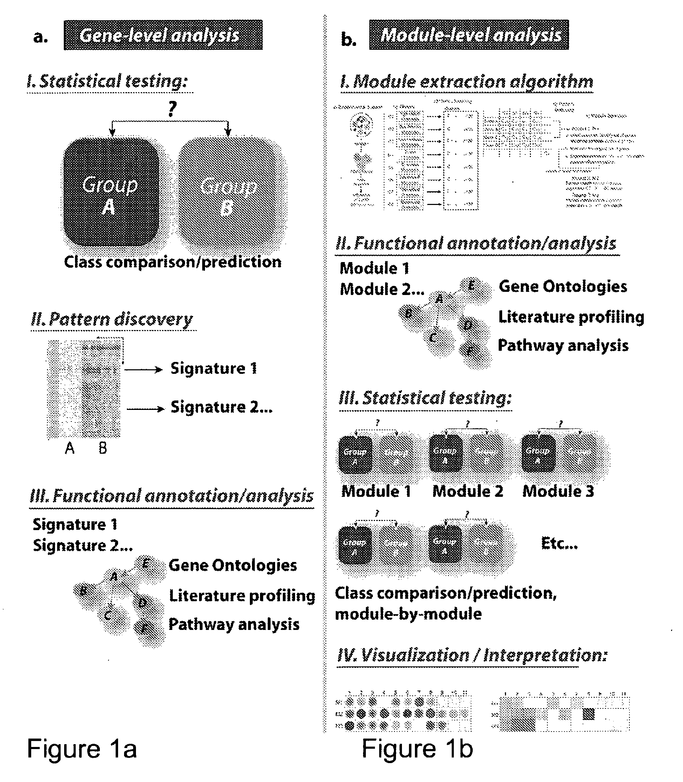

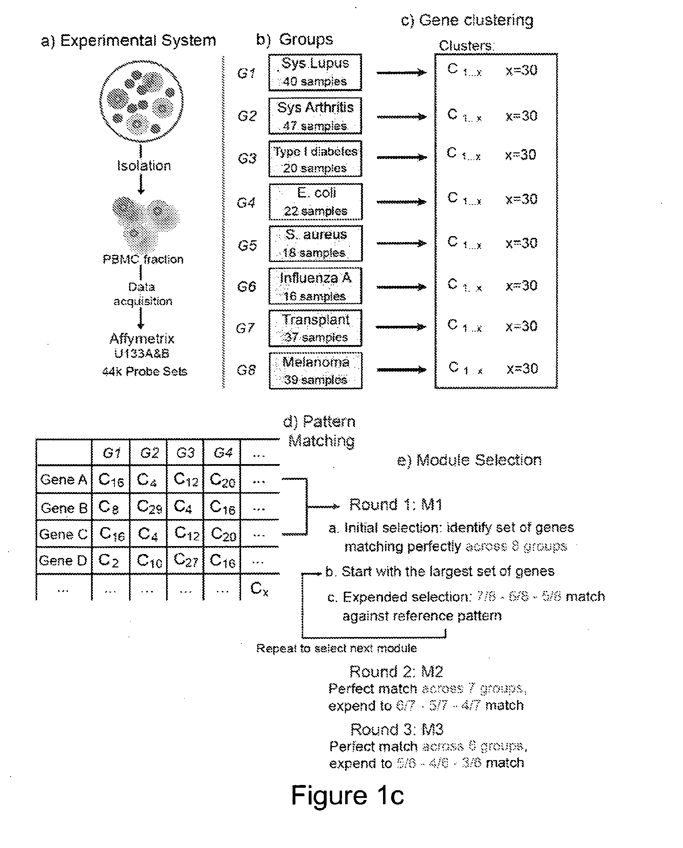

Modular data mining algorithm. First, one or more transcriptional components are identified that permit the characterization of biological systems beyond the level of single genes. Sets of coordinately regulated genes, or transcriptional modules, were extracted using a novel mining algorithm, which was applied to a large set of blood leukocyte microarray profiles (Figure 1). Gene expression profiles from a total of 239 peripheral blood mononuclear cells (PBMCs) samples were generated using Affymetrix U133A&B GeneChips (>44,000 probe sets). Transcriptional data were obtained for eight experimental groups (systemic juvenile idiopathic arthritis, systemic lupus erythematosus, type I diabetes, liver transplant recipients, melanoma patients, and patients with acute infections: *Escherichia coli, Staphylococcus aureus* and influenza A). For each group, transcripts with an absent flag call across all conditions were filtered out. The remaining genes were distributed among thirty sets by hierarchical clustering (clusters C1 through C30). The cluster assignment for each gene was recorded in a table and distribution patterns were compared among all the genes. Modules were selected using an iterative process, starting with the largest set of genes that belonged to the same cluster in all study groups (i.e. genes that were found in the same cluster in eight of the eight experimental groups). The selection was then expanded from this core reference pattern to include genes with 7/8, 6/8 and 5/8 matches. The resulting set of genes formed a transcriptional module and was withdrawn from the selection pool. The process was then repeated starting with the second largest group of genes, progressively reducing the level of stringency. This analysis led to the identification of 5348 transcripts that were distributed among twenty-eight modules (a complete list is provided as supplementary material). Each module is assigned a unique identifier indicating the round and order of selection (i.e. M3.1 was the first module identified in the third round of selection).

Analysis of "significance patterns" was performed on gene expression data generated from PBMCs obtained from patients and healthy volunteers using Affymetrix HG-U133A GeneChips that were run on the same Affymetrix system, using standard operating procedures. P values were obtained by comparing 7 groups of patients to their respective healthy control groups (Mann-Whitney rank test). The groups were composed of pediatric patients with: 1) Systemic Lupus Erythomatosus (SLE, 16 samples), 2) Influenza A (16 samples), 3) *Staphylococcus aureus* (16 samples), 4) *Escherichia coli* (16 samples) and 5) *Streptococcus pneumoniae* (14 samples); as well as adult transplant recipients: 6) Liver transplant patients that have accepted the graft under immunosuppressive therapy (16 samples) and 7) bone marrow transplant recipients undergoing graft versus host disease (GVHD, 12 samples). Control groups were also formed taking into account age, sex and project (10 samples in each group). Genes significantly changed (p<0.01) in the "study group" (Influenza A and/or SLE) were divided in two sets: over-expressed versus control and under-expressed versus control. P-values of the genes forming the over-expressed set were obtained for the "reference groups" (infections with *E. coli, S. aureus, S. pneumoniae,* Liver transplant recipients and graft versus host disease). P-values of the reference groups were set to 1 when genes were under-expressed. The same procedure was used in the set of genes under-expressed in study group, only this time P-values of the reference group were set to 1 when genes were over-expressed. P-value data were processed with a gene expression analysis software program, GeneSpring, Version 7.1 (Agilent), that was used to perform hierarchical clustering and group genes based on significance patterns.

Modules display distinct "transcriptional behavior". It is widely assumed that co-expressed genes are functionally linked. This concept of "guilt by association" is particularly compelling in cases where genes follow complex expression patterns across many samples. The present inventors discovered that transcriptional modules form coherent biological units and, therefore, predicted that the co-expression properties identified in the initial dataset would be conserved in an independent set of samples. Data were obtained for PBMCs isolated from the blood of twenty-one healthy volunteers. These samples were not used in the module selection process described above.

FIGURE 2 shows gene expression profiles of four different modules are shown (Figure 2: M1.2, M1.7, M2.11 and M2.1). In the graphs of Figure 2, each line represents the expression level (y-axis) of a single gene across multiple samples (21 samples on the x-axis). Differences in gene expression in this example represent inter-individual variation between "healthy" individuals. It was found that within each module genes display a coherent "transcriptional behavior". Indeed, the variation in gene expression appeared to be consistent across all the samples (for some samples the expression of all the genes was elevated and formed a peak, while in others levels were low for all the genes which formed a dip). Importantly, inter-individual variations appeared to be module-specific as peaks and dips formed for different samples in M1.2, M2.11 and M2.1. Furthermore, the amplitude of variation was also characteristic of each module, with levels of expression being more variable for M1.2 and M2.11 than M2.1 and especially M1.7. Thus, we find that transcriptional modules constitute independent biological variables.

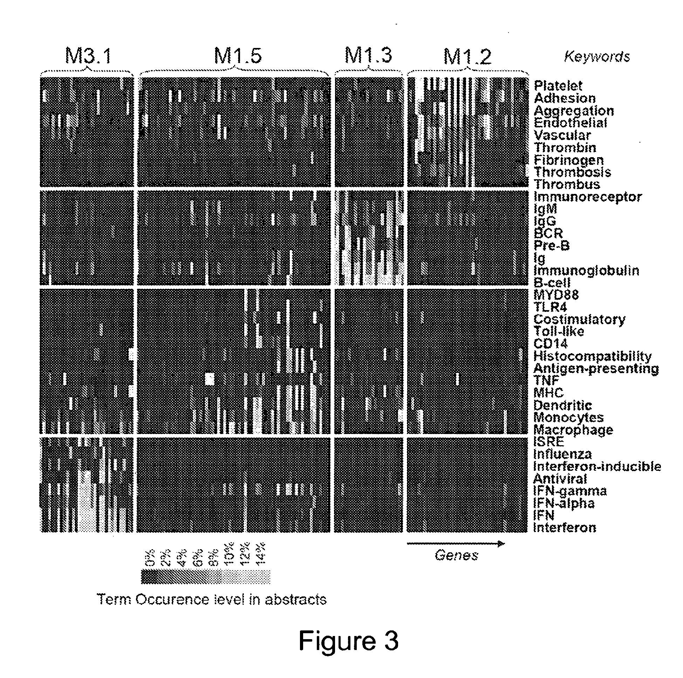

Functional characterization of transcriptional modules. Next, the modules were characterized at a functional level. A text mining approach was employed to extract keywords from the biomedical literature collected for each gene (described in ¹⁸). The distribution of keywords associated to the four modules that were analyzed is clearly distinct (Figure 3). The following is a list of keywords that may be associated with certain modules.
- Keywords highly specific for **M1.2** included *Platelet, Aggregation* or *Thrombosis,* and were associated with genes such as **ITGA2B** (Integrin alpha 2b, platelet glycoprotein IIb), **PF4** (platelet factor 4), **SELP** (Selectin P) and **GP6** (platelet glycoprotein 6).
- Keywords highly specific for **M1.3** included *B-cell, Immunoglobulin* or *IgG* and were associated with genes such as **CD19, CD22,** CD72A, **BLNK** (B cell linker protein), **BLK** (B lymphoid tyrosine kinase) and **PAX5** (paired box gene 5, a B-cell lineage specific activator).
- Keywords highly specific for **M1.5** included *Monocyte, Dendritic, CD14* or *Toll-like* and were associated with genes such as **MYD88** (myeloid differentiation primary response gene 88), **CD86, TLR2** (Toll-like receptor 2), **LILRB2** (leukocyte immunoglobulin-like receptor B2) and **CD163**.
- Keywords highly specific for **M3.1** included *Interferon, IFN-alpha, Antiviral,* or *ISRE* and were associated with genes such as **STAT1** (signal transducer and activator of transcription 1), **CXCL10** (CXC chemokine ligand 10, IP-10), **OAS2** (oligoadenylate synthetase 2) and **MX2** (myxovirus resistance 2).

This contrasted pattern of term occurrence denotes the remarkable functional coherence of each module. Information extracted from the literature for all the modules that have been identified permit a comprehensive functional characterization of the PBMC system at a transcriptional level. Table 2 provides an example of genes that may be used to distinguish between immune responses to, e.g., melanoma and liver transplant.

**Table 2: Genes in Module 1.4 Used to Distinguish Immune Responses**

| **moduleID** | **Entrez ID** | **Gene Symbol** | **Gene Title** |
|---|---|---|---|
| 1.4 | 55544 | RNPC1 | RNA-binding region (RNP1, RRM) containing 1 |
| 1.4 | 5930 | RBBP6 | retinoblastoma binding protein 6 |
| 1.4 | 80273 | GRPEL1 | GrpE-like 1, mitochondrial (*E*. *coli*) |
| 1.4 | 57162 | PELI1 | pellino homolog 1 (Drosophila) |
| 1.4 | 9921 | RNF10 | ring finger protein 10 /// ring finger protein 10 |
| 1.4 | 90637 | LOC90637 | hypothetical protein LOC90637 |
| 1.4 | 80314 | EPC1 | Enhancer of polycomb homolog 1 (Drosophila) |
| 1.4 | --- | --- | Full length insert cDNA clone ZB81B12 |
| 1.4 | 5756 | PTK9 | PTK9 protein tyrosine kinase 9 |
| 1.4 | 55038 | CDCA4 | cell division cycle associated 4 |
| 1.4 | 5187 | PER1 | period homolog 1 (Drosophila) |
| 1.4 | 9205 | ZNF237 | zinc finger protein 237 |
| 1.4 | 25976 | TIPARP | TCDD-inducible poly(ADP-ribose) polymerase |
| 1.4 | 57018 | CCNL1 | cyclin L1 |
| 1.4 | 64061 | TSPYL2 | TSPY-like 2 |
| 1.4 | 81488 | GRINL1A | glutamate receptor, ionotropic, N-methyl D-aspartate-like 1A |
| 1.4 | 22850 | KIAA0863 | KIAA0863 protein |
| 1.4 | 23764 | MAFF | v-maf musculoaponeurotic fibrosarcoma oncogene homolog F (avian) |
| 1.4 | 29035 | PRO0149 | PRO0149 protein |
| 1.4 | 7803 | PTP4A1 | protein tyrosine phosphatase type IVA, member 1 |
| 1.4 | 11171 | STRAP | serine/threonine kinase receptor associated protein |
| 1.4 | 5814 | PURB | purine-rich element binding protein B |
| 1.4 | 5142 | PDE4B | phosphodiesterase 4B, cAMP-specific (phosphodiesterase E4 dunce homolog, Drosophila) |
| 1.4 | 30836 | ERBP | estrogen receptor binding protein |
| 1.4 | 6782 | STCH | stress 70 protein chaperone, microsome-associated, 60kDa |
| 1.4 | 10950 | BTG3 | BTG family, member 3 |
| 1.4 | 7037 | TFRC | transferrin receptor (p90, CD71) |
| 1.4 | 54934 | FLJ20436 | hypothetical protein FLJ20436 |
| 1.4 | 5144 | PDE4D | phosphodiesterase 4D, cAMP-specific (phosphodiesterase E3 dunce homolog, Drosophila) |
| 1.4 | 9929 | KIAA0063 | KIAA0063 gene product |
| 1.4 | 143187 | VTI1A | Vesicle transport through interaction with t-SNAREs homolog 1A (yeast) |
| 1.4 | 440309 | --- | LOC440309 |
| 1.4 | 150094 | SNF1LK | SNF1-like kinase /// SNF1-like kinase |
| 1.4 | 1850 | DUSP8 | dual specificity phosphatase 8 |
| 1.4 | 9584 | RNPC2 | RNA-binding region (RNP1, RRM) containing 2 |
| 1.4 | 140735 | Dlc2 | dynein light chain 2 |
| 1.4 | 54542 | MNAB | membrane associated DNA binding protein |
| 1.4 | 9262 | STK17B | serine/threonine kinase 17b (apoptosis-inducing) |
| 1.4 | 7128 | TNFAIP3 | tumor necrosis factor, alpha-induced protein 3 |
| 1.4 | 3183 | HNRPC | heterogeneous nuclear ribonucleoprotein C (C1/C2) |
| 1.4 | 5144 | PDE4D | Phosphodiesterase 4D, cAMP-specific (phosphodiesterase E3 dunce homolog, Drosophila) |
| 1.4 | 80311 | KLHL15 | kelch-like 15 (Drosophila) |
| 1.4 | 22850 | KIAA0863 | KIAA0863 protein |
| 1.4 | 5996 | RGS1 | --- |
| 1.4 | 468 | ATF4 | activating transcription factor 4 (tax-responsive enhancer element B67) |
| 1.4 | --- | --- | --- |
| 1.4 | 7430 | VIL2 | villin 2 (ezrin) |
| 1.4 | 6627 | SNRPA1 | small nuclear ribonucleoprotein polypeptide A' |
| 1.4 | 7750 | ZNF198 | zinc finger protein 198 |
| 1.4 | 1390 | CREM | cAMP responsive element modulator |
| 1.4 | 10291 | SF3A1 | splicing factor 3a, subunit 1, 120kDa |
| 1.4 | 9308 | CD83 | CD83 antigen (activated B lymphocytes, immunoglobulin superfamily) |
| 1.4 | 63935 | C20orf67 | --- |
| 1.4 | 10049 | DNAJB6 | DnaJ (Hsp40) homolog, subfamily B, member 6 |
| 1.4 | 51526 | C20orf111 | chromosome 20 open reading frame 111 |
| 1.4 | 55500 | ETNK1 | ethanolamine kinase 1 /// ethanolamine kinase 1 |
| 1.4 | 79441 | C4orf15 | chromosome 4 open reading frame 15 |
| 1.4 | 11236 | RNF139 | ring finger protein 139 |
| 1.4 | 246243 | RNASEH1 | ribonuclease H1 |
| 1.4 | 3727 | JUND | jun D proto-oncogene |
| 1.4 | 6500 | SKP1A | S-phase kinase-associated protein 1A (p19A) |
| 1.4 | 4204 | MECP2 | Methyl CpG binding protein 2 (Rett syndrome) |
| 1.4 | 3189 | HNRPH3 | heterogeneous nuclear ribonucleoprotein H3 (2H9) |
| 1.4 | 222161 | DKFZp586I1420 | hypothetical protein DKFZp586I1420 |
| 1.4 | 266812 | NAP1L5 | nucleosome assembly protein 1-like 5 |
| 1.4 | 9908 | G3BP2 | Ras-GTPase activating protein SH3 domain-binding protein |
| | | | 2 |
| 1.4 | 10425 | ARIH2 | --- |
| 1.4 | 55422 | ZNF331 | Zinc finger protein 331 |
| 1.4 | 8454 | CUL1 | Cullin 1 |
| 1.4 | 51119 | SBDS | Shwachman-Bodian-Diamond syndrome |
| 1.4 | 6309 | SC5DL | sterol-C5-desaturase (ERG3 delta-5-desaturase homolog, fungal)-like |
| 1.4 | 5277 | PIGA | phosphatidylinositol glycan, class A (paroxysmal nocturnal hemoglobinuria) /// phosphatidylinositol glycan, class A (paroxysmal nocturnal hemoglobinuria) |
| 1.4 | 3422 | IDI1 | isopentenyl-diphosphate delta isomerase |
| 1.4 | 63935 | C20orf67 | chromosome 20 open reading frame 67 |
| 1.4 | 7975 | MAFK | v-maf musculoaponeurotic fibrosarcoma oncogene homolog K (avian) |
| 1.4 | 7456 | WASPIP | Wiskott-Aldrich syndrome protein interacting protein |
| 1.4 | 55975 | KLHL7 | kelch-like 7 (Drosophila) |
| 1.4 | 7128 | TNFAIP3 | tumor necrosis factor, alpha-induced protein 3 |
| 1.4 | 388796 | LOC388796 | hypothetical LOC388796 |
| 1.4 | 25852 | ARMC8 | armadillo repeat containing 8 |
| 1.4 | 54542 | MNAB | Membrane associated DNA binding protein |
| 1.4 | 55422 | ZNF331 | zinc finger protein 331 |
| 1.4 | 1390 | CREM | cAMP responsive element modulator |
| 1.4 | 10209 | SUI1 | putative translation initiation factor |
| 1.4 | 10049 | DNAJB6 | DnaJ (Hsp40) homolog, subfamily B, member 6 |
| 1.4 | 4929 | NR4A2 | nuclear receptor subfamily 4, group A, member 2 |
| 1.4 | 1540 | CYLD | cylindromatosis (turban tumor syndrome) |
| 1.4 | 4929 | NR4A2 | nuclear receptor subfamily 4, group A, member 2 |
| 1.4 | 5805 | PTS | 6-pyruvoyltetrahydropterin synthase |
| 1.4 | 10926 | ASK | activator of S phase kinase |
| 1.4 | 10923 | PC4 | activated RNA polymerase II transcription cofactor 4 /// similar to Activated RNA polymerase II transcriptional coactivator p15 (Positive cofactor 4) (PC4) (p14) |
| 1.4 | 388796 | RNU71A | Hypothetical LOC388796 |
| 1.4 | 133746 | JMY | junction-mediating and regulatory protein |
| 1.4 | 90634 | CG018 | Hypothetical gene CG018 |
| 1.4 | 10209 | SUI1 | putative translation initiation factor |
| 1.4 | 1847 | DUSP5 | dual specificity phosphatase 5 |
| 1.4 | 7088 | TLE1 | Transducin-like enhancer of split 1 (E(sp1) homolog, Drosophila) |
| 1.4 | 84275 | MGC4399 | mitochondrial carrier protein |
| 1.4 | --- | --- | --- |
| 1.4 | 7803 | PTP4A1 | protein tyrosine phosphatase type IVA, member 1 |
| 1.4 | 55422 | ZNF331 | zinc finger protein 331 |
| 1.4 | --- | --- | CDNA clone IMAGE:30332316, partial cds |
| 1.4 | 3609 | ILF3 | interleukin enhancer binding factor 3, 90kDa |
| 1.4 | --- | --- | Homo sapiens, clone IMAGE:4753714, mRNA |
| 1.4 | 6651 | SON | SON DNA binding protein |
| 1.4 | 11276 | AP1GBP1 | AP1 gamma subunit binding protein 1 |
| 1.4 | 84124 | ZNF394 | zinc finger protein 3 94 |
| 1.4 | 63935 | C20orf67 | --- |
| 1.4 | 1983 | EIF5 | eukaryotic translation initiation factor 5 |
| 1.4 | 80063 | ATF7IP2 | Activating transcription factor 7 interacting protein 2 |
| 1.4 | 285831 | LOC285831 | hypothetical protein LOC285831 |
| 1.4 | 81873 | ARPC5L | actin related protein 2/3 complex, subunit 5-like |
| 1.4 | 144438 | LOC144438 | hypothetical protein LOC144438 |
| 1.4 | 10209 | SUI1 | putative translation initiation factor |
| 1.4 | 3021 | H3F3B | H3 histone, family 3B (H3.3B) |
| 1.4 | 25948 | KBTBD2 | kelch repeat and BTB (POZ) domain containing 2 |
| 1.4 | --- | --- | CDNA FLJ40725 fis, clone TKIDN1000001, highly similar to Translocase of inner mitochondrial membrane 23 |
| 1.4 | 1540 | CYLD | cylindromatosis (turban tumor syndrome) |
| 1.4 | 5144 | PDE4D | phosphodiesterase 4D, cAMP-specific (phosphodiesterase E3 dunce homolog, Drosophila) |
| 1.4 | 51182 | HSPA14 | heat shock 70kDa protein 14 |
| 1.4 | 29080 | HSPC128 | HSPC128 protein |
| 1.4 | 8731 | RNMT | RNA (guanine-7-) methyltransferase |
| 1.4 | 3423 | IDS | iduronate 2-sulfatase (Hunter syndrome) |
| 1.4 | 283991 | MGC29814 | hypothetical protein MGC29814 |
| 1.4 | 1454 | CSNK1E | Casein kinase 1, epsilon |
| 1.4 | 26051 | PPP1R16B | protein phosphatase 1, regulatory (inhibitor) subunit 16B |
| 1.4 | 3422 | IDI1 | isopentenyl-diphosphate delta isomerase |
| 1.4 | 5887 | RAD23B | RAD23 homolog B (S. cerevisiae) |
| 1.4 | 5144 | PDE4D | Phosphodiesterase 4D, cAMP-specific (phosphodiesterase E3 dunce homolog, Drosophila) |
| 1.4 | 49854 | ZNF295 | zinc finger protein 295 |
| 1.4 | 60493 | FLJ13149 | hypothetical protein FLJ13149 |
| 1.4 | 10950 | BTG3 | BTG family, member 3 |

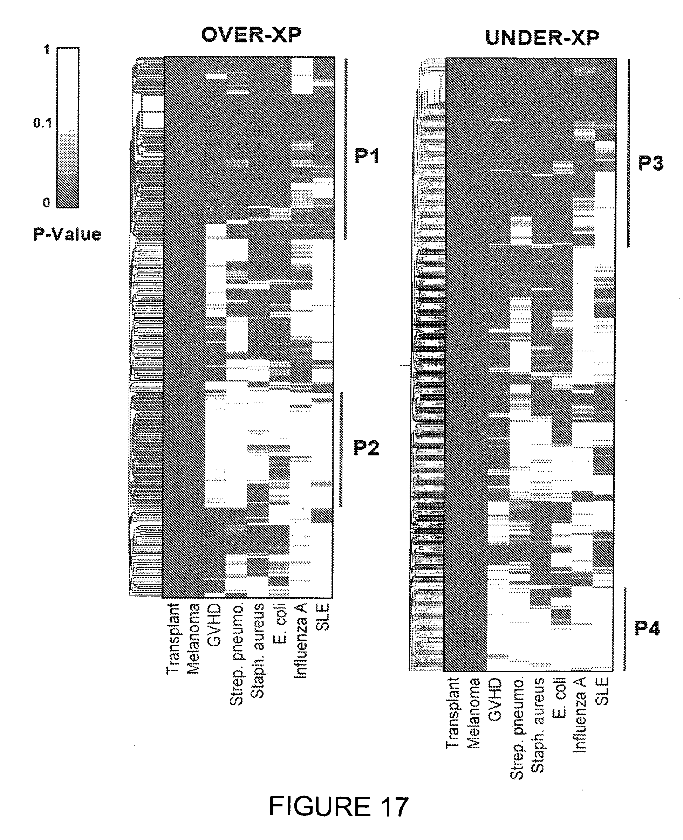

Yet another group that may be used alone or in combination with the genes listed in supplementary table that includes the data shown in Figure 17, denoted P2, and which may include one or more the following genes that are overexpressed, e.g., WARS; IFI53; IFP53; GAMMA-2; FAM46C; FLJ20202; H3F3B; H3.3B; FOXK2; ILF; ILF1; ILF-1; DUSP5; HVH3; ARF6; DKFZp762C186; BRD2; NAT; RNF3; FSRG1; RING3; D6S113E; KIAA9001; RORA; ROR1; ROR2; ROR3; RZRA; NR1F1; DKFZp762C186; DNAJB1; SUI1; CXCR4; HM89; LAP3; NPYR; WHIM; LESTR; NPY3R; HSY3RR; NPYY3R; D2S201E; GRINL1A; CTSB; TRIP-Br2; PDE4B; DPDE4; PDEIVB; PMAIP1; APR; NOXA; BTG2; PC3; TIS21; ASAHL; SON; SUI1; A121; ISO1; HERPUD1; SUP; Mif1; KIAA0025; DUSP2; PAC1; PAC-1; RNF139; RCA1; TRC8; HRCA1; MGC31961; TNFAIP3; A20; TNFA1P2; ARS2; HNRPL; hnRNP-L; P/OKc1.14; C20orf67; C20orf111; HSPC207; dJ1183I21.1; ZNF331; RITA; ZNF361; ZNF463; C20orf67; IER5; SBBI48; ; SUI1; JUN; AP1; CD69; TOB1; H3F3B; H3.3B; FOLR1; TNFAIP3; TCF8; BZP; ZEB; ZEB1; AREB6; ZFHEP; NIL-2A; ZFHX1A; NIL-2-A; DUSP10; MKP5; MKP-5; GGTLA4; MGC50550; dJ831C21.2; PMAIP1; ZC3HAV1; ZAP; FLB6421; ZC3HDC2; FLJ13288; MGC48898; DSIPI; DIP; GILZ; hDIP; TSC-22R; MCL1; TM; EAT; MCL1L; MCL1S; MGC1839; SH3TC1; FLJ20356; CIAS1; FCU; MWS; FCAS; NALP3; Clorf7; PYPAF1; AII/AVP; AGTAVPRL; SLC15A3; PHT2; PTR3; hPTR3; PTDSR; PSR; PTDSR1; KIAA0585; BHLHB2; DEC1; STRA13; Stra14; HMGE; KIAA0063; NR4A2; NOT; RNR1; HZF-3; NURR1; TINUR; NR4A2; NOT; RNR1; HZF-3; NURR1; TINUR; PTS; PTPS; HEAB; CLP1; hClp1; AREG; SDGF; CRDGF; MGC13647; EDG4; LPA2; EDG-4; LPAR2; CREM; ICER; MGC17881; MGC41893; CD83; BL11; HB15; ZNF394; FLJ12298, and combinations thereof.

Module-based microarray data mining strategy. Results from "traditional" microarray analyses are notoriously noisy and difficult to interpret. A widely accepted approach for microarray data analyses includes three basic steps: 1) Use of a statistical test to select genes differentially expressed between study groups; 2) Apply pattern discovery algorithms to identify signatures among the resulting gene lists; and 3) Interpret the data using knowledge derived from the literature or ontology databases.

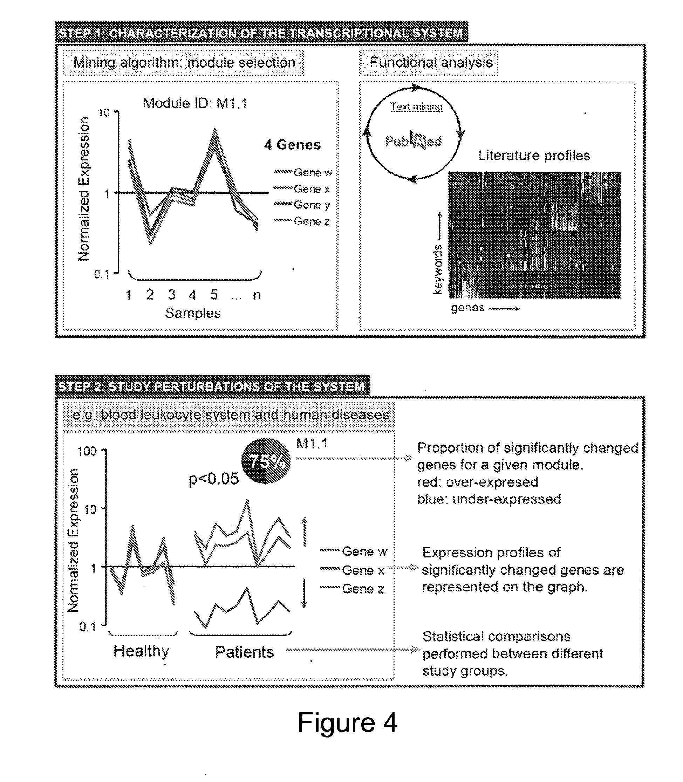

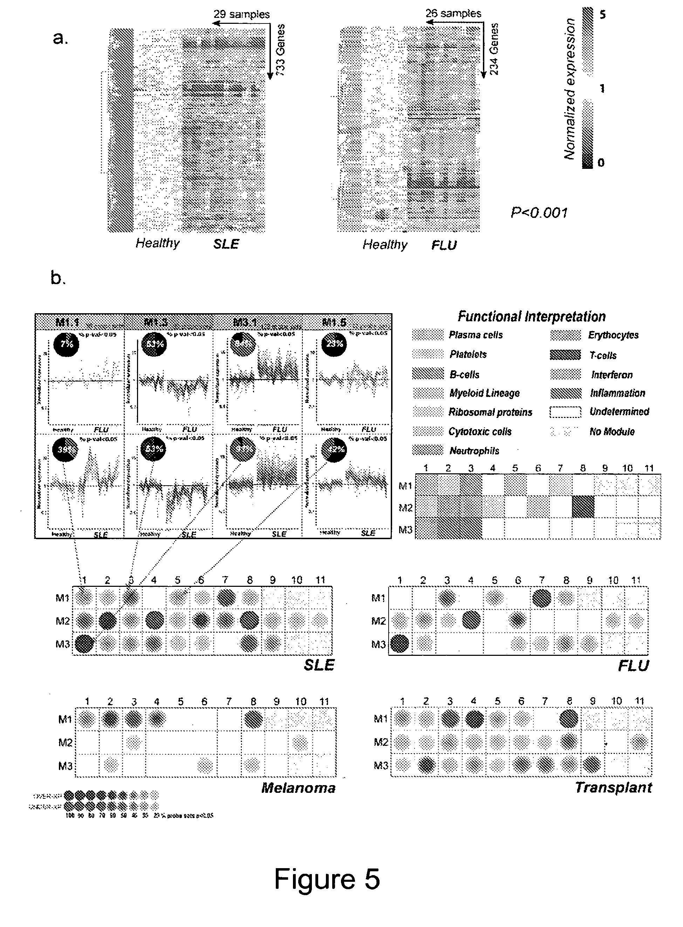

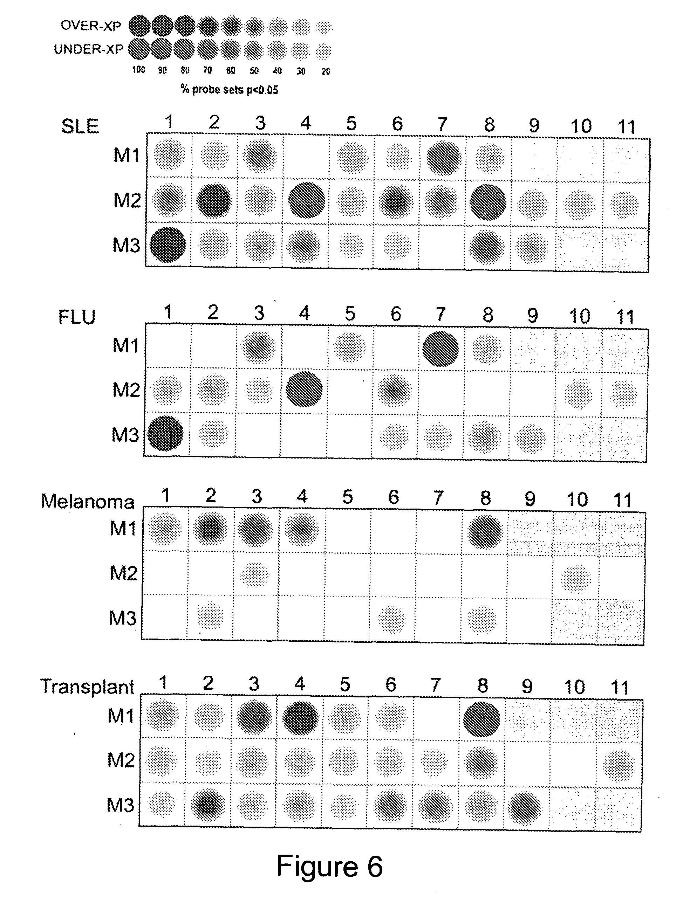

The present invention uses a novel microarray data mining strategy emphasizing the selection of biologically relevant transcripts at an early stage of the analysis. This first step can be carried out using for instance the modular mining algorithm described above in combination with a functional mining tool used for in-depth characterization of each transcriptional module (FIGURE 4: top panel, Step 1). The analysis does not take into consideration differences in gene expression levels between groups. Rather, the present invention focuses instead on complex gene expression patterns that arise due to biological variations (e.g., inter-individual variations among a patient population). After defining the transcriptional components associated to a given biological system the second step of the analysis includes the analysis of changes in gene expression through the comparison of different study groups (FIGURE 4: bottom panel, Step 2). Group comparison analyses are carried out independently for each module. Changes at the module level are expressed as the proportion of genes that meet the significance criteria (represented by a pie chart in FIGURE 5 or a spot in FIGURE 6). Notably, carrying out comparisons at the modular level permits to avoid the noise generated when thousands of tests are performed on "random" collections of genes.

Perturbation of modular PBMC transcriptional profiles in human diseases. To illustrate the second step of the microarray data mining strategy described above (FIGURE 4), gene expression data for PBMC samples obtained from two pediatric patient populations composed of eighteen children with systemic lupus erythematosus (SLE) and sixteen children with acute influenza A infection was obtained, compared and analyzed. Each patient cohort was matched to its respective control group (healthy volunteers: eleven and ten donors were matched to the SLE and influenza groups, respectively). Following the analytical scheme depicted in FIGURE 4, a statistical group comparisons between patient and healthy groups for each individual module and measured the proportion of genes significantly changed in each module (FIGURE 5) was performed. The statistical group comparison approach allows the user to focus the analysis on well defined groups of genes that contain minimal amounts of noise and carry identifiable biological meaning. A key to the graphical representation of these results is provided in Figure 4.

The following findings were made: (1) that a large proportion of genes in M3.1 ("interferon-associated") met the significance level in both Flu and SLE groups (84% and 94%, respectively). This observation confirms earlier work with SLE patients ¹⁹ and identifies the presence of an interferon signature in patients with acute influenza infection. (2) Equivalent proportions of genes in M1.3 ("B-cell-associated") were significantly changed in both groups (53%), with over 50% overlap between the two lists. This time, genes were consistently under-expressed in patient compared to healthy groups. (3) Modules were also found that differentiate the two diseases. The proportion of genes significantly changed in Module 1.1 reaches 39% in SLE patients and is only 7% in Flu patients, which at a significance level of 0.05 is very close to the proportion of genes that would be expected to be differentially expressed only by chance. Interestingly, this module is almost exclusively composed of genes encoding immunoglobulin chains and has been associated with plasma cells. However, this module is clearly distinct from the B-cell associated module (M1.3), both in terms of gene expression level and pattern (not shown). (4) As illustrated by module M1.5, gene-level analysis of individual modules can be used to further discriminate the two diseases. It is also the case for M1.3, where, despite the absence of differences at the module-level (FIGURE 4: 53% under-expressed transcripts), differences between Flu and SLE groups could be identified at the gene-level (only 51% of the under-expressed transcripts in M1.3 were common to the two disease groups). These examples illustrate the use of a modular framework to streamline the analysis and interpretation of microarray results.

Mapping changes in gene expression at the modular level. Data visualization is paramount for the interpretation of complex datasets and the present invention includes a comprehensive graphical illustration of changes that occur at the modular level. Changes in gene expression levels caused by different diseases were represented for the twenty-eight PBMC transcriptional modules (FIGURE 6). Each disease group is compared to its respective control group composed of healthy donors who were matched for age and sex (eighteen patients with SLE, sixteen with acute influenza infection, sixteen with metastatic melanoma and sixteen liver transplant recipients receiving immunosuppressive drug treatment were compared to control groups composed of ten to eleven healthy subjects). Module-level data were represented graphically by spots aligned on a grid, with each position corresponding to a different module (See Table 1 for functional annotations on each of the modules).

The spot intensity indicates the proportion of genes significantly changed for each module. The spot color indicates the polarity of the change (red: proportion of over-expressed genes, blue: proportion of under-expressed genes; modules containing a significant proportion of both over- and under-expressed genes would be purple-though none were observed). This representation permits a rapid assessment of perturbations of the PBMC transcriptional system. Such "module maps" were generated for each disease. When comparing the four maps, we found that diseases were characterized by a unique modular combination. Indeed, results for M1.1 and M1.2 alone sufficed to distinguish all four diseases (M1.1/M1.2: SLE = +/+; FLU=0/0; Melanoma=-/+; transplant=-/-). A number of genes in M3.2 ("inflammation") were over-expressed in all diseases (particularly so in the transplant group), while genes in M3.1 (interferon) were over-expressed in patients with SLE, influenza infection and, to some extent, transplant recipients. "Ribosomal protein" module genes (M1.7 and M2.4) were under-expressed in both SLE and Flu groups. The level of expression of these genes was recently found to be inversely correlated to disease activity in SLE patients (Bennett *et al.,* submitted). M2.8 includes T-cell transcripts which are under-expressed in lymphopenic SLE patients and transplant recipients treated with immunosuppressive drugs targeting T-cells.

Interestingly, differentially expressed genes in each module were predominantly either under-expressed or over-expressed (FIGURE 5 and FIGURE 6). Yet, modules were purely selected on the basis of similarities in gene expression profiles, not changes in expression levels between groups. The fact that changes in gene expression appear highly polarized within each module denotes the functional relevance of modular data. Thus, the present invention enables disease fingerprinting by a modular analysis of patient blood leukocyte transcriptional profiles.

Validation of PBMC modules in a published dataset. Next, the validity of the PBMC transcriptional modules described above in a "third-party" dataset was tested. The study from Connolly, et al., who investigated the effects of exercise on gene expression in human PBMCs²⁰ was tested.

Blood samples were obtained from 35 patients with metastatic melanoma enrolled in three phase I/II clinical trials designed to test the efficacy of a dendritic cell therapeutic vaccine as seen in the table below. Gene expression signatures were generated from blood samples collected prior to the initiation of vaccine therapy, and at least 4 weeks after the last systemic therapy if a patient had undergone such.

**Table 3: Clinical and demographic characteristics of 35 patients with metastatic melanoma**

| ID | Sex | Age | Stage | Diagnosis | Time from Dx to Blood Draw (Months) | Blood Draw | Status | Blood Draw to Time of Death (Months) |
|---|---|---|---|---|---|---|---|---|
| MEL 23 | F | 61 | M1a | 02/14/00 | 16 | 06/28/01 | Deceased | 28 |
| MEL 24 | M | 53 | M1c | 09/01/99 | 22 | 07/05/01 | Deceased | 5 |
| MEL 26 | F | 52 | M1c | 10/01/97 | 45 | 07/18/01 | Deceased | 2 |
| MEL 27 | F | 54 | M1a | 07/10/93 | 98 | 09/14/01 | Deceased | 5 |
| MEL 29 | M | 41 | M1c | 10/26/94 | 83 | 09/26/01 | Deceased | 14 |
| MEL 30 | F | 58 | M1c | 10/04/99 | 23 | 09/25/01 | Deceased | 11 |
| MEL 32 | M | 56 | M1b | 01/17/94 | 95 | 12/17/01 | Deceased | 24 |
| MEL 34 | F | 28 | M1b | 04/01/01 | 10 | 02/05/02 | Deceased | 42 |
| MEL 35 | M | 29 | M1a | 08/25/98 | 43 | 03/12/02 | Deceased | 12 |
| MEL 36 | F | 69 | M1b | 01/01/85 | 205 | 02/19/02 | Deceased | 7 |
| MEL 40 | F | 43 | M1c | 07/02/91 | 132 | 07/19/02 | Deceased | 19 |
| MEL 43 | M | 60 | M1a | 08/14/94 | 100 | 12/04/02 | Alive 05/16/05 | * |
| MEL 44 | F | 68 | M1c | 05/01/99 | 44 | 01/28/03 | Deceased | 12 |
| MEL 45 | F | 53 | M1a | 02/01/02 | 10 | 12/17/02 | Alive 5/5/05 | * |
| MEL 46 | F | 47 | M1c | 11/18/97 | 61 | 12/27/02 | Deceased | 22 |
| MEL 47 | F | 35 | M1c | 03/02/02 | 10 | 01/09/03 | Deceased | 2 |
| MEL 48 | M | 68 | M1b | *1992 | >120 | 03/12/03 | Alive 05/10/05 | * |
| MEL 49 | M | 71 | M1b | 12/05/97 | 64 | 04/10/03 | Alive 07/05/05 | * |
| MEL 50 | M | 52 | M1c | 07/08/97 | 69 | 04/11/03 | Deceased | 18 |
| MEL 51 | M | 56 | M1c | 10/01/01 | 18 | 04/16/03 | Alive 05/17/05 | * |
| MEL 52 | M | 42 | M1c | 03/01/02 | 13 | 04/17/03 | Deceased | 9 |
| MEL 54 | M | 50 | M1b | 07/20/90 | 153 | 04/25/03 | Alive 04/08/05 | * |
| MEL 56 | M | 71 | M1c | 03/01/01 | 26 | 05/29/03 | Deceased | 9 |
| MEL 57 | F | 36 | M1b | 07/01/02 | 11 | 06/05/03 | Deceased | 20 |
| MEL 58 | M | 67 | M1c | 10/01/99 | 45 | 07/18/03 | Deceased | 10 |
| MEL 59 | M | 61 | M1c | unknown | * | 07/25/03 | Alive 06/22/05 | * |
| MEL 60 | M | 41 | M1c | 11/01/02 | 9 | 08/14/03 | Deceased | 7 |
| MEL 61 | F | 54 | M1a | 03/03/99 | 54 | 09/10/03 | Alive 05/18/05 | * |
| MEL 62 | M | 46 | M1b | 12/01/01 | 22 | 10/09/03 | Deceased | 5 |
| MEL 63 | M | 75 | M1b | 12/01/00 | 34 | 10/29/03 | Alive 03/16/05 | * |
| MEL 64 | F | 53 | M1b | 04/01/00 | 42 | 10/30/03 | Alive 03/05/04 | * |
| MEL 65 | M | 62 | M1b | 08/14/94 | 111 | 11/14/03 | Alive 05/16/05 | * |
| MEL 68 | M | 74 | M1b | 06/09/04 | 1 | 07/29/04 | Deceased | 9 |
| MEL 70 | M | 67 | M1b | 04/06/04 | 5 | 09/23/04 | Alive 8/18/2005 | * |
| MEL 72 | M | 50 | M1c | 09/23/04 | 2 | 11/10/04 | Deceased | * |

The Table provides both clinical and demographic characteristics of 35 patients with metastatic melanoma.

A second group of patients included 39 liver transplant recipients maintaining their graft under pharmacological immunosuppressive therapy, time from transplant had a median of 729 days and a range of between 338 and 1905 days. Outpatients coming for routine exams were recruited for this study. All patients received standard treatment regimens with calcineurin inhibitors (e.g., Tacrolimus: n=25; Cyclosporin A: n=13). The main indications for liver transplant were hepatitis C (n = 19) and Laennec's cirrhosis (n = 7). The table below provides both clinical and demographic characteristics of 39 liver transplant recipients.

**Table 4: Clinical and demographic characteristics of 39 liver transplant recipients**

| Patient ID | Age | Sex | Transpl. to Blood Draw (Days) | TAC | CsA | Primary Dx |
|---|---|---|---|---|---|---|
| R1292 | 47 | M | 1854 | yes | | Hepatitis C |
| R1297 | 48 | M | 1868 | | yes | Hepatitis C |
| R1308 | 66 | F | 1905 | | yes | Primary Biliary Cirrhosis |
| R1322 | 32 | F | 1821 | | yes | Hepatitis C |
| R1323 | 45 | M | 1828 | | yes | Hepatitis C |
| R1325 | 50 | M | 1801 | | yes | Hepatitis C |
| R1329 | 51 | F | 1781 | yes | | Laennec's Cirrhosis |
| R1340 | 50 | M | 1829 | | yes | Laennec's Cirrhosis |
| R1348 | 64 | F | 1802 | yes | | Fulminant Hepatic Failure |
| R1355 | 61 | M | 1780 | yes | | Cryptogenic |
| R1364 | 42 | M | 1756 | yes | | Hepatitis C |
| R1413 | 52 | M | 1856 | | yes | Laennec's Cirrhosis |
| R1673 | 60 | F | 756 | yes | | Hepatitis B |
| R1674 | 45 | M | 729 | yes | | Hepatitis C |
| R1684 | 65 | F | 721 | yes | | Mx. Carcinoid |
| R1686 | 59 | M | 704 | yes | | Hepatitis B |
| R1689 | 43 | M | 692 | yes | | Hepatitis C |
| R1700 | 53 | M | 732 | | yes | Hepatitis C |
| R1701 | 45 | M | 737 | yes | | Hepatitis C |
| R1702 | 48 | M | 726 | yes | | Hepatitis C |
| R1706 | 57 | M | 721 | yes | | Laennec's Cirrhosis |
| R1710 | 50 | F | 736 | yes | | Cryptogenic |
| R1714 | 63 | F | 718 | yes | | Nonalcoholic Steatohepatitis |
| R1718 | 53 | F | 707 | | yes | Hepatitis C |
| R1754 | 51 | F | 589 | yes | | Primary Sclerosing Cholangitis |
| R1771 | 42 | F | 812 | | | Hepatitis C |
| R1787 | 60 | F | 794 | yes | | Laennec's Cirrhosis |
| R1805 | 42 | M | 735 | yes | | Laennec's Cirrhosis & Postnecrotic Cirrhosis Type C |
| R1814 | 45 | M | 427 | yes | | Hepatitis C |
| R1838 | 66 | F | 360 | yes | | Autoimmune Hepatitis |
| R1839 | 56 | M | 354 | yes | | Cryptogenic |
| R1841 | 52 | M | 363 | yes | | PSC-UC |
| R1843 | 48 | M | 361 | | yes | Hepatitis C |
| R1845 | 44 | F | 338 | yes | | Primary Biliary Cirrhosis |
| R1846 | 41 | F | 338 | | yes | Hepatitis C |
| R1847 | 53 | M | 358 | yes | | Laennec's Cirrhosis |
| R1854 | 50 | M | 350 | | yes | Hepatitis C |
| R1971 | 46 | M | 367 | | yes | Hepatitis C; Hepatocellular Carcinoma with Cirrhosis |
| R1974 | 54 | M | 341 | yes | | Hepatitis C |

Blood samples were also obtained from 25 healthy donors that constituted the control groups. The table below provides the demographic characteristics of the 25 healthy donors.

**Table 5: Demographic characteristics of 25 healthy donors**

| Healthy Volunteers | Sex | Age |
|---|---|---|
| D-001 | M | 41 |
| D-002 | F | 53 |
| D-005 | F | 40 |
| D-007 | F | 44 |
| D-008 | M | 40 |
| D-010 | M | 46 |
| D-011 | F | 43 |
| D-013 | M | 58 |
| D-014 | F | 47 |
| D-015 | M | 42 |
| D-016 | F | 40 |
| D-017 | M | 25 |
| D-018 | M | 46 |
| D-019 | F | 40 |
| D-020 | M | 39 |
| D-021 | M | 45 |
| D-022 | M | 50 |
| D-024 | F | 44 |
| D-025 | F | 48 |
| D-027 | F | 43 |
| D-028 | F | 43 |
| D-029 | M | 43 |
| D-031 | M | 35 |
| D-032 | F | 43 |
| D-033 | F | 43 |

Identification of disease-associated blood leukocyte transcriptional signatures. Blood leukocyte gene expression signatures were identified in patients with metastatic melanoma and liver transplant recipients. Each set of patients was compared to a control group of healthy volunteers. Patient samples were divided into a training set, used to identify disease-associated and predictive expression signatures, and an independent test set. This step-wise analysis allowed validation of results in samples that were not used to establish the disease signature. Stringent criteria were employed to select the samples forming training sets in order to avoid confounding the analysis with biological and/or technical factors. The table below illustrates the composition of sample sets taking into account age, gender and sample processing method used for the identification (training) and validation (testing) of expression signatures associated with metastatic melanoma.

**Table 6: Composition of sample sets associated with metastatic melanoma.**

| **Training Set** | | | | | **Test** | | | | Total n |
|---|---|---|---|---|---|---|---|---|---|
| **HV** | | Fresh | Frozen | | | Fresh | Frozen | | |
| | Male | 7 | 7 | 14 | Male | 5 | 0 | 5 | |
| | Female | 0 | 9 | 9 | Female | 8 | 0 | 8 | |
| | | 7 | 16 | 23 | | 13 | 0 | 13 | 36 |
| | | | | | | | | | |

| **Melanoma** | | Fresh | Frozen | | | Fresh | Frozen | | |
|---|---|---|---|---|---|---|---|---|---|
| | Male | 3 | 9 | 12 | Male | 0 | 11 | 11 | |
| | Female | 0 | 10 | 10 | Female | 0 | 5 | 5 | |
| | | 3 | 19 | 22 | | 0 | 16 | 16 | 38 |
| | | | Total | 45 | | | Total | 29 | |

Similarly, the table below provides the composition of sample sets used taking into account age, gender and sample processing method for the identification (training) and validation (testing) of expression signatures associated with liver transplant recipients undergoing immunosuppressive drug therapy.

**Table 7: Composition of sample sets associated with liver transplant recipients undergoing immunosuppressive drug therapy.**

| **Training Set** | | | | | **Test** | | | | Total n |
|---|---|---|---|---|---|---|---|---|---|
| **HV** | | Fresh | Frozen | | | Fresh | Frozen | | |
| | Male | 12 | 5 | 17 | Male | 0 | 2 | 2 | |
| | Female | 8 | 2 | 10 | Female | 0 | 7 | 7 | |
| | | 20 | 7 | 27 | | 0 | 9 | 9 | 36 |
| | | | | | | | | | |
| **LTx** | | Fresh | Frozen | | | Fresh | Frozen | | |
| | Male | 9 | 3 | 12 | Male | 15 | 0 | 15 | |
| | Female | 9 | 1 | 10 | Female | 6 | 0 | 6 | |
| | | 18 | 4 | 22 | | 21 | 0 | 21 | 43 |
| | | | Total | 49 | | | Total | 30 | |

Table 8 lists the genes differentially expressed in patients with metastatic melanoma in comparison to healthy volunteers. Statistical comparison of a group of twenty-two patients with metastatic melanoma versus twenty-three healthy volunteers, training set, identified 899 differentially expressed genes (p<0.01, non parametric Mann-Whitney rank test and >1.25 fold change; 218 overexpressed and 681 underexpressed genes). The Tables provide a correlation to the modules from which there were identified, their expression level, various nomenclatures for their individual identification.

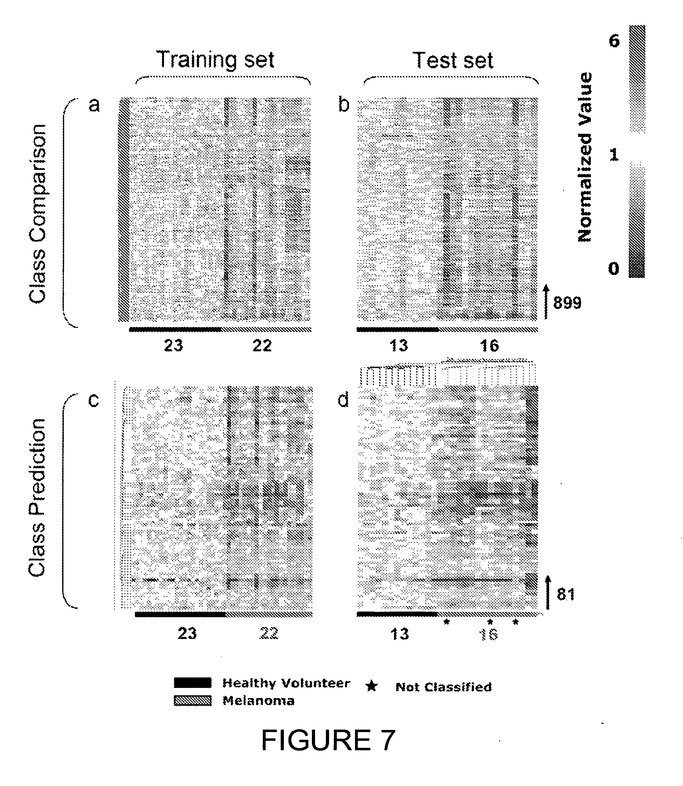

FIGURES 7A-7D are images of the hierarchical clustering of genes. FIGURE 7A illustrates the hierarchical clustering of genes that produce a reciprocal expression pattern; which was confirmed in an independent test set shown in FIGURE 7B. FIGURE 7B displays results from 13 healthy volunteers versus 16 patients. Next, class prediction algorithms were applied to the initial training set. These algorithms yielded 81 genes with the best ability to classify healthy volunteers and patients based on their differential expression as shown in FIGURE 7C and Table 9). Table 9 illustrates the expression levels of a set of transcripts discriminating patients with melanoma from healthy volunteers. Using these 81 genes, an independent test set was classified with 90% accuracy; the class of only three was indeterminate as illustrated in FIGURE 7D.

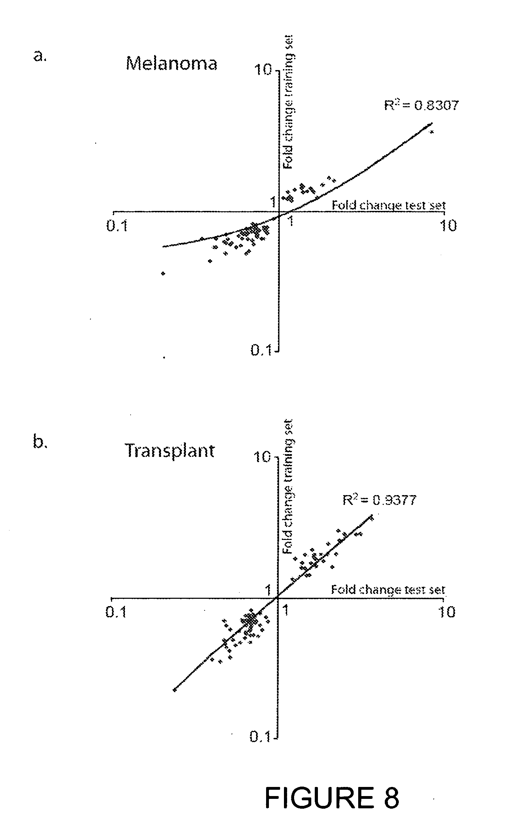

FIGURES 8A and 8B are plots of the microarray results in an independent set of samples to confirm the reliability of the results by correlating expression levels obtained for the training and test sets. Genes with the best ability to discriminate between patients and healthy volunteers were identified in a training set using a class prediction algorithm (k-Nearest Neighbors). Fold change expression levels between healthy controls and patients were measured for discriminative genes in the training set and an independent test set. Fold change values obtained in training and test sets were correlated: FIGURE 8A illustrates results for metastatic melanoma and produced 81 genes with a Pearson correlation of r²=0.83 and a p<0.0001 and FIGURE 8B illustrates results for liver transplant recipients and produced 65 genes with a Pearson correlation of r²=0.94 and a p<0.0001.

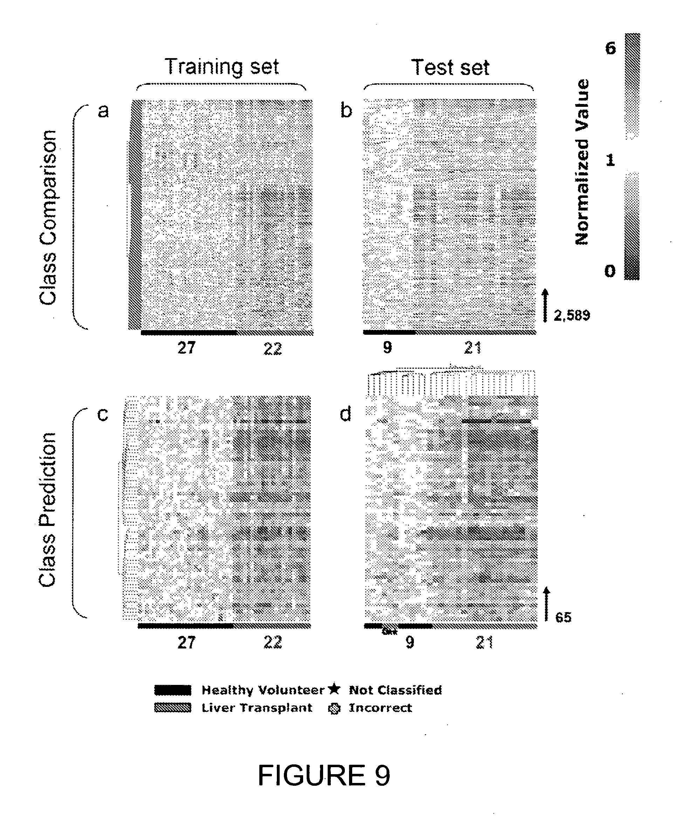

FIGURES 9A-9D are images of the hierarchical clustering of genes for the identification of a blood leukocyte transcriptional signature in transplant recipients under immunosuppressive drug therapy. Samples were divided into a training set (27 healthy, 22 patients) used to identify differentially expressed genes in liver transplant recipients versus healthy volunteers as seen in FIGURE 9A and FIGURE 9C respectively. A test set of nine healthy and 21 patients were used to independently validate this signature as seen in FIGURE 9B and FIGURE 9D. Class comparison identified 2,589 differentially expressed genes (Mann-Whitney test p < 0.01, fold change > 1.25). FIGURE 9A illustrates a similar signature in the test set and FIGURE 9B illustrates the class prediction that identified 81 genes. FIGURE 9C shows that the discrimination of the independent test set with 90% accuracy. FIGURE 9D illustrates the class could not be identified for two samples out of 30; one sample was incorrectly predicted (i.e. transplant recipient classified as healthy).

The same analysis strategy was applied to Liver transplant patients. Statistical comparison of a group of 22 transplant recipients versus 27 healthy volunteers identified 2,589 differentially expressed genes (p<0.01, non-parametric Mann-Whitney rank test and >1.25 fold change; 938 overexpressed and 1651 underexpressed genes; Table 10). Table 10 illustrates genes that are expressed differentially in liver transplant recipients under treatment with immunosuppressive drugs in comparison to healthy volunteers. Hierarchical clustering of genes produced a reciprocal expression pattern that was observed in both training as seen in FIGURE 9A and independent test sets as seen in FIGURE 9B. Sixty-five classifier genes were established in the training set and are illustrates in FIGURE 9C and Table 11. Table 11 illustrates the expression level of a set of transcripts discriminating liver transplant recipients under treatment with immunosuppressive drugs from healthy volunteers. The sixty-five classifier genes were applied to an independent test set of 9 healthy donors and 21 patients. Samples were correctly classified 90% of the time: class could not be determined in two cases and one sample was misclassified as seen in FIGURE 9D. The results obtained for both of these sets were highly correlated, e.g., pearson correlation, r²=0.94, p<0.0001, as seen in FIGURE 8B. Thus, the blood leukocyte transcriptional signatures associated with patients with metastatic melanoma and in liver transplant recipients have been identified and validated.

Module-level analysis of patients PBMC transcriptional profiles was performed. A custom microarray data mining strategy was used to further characterize disease-associated gene expression patterns. The analysis of a set of 239 blood leukocytes transcriptional signatures identified 28 transcriptional modules regrouping 4,742 probe sets. These "transcriptional modules" are sets of genes that follow similar expression patterns across a large number of samples in multiple studies, identified through co-expression meta-analysis. Each module is associated with a unique identifier that indicates the round of selection and order (e.g., M2.8 designates the eighth module of the second round of selection). Upon extraction each transcriptional module is functionally characterized with the help of a literature profiling algorithm (Chaussabel and Sher, 2002).

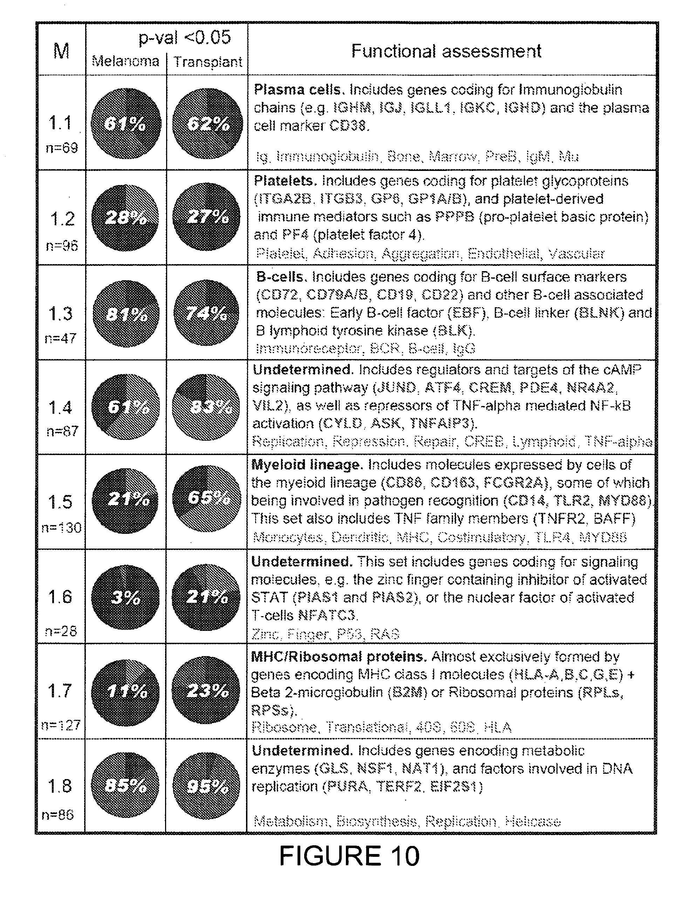

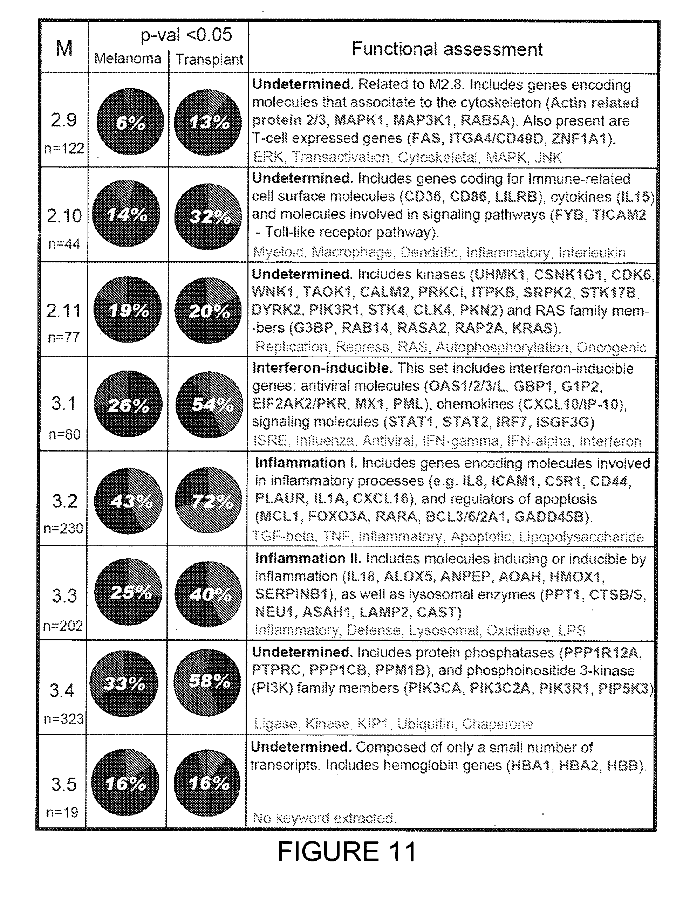

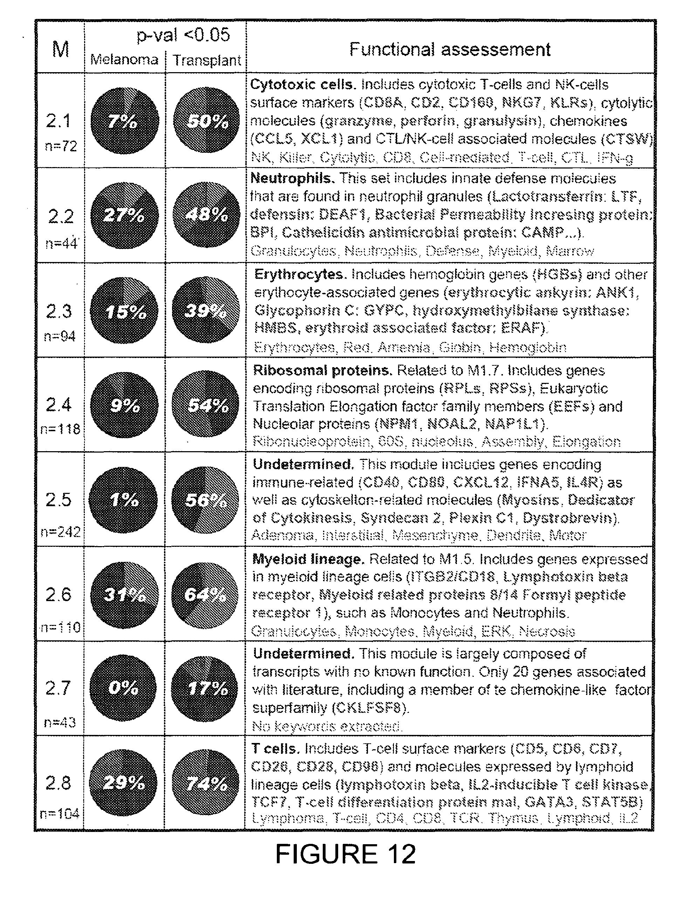

FIGURES 10 to 13 illustrate detailed statistical comparisons between healthy and disease groups of the module-level analysis. For example, twenty-eight sets of coordinately expressed genes, or transcriptional modules, were identified through the analysis of 239 PBMC microarray profiles. For each of these modules changes in expression levels between groups of healthy volunteers and either patients with metastatic melanoma or transplant recipients were tested. A pie chart indicates the proportion of genes that were significantly changed in each module, where red indicates overexpressed genes and blue illustrates underexpressed genes, with a p<0.05 for the Mann-Whitney test. For each module, keywords extracted from the literature are listed in green along with a functional assessment of relationships existing among the genes.

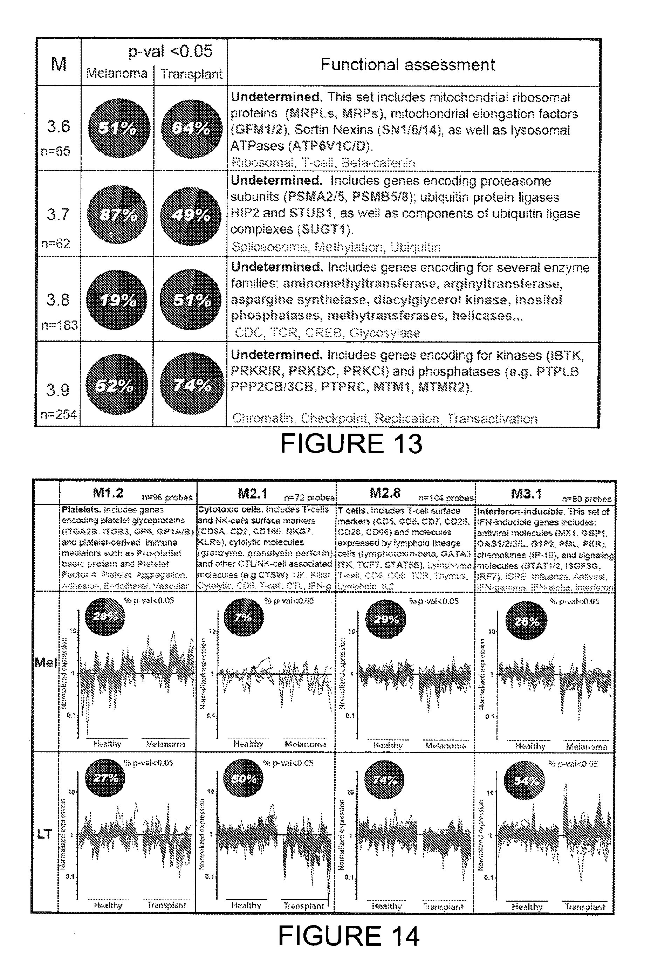

FIGURE 14 is a plot of the changes observed in a few representative modules represented by a transcriptional profile. Each differentially expressed gene is represented by a line that indicates relative levels of expression across healthy volunteer and patient samples. Peaks and dips respectively indicate relatively higher and lower gene expression in a given patient. Genes that were not significantly different are not represented. The levels of expression of genes associated with a platelet signature changed in opposite directions: 28% of the genes forming this signature (M1.2) were overexpressed in patients with melanoma and 27% were underexpressed in transplant recipients. Furthermore, half of the genes belonging to module M2.1 (cytotoxic cell signature) were underexpressed in transplant recipients. This trend was not observed in patients with melanoma (7% overexpressed with p<0.05 where 5% of changes are expected by chance only). Similarly, a massive down-regulation of genes associated to T cells was observed in transplant recipients (74% of genes in M2.8). This finding most likely reflects pharmacological immunosuppression. In patients with melanoma 29% of these T-cell related genes were down-regulated. In addition, 44% of interferon-inducible genes that form module M3.1 were overexpressed in transplant recipients, while 26% were underexpressed in patients with melanoma. Lists of differentially expressed genes in each module are available in Tables 12 and 13.

Patients with metastatic melanoma and transplant recipients display common transcriptional profiles at the modular level. This analysis identified similarities as well as differences in blood leukocyte transcriptional signatures of patients with metastatic melanoma and liver transplant recipients.

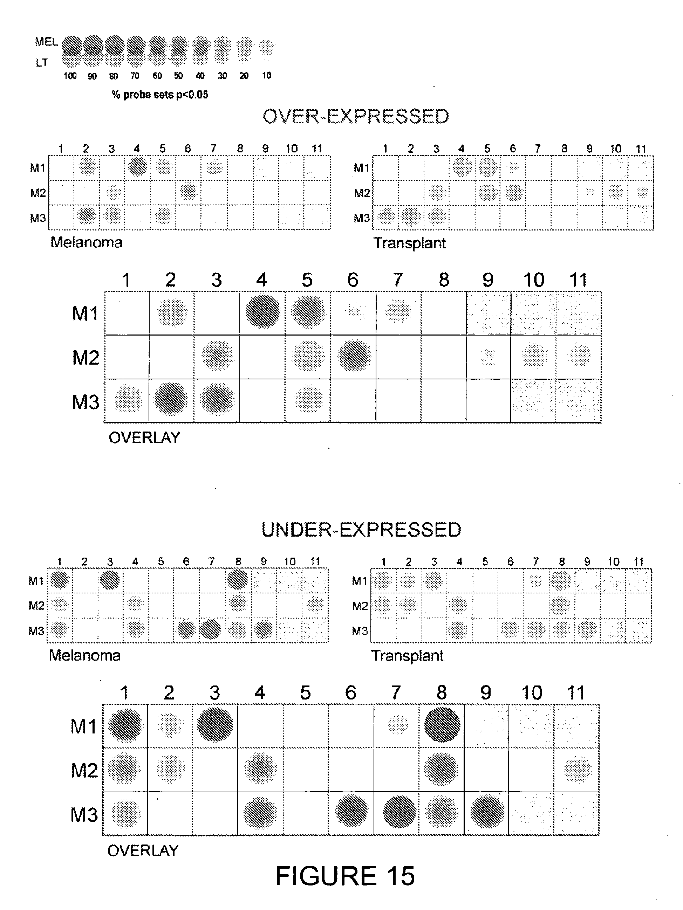

FIGURE 15 is an image of the modular changes observed in both groups of patients vs. their respective healthy control group. The proportion of differentially expressed genes for each module is indicated by a spot of variable intensity. For example, in the overlay a change in transplant is represented by a yellow, while a change in melanoma is indicated by a blue color and a change in both is indicated by a green color.

Proportions of underexpressed and overexpressed transcripts are represented on separate grids. Modules that were common between the two groups of patients include M1.4 (regulator of cAMP and NF-kB signaling pathways), M2.6 (including genes expressed in myeloid lineage cells), M3.2 and M3.3 (both M3.2 and 3.3 include factors involved in inflammation; as seen in FIGURES 10-13).

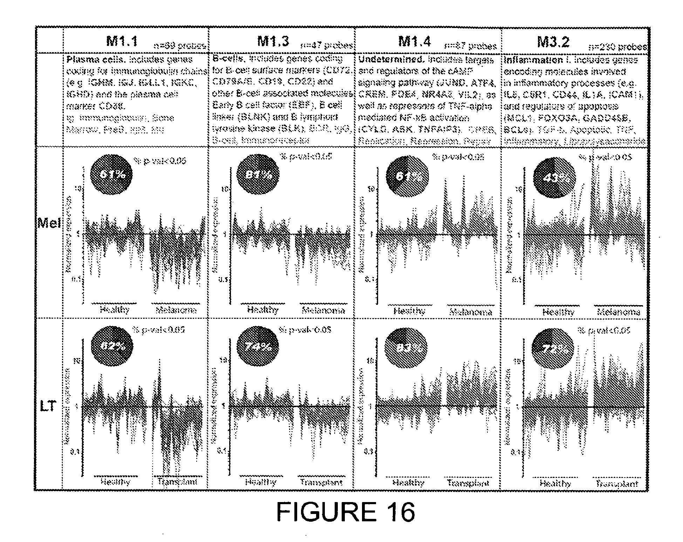

FIGURE 16 is an image illustrating the module-level analysis of the present invention. Common transcriptional signatures in blood from patients with metastatic melanoma and from liver transplant recipients. Expression profiles of genes belonging to blood leukocyte transcriptional modules M1.1, M1.3, M1.4 and M3.2. The total number of probes is indicated for each module (U133A), along with a brief functional interpretation. Keywords extracted by literature profiling are indicated in green. From the total number in each module, the proportion of genes that were significantly changed (Mann-Whitney test, p<0.05) in patients compared to the appropriate healthy control group is indicated in a pie chart with overexpressed genes are expressed in red and underexpressed genes are expressed in blue. Graphs represent transcriptional profiles of the genes that were significantly changed, with each line showing levels of expression on the y-axis of a single transcript across multiple conditions (samples, x-axis).

The association between metastatic melanoma and liver transplant phenotype was strongest for M1.4 and M3.2 as seen in FIGURE 16. Interestingly, a majority of underexpressed modules were common to melanoma and transplant groups, with the most striking similarities in the case of M1.1 (including plasma cell associated genes), M1.3 (including B-cell associated genes) and M1.8 (including genes coding for metabolic enzymes and factors involved in DNA replication).

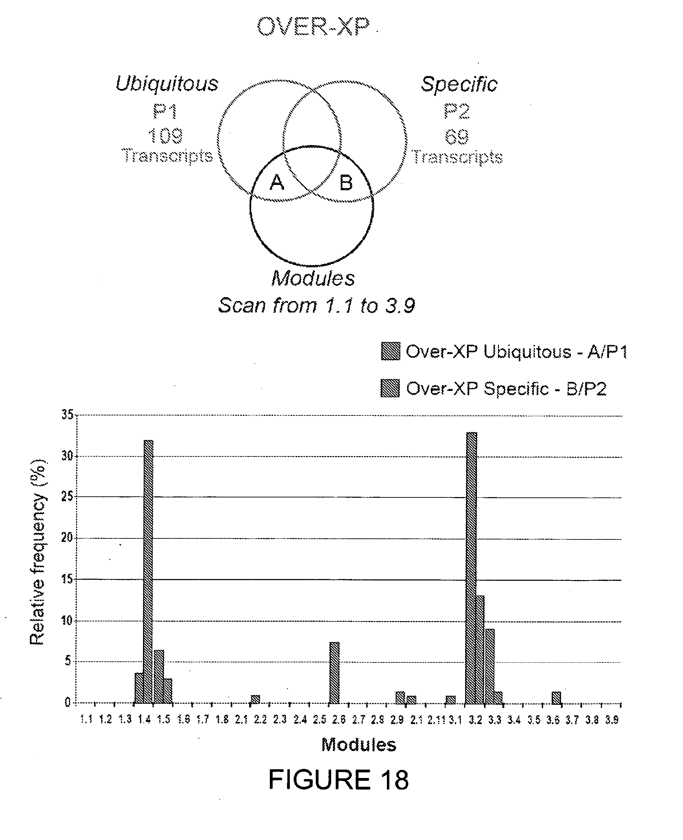

Identification of a common transcriptional signature that is unique to metastatic melanoma and liver transplant patients. The extent of the similarities between patients with metastatic melanoma and liver transplant recipients were specific to these two groups of patients were examined. Statistical group comparison was carried out between patients and healthy controls across all samples (e.g., thirty-eight melanoma, forty-three transplant, thirty-six healthy). Briefly, 323 transcripts were identified that were significantly overexpressed and 918 that were significantly underexpressed in both liver transplant recipients and patients with metastatic melanoma (Mann-Whitney test, p<0.01, filtered >1.25 fold change). Next, group comparisons for these transcripts were carried using samples from patients with Systemic Lupus Erythematosus ("SLE"), acute infections (*S. pneumoniae, S. aureus, E. coli,* and Influenza A) and Graft versus Host Disease ("GVHD") compared to relevant healthy controls. This analysis yielded p-values whose hierarchical clustering identified distinct significance patterns among transcripts common to the melanoma and transplant groups. This analysis identified sets of genes that changed across all diseases as seen in FIGURE 17 with P1 being ubiquitously overexpressed; P3 being ubiquitously underexpressed, while others were associated more specifically with the melanoma and transplant groups as seen in FIGURE 18 with P2 being overexpressed; and P4 being underexpressed. Table 14 illustrates the significance levels across 8 diseases of the genes forming patterns P1, P2, P3 and P4.

FIGURE 17 is an image of the analysis of significance patterns. Genes expressed at higher levels in both stage IV melanoma or liver transplant patients compared to healthy volunteers were selected. P-values were similarly obtained from gene expression profiles generated in other disease models: in PBMCs obtained from patients suffering from systemic lupus erythematosus (SLE), Graft versus Host Disease (GVHD), or acute infections with influenza virus (Influenza A), *Escherichia coli* (*E. coli*), *Streptococcus pneumoniae* (*Strep. Pneumo.*) or *Staphylococcus aureus* (*Staph. aureus*). Each of these cohorts was compared to the appropriate control group of healthy volunteers accrued in the context of these studies. The genes expressed at significantly higher or lower levels in PBMCs obtained from both patients with melanoma and liver transplant recipients (OVER-XP and UNDER-XP, respectively) were ranked by hierarchical clustering of p-values generated for all the conditions listed above. P-values are represented according to a color scale: Green represents low p-value/significant, while white represents high p-value/not significant. Distinct significant patterns are identified, where P1 and P3 are ubiquitous and P2 and P4 are most specific to melanoma and liver transplant groups.

FIGURE 18 is a chart of the modular distribution of ubiquitous and specific gene signatures common to melanoma and transplant groups. Distribution among 28 PBMC transcriptional modules was determined for genes that form ubiquitous (P1) and specific (P2) transcriptional signatures common to the melanoma and transplant groups. Gene lists of each of the modules were compared in turn to the 109 and 69 transcripts that form P1 and P2. For each module, the proportion of genes shared with either P1 or P2 was recorded. These results are represented by a bar graph of FIGURE 18.

Thus, genes forming transcriptional signatures common to the melanoma and transplant groups can be partitioned into distinct sets based on two properties: (1) coordinated expression as seen in the transcriptional modules of FIGURE 13; and (2) change in expression across diseases as seen in the significance patterns of FIGURE 17. The results from these two different mining strategies were recouped by examining the modular distribution of ubiquitous (P1) and specific (P2) PBMC transcriptional signatures. FIGURE 18 clearly shows that the distribution of P1 and P2 across the 28 PBMC transcriptional modules that have been identified to date is not random. Indeed, P1 transcripts are preferentially found among M3.2 (characterized by transcripts related to inflammation), whereas M1.4 transcripts almost exclusively belonged to P2, which includes genes that are more specifically overexpressed in patients with melanoma and liver transplant recipients.

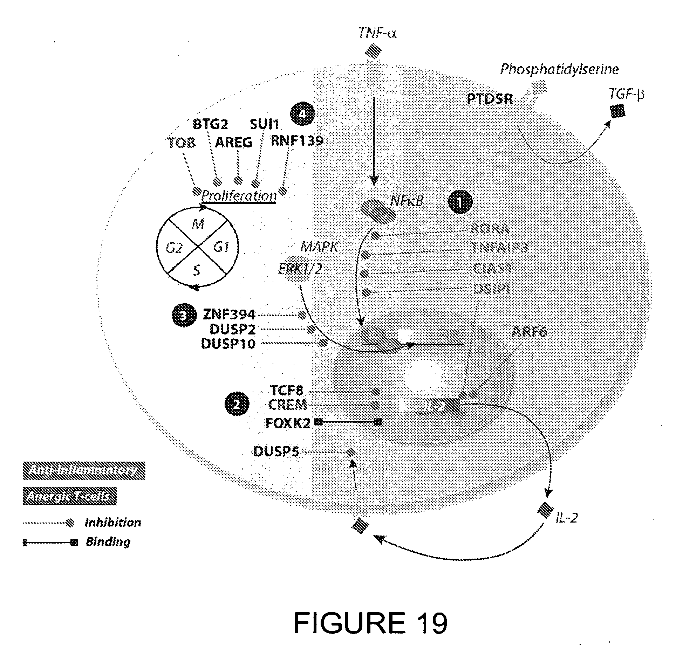

FIGURE 19 is an illustration of the transcriptional signature of immunosuppression. Transcripts overexpressed most specifically in patients with melanoma and transplant recipients (P1) include repressors of immune responses that inhibit: 1) NF-kB translocation; 2) Interleukin 2 production and signaling; 3) MAPK pathways and 4) cell proliferation. Some of these factors are well characterized anti-inflammatory molecules and others are expressed in anergic T-cells.

Molecular signature of immunosuppression. The genes that were most specifically overexpressed in melanoma and transplant groups (P1) were examined. From the 69 probe sets, 55 unique gene identifiers were identified. A query against a literature database indexed by gene, have developed to aid the interpretation of microarray gene expression data, identified 6527 publications associated with 47 genes, 30 of which were associated with more than ten publications. FIGURE 19 illustrates a remarkable functional convergence among the genes forming this signature and includes genes encoding molecules that possess immunoregulatory functions (e.g., anti-proliferative genes: BTG2, TOB1, AREG, SUI1 or RNF139; anti-inflammatory genes: TNFAIP3); inhibitors of transcription: (SON, ZC3HAV1, ZNF394); stress-induced molecules (HERPUD1); while others possess well established immunosuppressive properties. For example, dual specificity phosphatases 2, 5 and 10 (DUSP2, 5, 10) interfere with the MAP kinases ERK1/2, which are known targets of calcineurin inhibitors such as Tacrolimus/FK506. DUSP10 selectively dephosphorylates stress activated kinases(Theodosiou et al., 1999). Interestingly, DUSP5 was found to have a negative feedback role in IL2 signaling in T-cells(Kovanen et al., 2003). CREM, FOXK2 and TCF8 directly bind the IL2 promoter and can contribute to the repression of IL-2 production in T cell anergy(Powell et al., 1999). BHLHB2 (Stra13) negatively regulates lymphocyte development and function in vivo(Seimiya et al., 2004). CIAS1 codes for the protein Cryopyrin, which regulates NF-kappa B activation and production of proinflammatory cytokines. Mutations of this gene have been identified in several inflammatory disorders(Agostini et al., 2004). DSIPI, a leucine zipper protein, is known to mediate the immunosuppressive effects of glucocorticoids and IL10 by interfering with a broad range of signaling pathways (NF-kappa B, NFAT/AP-1, MEK, ERK 1/2), leading to the general inhibition of inflammatory responses in macrophages and down-regulation of the IL2 receptor in T cells. Noatably, the expression of DSIPI in immune cells was found to be augmented after drug treatment (dexamethasone)(D'Adamio et al., 1997) or long term exposure to tumor cells (Burkitt Lymphoma)(Berrebi et al., 2003).

Other immunosuppressive molecules, which did not belong to P1, were also found overexpressed in melanoma and transplant groups. Notably, DDIT4, another dexamethasone-induced gene which was recently found to inhibit mTOR, the mammalian target for rapamycin (Corradetti et al., 2005). Thus, this endogenous factor appears capable of reproducing the action of potent immunosuppressive drugs. HMOX1, a cytoprotective molecule that also demonstrates anti-inflammatory properties. Most recently, HMOX1 expression was found to be induced by FOXP3 and to mediate immunosuppressive effects of CD4+ CD25+ regulatory T cells (Choi et al., 2005). Accordingly, an increase in transcriptional activity of HMOX1 has been correlated with favorable outcomes in experimental transplant models (Soares et al., 1998). Both DDIT4 and HMOX1 genes were also overexpressed in patients with acute *E. coli* or *S*. *aureus* infections. The immunophilin FKBP1A (FKBP12), a member of the FK506-binding protein family, is a key mediator of T-cell immunosuppression by the drugs FK506 (tacrolimus) and rapamycin (Xu et al., 2002). Expression of this gene was elevated in comparison to healthy donors in all patient groups.

Blood is an accessible tissue and lends itself to comparative analyses across multiple diseases. Parmacological and tumor-mediated immunosuppression would produce a common transcriptional signature in blood leukocytes. Metastatic melanoma and transplant recipients disease-associated transcriptional signatures were identified in the blood of patients. These signatures were identified and confirmed through several analytical approaches. Analysis of transcriptional modules identified alterations in blood leukocytes transcriptional components associated to cell types (e.g., Plasma cells, B-cells, T-cells, Cytotoxic cells) and to immune reactions (e.g., Inflammation, Interferon). Furthermore, using both transcriptional modules and gene expression levels similarities between blood transcriptional signatures in patients with metastatic melanoma and liver transplant recipients were identified. However, this common transcriptional signature could not be entirely attributed to immunosuppression. For instance, expression levels of B-cell associated genes (M1.3) were not only decreased in the melanoma and transplant groups, but also in patients with acute influenza infection and systemic lupus erythematosus (SLE) (53% of genes were underexpressed, in comparison to healthy controls; Chaussabel et al.). Conversely, nearly 40% of the genes associated with plasma cells (M1.1) were overexpressed in SLE patients and there was no change in patients with acute influenza infection (7% of the genes were overexpressed at p<0.05), whereas expression levels were significantly decreased in both patients with melanoma and transplant recipients (61 % and 62% of the genes in M1.1, respectively). In order to select the most specific transcripts common between melanoma and transplant signatures a gene-level analysis was carried out across a total of eight groups of patients. This led to the identification of a set of transcripts that was most specifically overexpressed in immunosuppressed patients. The identified set of genes showed marked functional convergence and included genes coding for repressors of Interleukin-2 transcription, inhibitors of NF-kB or MAPK pathways, and anti-proliferative molecules. Interestingly, these signatures are consistent with the mechanism of action of drugs used for pharmacological immunosuppression, which inhibit the activity of calcineurin, a calcium-dependent serine threonine protein phosphatase responsible for the nuclear translocation of NF-AT and NF-kappaB upon T-cell activation. Indicating a functional convergence between immunosuppressive mechanisms operating in patients with advanced melanoma and pharmacologically treated transplant recipients. The fact that the transcripts more specifically induced in immunosuppressed patients include glucocorticoids-inducible genes (e.g., DSIPI, CXCR4, JUN) and hormone nuclear receptors (NR4A2 and RORA)(Winoto and Littman, 2002) suggest a possible role for steroid hormones in tumor-mediated immunosuppression.

Patients with metastatic melanoma display an endogenous transcriptional signature of immunosuppression similar to that induced by pharmacological treatments in patients who underwent liver transplant. The present invention provides a method and apparatus to identify patients at high risk of melanoma progression. In addition the present invention also provides a method and apparatus for monitoring indicators of immunosuppression could help adjusting the dosage of immunosuppressive drugs and balance risks of rejection and side effects for liver transplant recipients.

Examples of patient information and processing of blood samples include the following. Blood was obtained after informed consent as approved by the institutional IRB (Liver transplant recipients: 002-1570199-017; patients with melanoma: 000-048, 002-094; 003-187). Blood samples were obtained in acid citrate dextrose yellow-top tubes (BD Vaccutainer) at the Baylor University Medical Center in Dallas, TX. Samples were immediately delivered at room temperature to the Baylor Institute for Immunology Research, Dallas, TX, for processing. Fresh PBMCs isolated via Ficoll gradient were either stored in liquid nitrogen (e.g., viable freezing) or immediately lysed in RLT buffer, containing β-mercaptoethanol (Qiagen, Valencia, CA). Total RNA was extracted from cells previously frozen in liquid nitrogen ("frozen") or from cells that were lysed immediately after isolation ("fresh"), using the RNEASY^{®} Mini Kit according to the manufacturer's recommended protocol (Qiagen, Valencia, CA). This parameter was taken into account in the experimental design taking into account the age, gender and sample processing method used for the identification (training) and validation (testing) of expression signatures associated with metastatic melanoma and liver transplant recipients undergoing immunosuppressive drug therapy.

Microarray assays. Total RNA was isolated using the RNEASY^{®} kit (Qiagen, Valencia, CA) according to the manufacturer's instructions and the RNA integrity was assessed using an Agilent 2100 Bioanalyzer (Agilent, Palo Alto, CA). Although, the skilled artisan will recognize that other methods of isolation may be used. From 2-5 micrograms of total RNA, double-stranded cDNA containing the T7-dT (24) promoter sequence (Operon Biotechnologies, Huntsville, AL) was generated. This cDNA was then used as a template for in vitro transcription single round amplification with biotin labels (Enzo BioArray HighYield RNA Transcript Labeling Kit from Affymetrix Inc, Santa Clara, CA). Biotinylated cRNA targets were purified using the Sample Cleanup Module and subsequently hybridized to human U133A GeneChips (Affymetrix Inc, Santa Clara, CA) according to the manufacturer's standard protocols. Affymetrix U133A GeneChips that contain 22,283 probe sets, represented by ten to twenty unique probe pairs (perfect match and its corresponding mismatch), which allow detection of 14,500 different genes and expressed sequence tags (ESTs). Arrays were scanned using a laser confocal scanner (Agilent). The samples were processed by the same team, at the same core facility, and were randomized between each array run. Raw data are deposited with GEO (www.ncbi.nltn.nih.gov/geo/).

Data analysis. For each Affymetrix U133A GENE CHIP^{®} raw intensity data were normalized to the mean intensity of all measurements on that array and scaled to a target intensity value of 500 (TGT) in Affymetrix Microarray Suite 5.0. With the aid of GeneSpring software, version 7.2, the measurement for each gene per patient sample array was divided by the median of that gene's measurement from the cohort of healthy volunteers. A filter was applied based on Affymetrix flag calls: probe sets were selected if "Present" in at least 75% of samples in either group (healthy controls or patients). This step insured a more reliable intensity measurement of the genes used in downstream analyses. Class comparison was performed using a non-parametric ranking statistical analysis test (Mann-Whitney) applied to the selected set of genes. In the vertical direction, hierarchical clusters of genes were generated using the Pearson correlation around zero, Genespring's standard correlation measure. Normalized gene expression data were examined with a nonparametric univariate analysis (Fisher's exact test) to identify genes potentially discriminating two different groups. A supervised learning algorithm, the K-Nearest Neighbors Method, was applied that assigned a sample to pre-defined classes in three steps: 1) identification of genes (observations) that have strong correlations to classes to be distinguished; 2) confirmation that identified genes distinguish pre-defined classes; and 3) validation with "unknown samples".

Identification of transcriptional modules. A total of 239 blood leukocyte gene expression profiles were generated using Affymetrix U133A&B GENECHIPS (>44K probe sets). Transcriptional data were obtained for 8 groups including Systemic Juvenile Idiopathic Arthritis, SLE, liver transplant recipients, melanoma patients, and patients with acute infections: *Escherichia coli, Staphylococcus aureus* and Influenza A. For each group, transcripts that were present in at least 50% of all conditions were segregated into 30 clusters (k-means clustering: clusters C1 through C30). The cluster assignment for each gene was recorded in a table and distribution patterns were compared among all the genes. Modules were selected using an iterative process, starting with the largest set of genes that belonged to the same cluster in all study groups (i.e. genes that were found in the same cluster in 8 of the 8 groups). The selection was then expanded from this core reference pattern to include genes with 7/8, 6/8 and 5/8 matches. The resulting set of genes formed a transcriptional module and was withdrawn from the selection pool. The process was then repeated starting with the second largest group of genes, progressively reducing the level of stringency. This analysis led to the identification of 4742 transcripts that were distributed among 28 modules. Each module is attributed a unique identifier indicating the round and order of selection (e.g., M3.1 was the first module identified in the third round of selection).

Analysis of significance patterns. Gene expression data were generated for PBMCs obtained from patients and healthy volunteers using Affymetrix HG-U133A GENECHIPS. P values were obtained for six reference datasets by comparing groups of patients to their respective healthy control groups (Mann-Whitney rank test). The groups were composed of patients with: 1) Systemic Lupus Erythematosus (SLE, 16 samples), 2) Influenza A (16 samples), 3) *Escherichia coli* (16 samples), 4) *Staphylococcus aureus* (16 samples), and 5) *Streptococcus pneumoniae* (14 samples); and 7) bone marrow transplant recipients undergoing graft versus host disease (GVHD, 12 samples). Control groups were also formed taking into account age, sex and project (10 samples in each group). Genes significantly changed (p<0.01) in the "study group" (Melanoma and Transplant) were divided in two sets: overexpressed versus control and underexpressed versus control. P-values of the genes forming the overexpressed set were obtained for the "reference groups" (SLE, GVHD and infections with influenza virus, *E. coli, S. aureus, S. pneumoniae*). P-value data were processed with a gene expression analysis software program, GeneSpring, Version 7.2 (Agilent), which was used to perform hierarchical clustering and group genes based on significance patterns.

Example 2. Determination and Analysis of Patterns of Significance are used to identify ubiquitous and disease-specific gene expression signatures in patient peripheral blood leukocytes.

The use of gene expression microarrays in patient-based research creates new prospects for the discovery of diagnostic biomarkers and the identification of genes or pathways linked to pathogenesis. Gene expression signatures were generated from peripheral blood mononuclear cells isolated from over one hundred patients with conditions presenting a strong immunological component (patient with autoimmune, graft versus host and infectious diseases, as well as immunosuppressed transplant recipients). This dataset permitted the opportunity to carry out comparative analyses and define disease signatures in a broader context. It was found that nearly 20% of overlap between lists of genes significantly changed versus healthy controls in patients with Systemic Lupus Erythematosus (SLE) and acute influenza infection. Transcriptional changes of 22,283 probe sets were evaluated through statistical group comparison performed systematically for 7 diseases versus their respective healthy control groups. Patterns of significance were generated by hierarchical clustering of p-values. This "Patterns of Significance" approach led to the identification of a SLE-specific "diagnostic signature", formed by genes that did not change compared to healthy in the other 6 diseases. Conversely, "sentinel signatures" were characterized that were common to all 7 diseases. These findings allow for the use of blood leukocyte expression signatures for diagnostic and early disease detection.

Briefly, blood is a reservoir and migration compartment for immune cells exposed to infectious agents, allergens, tumors, transplants or autoimmune reactions. Leukocytes isolated from the peripheral blood of patients constitute an accessible source of clinically-relevant information and a comprehensive molecular phenotype of these cells can be obtained by microarray analysis. Gene expression microarrays have been extensively used in cancer research, and proof of principle studies analyzing Peripheral Blood Mononuclear Cell (PBMC) samples isolated from patients with Systemic Lupus Erythematosus (SLE) lead to a better understanding of mechanisms of disease onset and responses to treatment. Two main applications have been found for gene expression microarrays in the context of patient-based research: (1) the discovery of biomarkers and establishment of diagnosis / prognosis signatures (e.g. prediction of survival of breast cancer patients) (2) the identification of genes/pathways involved in pathogenesis, leading for instance to the discovery of the role of interleukin-1 in the pathogenesis of systemic onset juvenile idiopathic arthritis. However, the analysis of microarray data still constitutes a considerable challenge. The ability to simultaneously acquire data for tens of thousands of features in a single test is one of the most appealing characteristic of microarrays, but it can also be a major shortcoming7. This 'curse of dimensionality' is compounded by the fact that the numbers of samples analyzed is usually small. The imbalance between the numbers of genes and conditions analyzed considerably weakens data interpretation capabilities. A microarray gene expression database was created that constitutes samples obtained from patients with diseases that possess a strong immune component. The meta-analysis strategy of the present invention allows for the identification of ubiquitous as well as disease-specific signatures.

Processing of Blood Samples. Blood samples were collected by venipuncture and immediately delivered at room temperature to the Baylor Institute for Immunology Research, Dallas, TX, for processing. Peripheral blood mononuclear cells (PBMCs) from 3-4 ml of blood were isolated via Ficoll gradient and immediately lysed in RLT reagent (Qiagen, Valencia, CA) with beta-mercaptoethanol (BME) and stored at -80°C prior to the RNA extraction step.

Microarray analysis. Total RNA was isolated using the RNeasy kit (Qiagen, Valencia, CA) according to the manufacturer's instructions and RNA integrity was assessed by using an Agilent 2100 Bioanalyzer (Agilent, Palo Alto, CA). Target labeling was performed according to the manufacturer's standard protocol (Affymetrix Inc, Santa Clara, CA). Biotinylated cRNA targets were purified and subsequently hybridized to Affymetrix HG-U133A GeneChips (22,283 probe sets). Arrays were scanned using an Affymetrix confocal laser scanner. Microarray Suite, Version 5.0 (MAS 5.0; Affymetrix) software was used to assess fluorescent hybridization signals, to normalize signals, and to evaluate signal detection calls. Normalization of signal values per chip was achieved using the MAS 5.0 global method of scaling to the target intensity value of 500 per GeneChip. A gene expression analysis software program, GeneSpring, Version 7.1 (Agilent), was used to perform statistical analysis, hierarchical clustering and classification of samples.

Development and Analysis of Patterns of Significance. Gene expression data were generated for PBMCs obtained from patients and healthy volunteers using Affymetrix HG-U133A GeneChips that were run on the same Affymetrix system, using standard operating procedures. P values were obtained by comparing 7 groups of patients to their respective healthy control groups (Mann-Whitney rank test). The groups were composed of pediatric patients with: 1) Systemic Lupus Erythomatosus (SLE, 16 samples), 2) Influenza A (16 samples), 3) *Staphylococcus aureus* (16 samples), 4) *Escherichia coli* (16 samples) and 5) *Streptococcus pneumoniae* (14 samples); as well as adult transplant recipients: 6) Liver transplant patients that have accepted the graft under immunosuppressive therapy (16 samples) and 7) bone marrow transplant recipients undergoing graft versus host disease (GVHD, 12 samples). Control groups were also formed taking into account age, sex and project (10 samples in each group). Genes significantly changed (p<0.01) in the "study group" (Influenza A and/or SLE) were divided in two sets: over-expressed versus control and under-expressed versus control. P-values of the genes forming the over-expressed set were obtained for the "reference groups" (infections with *E .coli, S. aureus, S. pneumoniae,* Liver transplant recipients and graft versus host disease). P-values of the reference groups were set to 1 when genes were under-expressed. The same procedure was used in the set of genes under-expressed in study group, only this time P-values of the reference group were set to 1 when genes were over-expressed. P-value data were processed with a gene expression analysis software program, GeneSpring, Version 7.1 (Agilent), that was used to perform hierarchical clustering and group genes based on significance patterns.

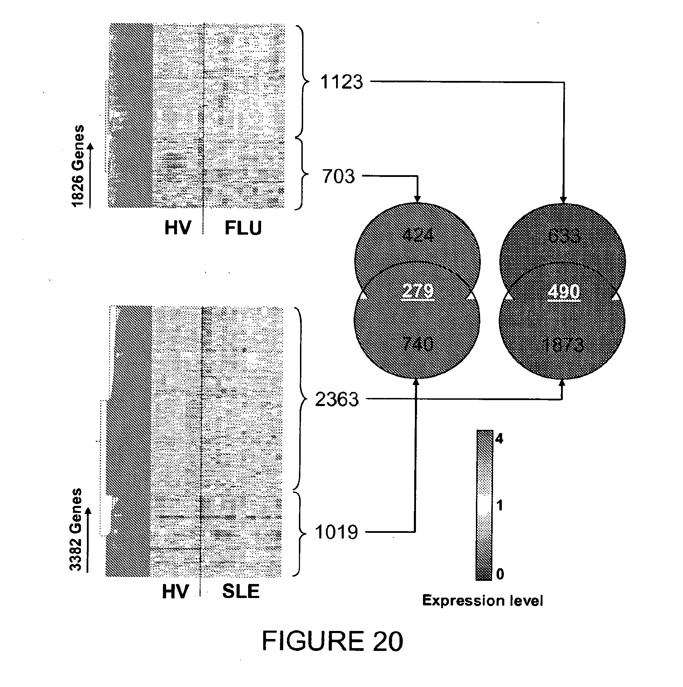

Identification of blood leukocytes transcriptional signatures associated with acute Influenza A infection and SLE. Microarray gene expression data obtained from pediatric patients with either SLE or acute influenza A infections were used to identify transcriptional signatures characteristic of these two diseases (Figure 20). Statistical comparison of a similar number of patients (18 samples) to their respective control group (10 samples) identified: (1) 1826 differentially expressed genes that formed the Influenza signature (of those 703 were over-expressed (red) relative to controls and 1123 were under-expressed (blue), see Figure (20A); 2) 3382 differentially expressed genes formed the SLE signature (of those 1019 were over-expressed relative to controls and 2363 were under-expressed, see Figure 20B).

Figure 20 shows a statistical group comparison between patients and their respective controls. Figure 20A. Microarray expression obtained for PBMC isolated from 16 children with acute Influenza A infection (FLU) and 10 healthy volunteers (HV) were compared (Mann-Whitney rank test, p<0.01). Out of 1826 differentially expressed genes, 703 were over-expressed and 1123 under-expressed in patients. Figure 20B. An equivalent number of children with Systemic Lupus Erythomatosus (SLE) were compared to their respective set of 10 healthy volunteers (HV) (Mann-Whitney rank test, p<0.01). Out of 3382 differentially expressed genes, 1019 were over-expressed and 2363 under-expressed in patients. Figure 20C. Comparison of over-expressed and under-expressed gene lists obtained for SLE and FLU samples relative to their respective control groups (healthy volunteers).

Transformed expression levels are indicated by color scale, with red representing relatively high expression and blue indicating relatively low expression compared to the median expression for each gene across all donors.

Analysis of significance patterns. Next, the specificity of these signatures for each disease was determined. A substantial overlap was found between the sets of genes that were differentially expressed in FLU and SLE (Figure 20C), with 279 over-expressed and 490 under-expressed genes common to both diseases (19% and 16% of similarities, respectively). This observation was used to determine whether a specific disease signature could be obtained in the context of a broader set of diseases.

In order to address this question the analysis was extended to PBMC transcriptional datasets obtained for patients with acute infections caused by bacteria *(E. coli, S. aureus and S. pneumoniae)* as well as transplant recipients (liver recipients who have accepted the allograft under pharmacological immunosuppressive therapy and bone marrow recipients with graft versus host disease). Patterns of significance were analyzed for genes that were specifically over-expressed in SLE compared to Influenza (Figure 1, 740 genes). This approach allowed the visualization of the significance of changes in levels of gene expression for each disease compared to its respective control group (age and sex matched healthy volunteers). Genes were arranged according to significance patterns by hierarchical clustering.

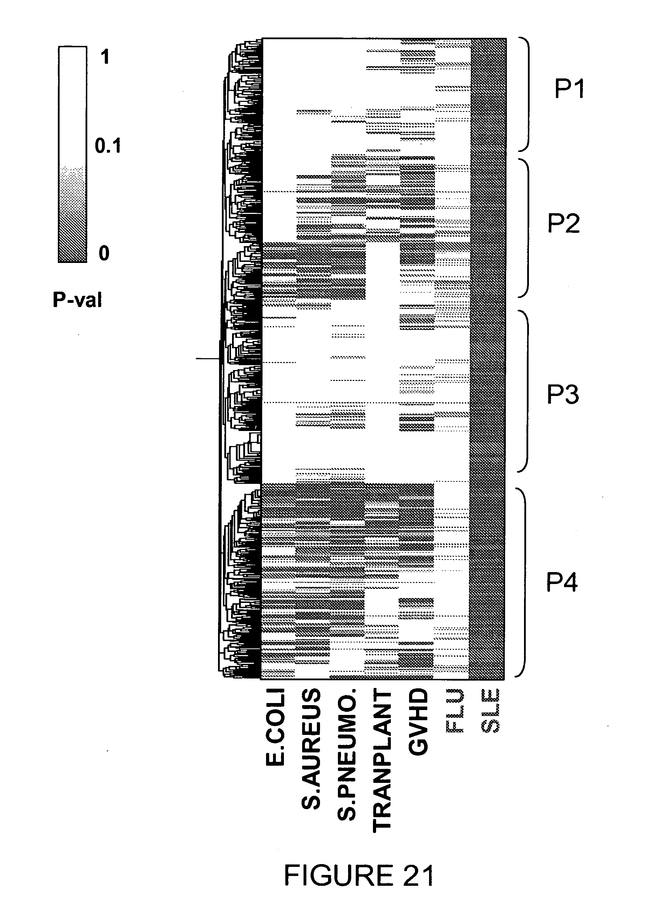

Of the 4 patterns identified, 2 were found to be largely specific to SLE (Figure 21: P1 - 98 genes, and P3 - 193 genes). In conclusion, the method was used to identify sets of genes, particularly among P3, that displayed a high degree of specificity for SLE when compared to 6 other diseases.

Figure 21 is an analysis of patterns of significance for genes over-expressed in SLE patients but not in patients with acute Influenza A infection. The genes used for this analysis were significantly over-expressed in patients with SLE compared to their respective control group (Mann-Whitney P<0.05) and not in patients with acute influenza A infection were selected for this analysis (740 genes). P values were obtained for five additional groups of patients: *E. coli, S. aureus, S. pneumoniae,* Liver transplant recipients and patients with graft vs host disease. The values were imported into a microarray data analysis software package (see methods for details). Four patterns were identified: SLE-1 to 4. Significance levels are indicated by color scale, with darker green representing lower P-values and white indicating a P-value of 1.

Identification of a common disease signature. An important proportion of genes from Figure 21 were induced ubiquitously (P2 - 222 genes and P4 - 225 genes). This finding suggests that these different diseases may share common transcriptional components in the blood constituting a "sickness" signature. In order to investigate this possibility sets of genes were analyzed that were shared between Influenza and SLE signatures (Figure 20C: 279 genes over-expressed, and 490 under-expressed).

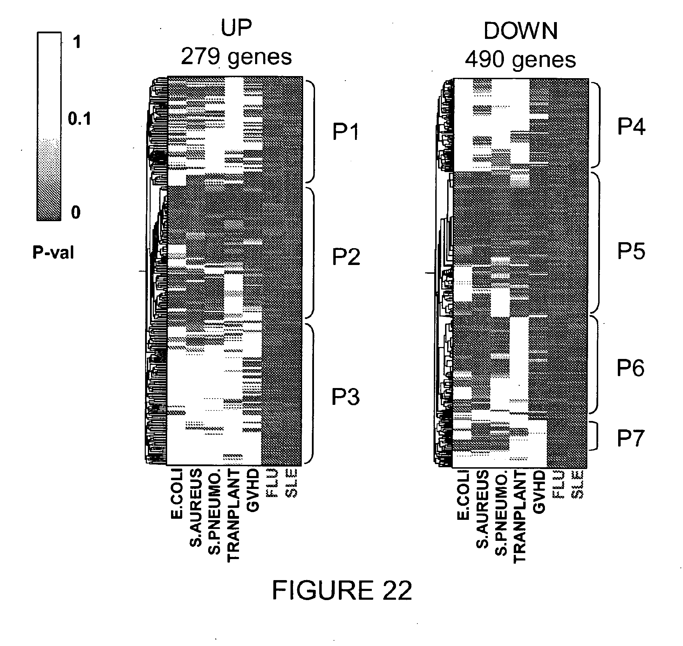

Figure 22 shows Patterns of Significance for genes common to Influenza A and SLE. Genes overexpressed (left panel, OVER) and underexpressed (right panel, UNDER) in both patients with Influenza A (FLU) and SLE were examined in the context of other diseases: acute infections with *E. coli, S. aureus, S. pneumoniae,* liver transplant recipients (transplant) and bone marrow recipients with graft versus host disease (GVHD). Significance levels are indicated by color scale, with dark green representing lower P-values and white indicating a P-value of 1.

Patterns of significance were generated for these genes across all 7 diseases as described above. Three subsets were identified among the genes that were over-expressed in patients with Influenza A infection and SLE: one changing in most diseases, another presenting significant differences in all diseases, while the third was more specific to Influenza and SLE (Figure 22A, respectively P1, P2 and P3). Equivalent patterns can be found upon analysis of a set of under-expressed genes common to Influenza and SLE (Figure 22B, P4-7). Interestingly, the group of patient with significance patterns that were the most similar to Influenza and SLE had Graft Versus Host Disease. The parallelism was particularly striking for the set of under-expressed genes (Figure 22B).

Functional analysis of significance patterns. Finally, functional annotations associated to the patterns identified on Figure 22 were extracted. Genes associated with "defense response" were preferentially found in two patterns (P2-3 on Figures 3 & 4; Fisher's test for over-representation of this functional category: p<0.0005). These genes were expressed at higher levels compared to healthy. The list includes Defensin alpha 3, Azurocidin 1, Stabilin 1 (P2); the tumor necrosis factor family member TRAIL, and Galectin 3 binding protein (P3). Conversely under-expressed genes belonging to patterns P4-6 were preferentially associated with "structural constituent of ribosome" (Fisher's test for over-representation of this functional category in P4-6: p<0.0001). These genes include multiple ribosomal protein family members (e.g. RPS10, RPL37, and RPL13). Genes belonging to the set of over-expressed genes the most specific to Influenza and SLE (P3) were preferentially associated to "interferon response" (p<0.0001, e.g. myxovirus resistance 1, interferon alpha-inducible protein 16, double stranded RNA inducible protein kinase), while genes in P1 were uniquely associated to "heavy metal binding" (p<0.0001, reflecting an overabundance of members of the metallothionein family).

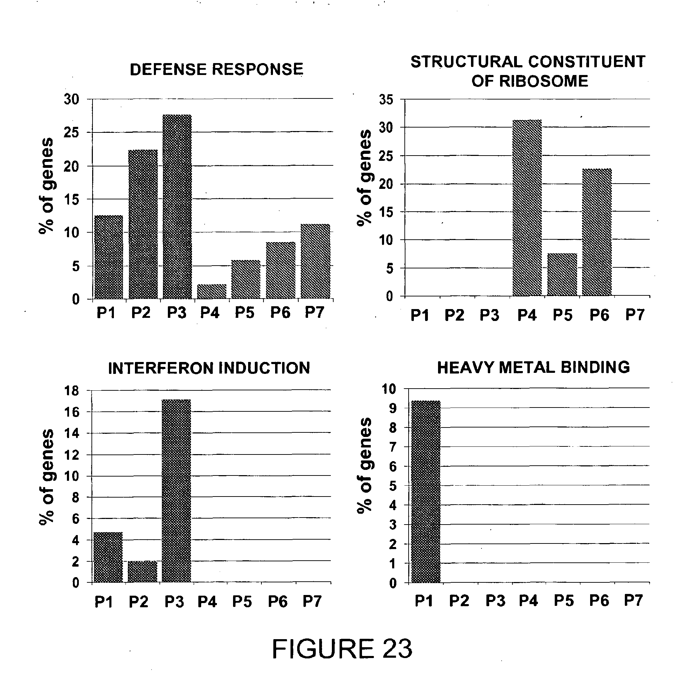

Figure 23 is a functional analysis of genes shared by patients with Influenza infection and Lupus grouped according to significance patterns. Sets of genes forming the different patterns indicated on Figure 22 (P1-7) were subjected to functional analyses. The histograms indicate the percentage of genes associated to a given annotation for each of the sets. Over-expressed genes = red, under-expressed = blue. P1 n= 71 genes; P2 n=118; P3 n=85; P4 n=117; P5 n=184; P6 n=120; P7 n=46.

The comparative analysis of PBMC transcriptional patterns identified disease-specific as well as ubiquitous expression signatures. Different degrees of disease specificity were observed among the genes found to be common between the transcriptional profiles of PBMCs obtained from patients with Influenza infection and SLE. Differences in significance patterns were translated into distinct functional associations. Indeed, the genes that were most specific to Influenza and SLE relative to 5 other diseases were the most strongly associated to biological themes such as: "Interferon induction" (over-expressed genes; Figures 22 and 23: P3) or "structural constituent of ribosome" (under-expressed genes; Figures 22 and 24: P4). These observations permit to validate the relevance of this approach. This analysis facilitates the interpretation of microarray data by placing disease signatures in a much broader context.

In addition to contributing to a better understanding of disease processes the meta-analysis of PBMC transcriptional datasets has important implications for clinical diagnostic with: (1) the identification of discriminatory disease-specific signatures; as the screening of tens of thousands of potential markers will in most cases permit to pinpoint a limited number of transcripts that uniquely characterize a disease; and (2) the identification of a sentinel signature; as sets of genes for which expression changes in a wide range of health disorders could potentially be used in a screening assay for early disease detection.

Patients with metastatic melanoma display an endogenous transcriptional signature of immunosuppression similar to that induced by pharmacological treatments in patients who underwent liver transplant. The present invention provides a method and apparatus to identify patients at high risk of melanoma progression. In addition, the present invention also provides a method and apparatus for monitoring indicators of immunosuppression could help adjusting the dosage of immunosuppressive drugs and balance risks of rejection and side effects for liver transplant recipients.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

### REFERENCES

Agostini, L., Martinon, F., Bums, K., McDermott, M. F., Hawkins, P. N., and Tschopp, J. (2004). NALP3 forms an IL-1beta-processing inflammasome with increased activity in Muckle-Wells autoinflammatory disorder. Immunity 20, 319-325.
Barrett, W. L., First, M. R., Aron, B. S., and Penn, I. (1993). Clinical course of malignancies in renal transplant recipients. Cancer 72, 2186-2189.
Berrebi, D., Bruscoli, S., Cohen, N., Foussat, A., Migliorati, G., Bouchet-Delbos, L., Maillot, M. C., Portier, A., Couderc, J., Galanaud, P., et al. (2003). Synthesis of glucocorticoid-induced leucine zipper (GILZ) by macrophages: an anti-inflammatory and immunosuppressive mechanism shared by glucocorticoids and IL-10. Blood 101, 729-738.
Bordea, C., Wojnarowska, F., Millard, P. R., Doll, H., Welsh, K., and Morris, P. J. (2004). Skin cancers in renal-transplant recipients occur more frequently than previously recognized in a temperate climate. Transplantation 77, 574-579.
Carroll, R. P., Ramsay, H. M., Fryer, A. A., Hawley, C. M., Nicol, D. L., and Harden, P. N. (2003). Incidence and prediction of nonmelanoma skin cancer post-renal transplantation: a prospective study in Queensland, Australia. Am J Kidney Dis 41, 676-683.
Chaussabel, D., and Sher, A. (2002). Mining microarray expression data by literature profiling. Genome Biol 3, RESEARCH0055.
Choi, B. M., Pae, H. O., Jeong, Y. R., Kim, Y. M., and Chung, H. T. (2005). Critical role of heme oxygenase-1 in Foxp3-mediated immune suppression. Biochem Biophys Res Commun 327, 1066-1071.
Corradetti, M. N., Inoki, K., and Guan, K. L. (2005). The stress-inducted proteins RTP801 and RTP801L are negative regulators of the mammalian target of rapamycin pathway. J Biol Chem 280, 9769-9772.
D'Adamio, F., Zollo, O., Moraca, R., Ayroldi, E., Bruscoli, S., Bartoli, A., Cannarile, L., Migliorati, G., and Riccardi, C. (1997). A new dexamethasone-induced gene of the leucine zipper family protects T lymphocytes from TCR/CD3-activated cell death. Immunity 7, 803-812.
Gabrilovich, D. (2004). Mechanisms and functional significance of tumour-induced dendritic-cell defects. Nat Rev Immunol 4, 941-952.
Gerlini, G., Romagnoli, P., and Pimpinelli, N. (2005). Skin cancer and immunosuppression. Crit Rev Oncol Hematol 56, 127-136.
Jachimczak, P., Apfel, R., Bosserhoff, A. K., Fabel, K., Hau, P., Tschertner, I., Wise, P., Schlingensiepen, K. H., Schuler-Thurner, B., and Bogdahn, U. (2005). Inhibition of immunosuppressive effects of melanoma-inhibiting activity (MIA) by antisense techniques. Int J Cancer 113, 88-92.
Kovanen, P. E., Rosenwald, A., Fu, J., Hurt, E. M., Lam, L. T., Giltnane, J. M., Wright, G., Staudt, L. M., and Leonard, W. J. (2003). Analysis of gamma c-family cytokine target genes. Identification of dual-specificity phosphatase 5 (DUSP5) as a regulator of mitogen-activated protein kinase activity in interleukin-2 signaling. J Biol Chem 278, 5205-5213.
Lee, J. H., Torisu-Itakara, H., Cochran, A. J., Kadison, A., Huynh, Y., Morton, D. L., and Essner, R. (2005a). Quantitative analysis of melanoma-induced cytokine-mediated immunosuppression in melanoma sentinel nodes. Clin Cancer Res 11, 107-112.
Lee, Y. R., Yang, I. H., Lee, Y. H., Im, S. A., Song, S., Li, H., Han, K., Kim, K., Eo, S. K., and Lee, C. K. (2005b). Cyclosporin A and tacrolimus, but not rapamycin, inhibit MHC-restricted antigen presentation pathways in dendritic cells. Blood.
Liyanage, U. K., Moore, T. T., Joo, H. G., Tanaka, Y., Herrmann, V., Doherty, G., Drebin, J. A., Strasberg, S. M., Eberlein, T. J., Goedegebuure, P. S., and Linehan, D. C. (2002). Prevalence of regulatory T cells is increased in peripheral blood and tumor microenvironment of patients with pancreas or breast adenocarcinoma. J Immunol 169, 2756-2761.
Monti, P., Leone, B. E., Zerbi, A., Balzano, G., Cainarca, S., Sordi, V., Pontillo, M., Mercalli, A., Di Carlo, V., Allavena, P., and Piemonti, L. (2004). Tumor-derived MUC1 mucins interact with differentiating monocytes and induce IL-10highIL-12low regulatory dendritic cell. J Immunol 172, 7341-7349.
Powell, J. D., Lerner, C. G., Ewoldt, G. R., and Schwartz, R. H. (1999). The -180 site of the IL-2 promoter is the target of CREB/CREM binding in T cell anergy. J Immunol 163, 6631-6639.
Puente Navazo, M. D., Valmori, D., and Ruegg, C. (2001). The alternatively spliced domain TnFnIII A1A2 of the extracellular matrix protein tenascin-C suppresses activation-induced T lymphocyte proliferation and cytokine production. J Immunol 167, 6431-6440.
Seimiya, M., Wada, A., Kawamura, K., Sakamoto, A., Ohkubo, Y., Okada, S., Hatano, M., Tokuhisa, T., Watanabe, T., Saisho, H., et al. (2004). Impaired lymphocyte development and function in ClastS/Stral3/DECl-transgenic mice. Eur J Immunol 34, 1322-1332.
Soares, M. P., Lin, Y., Anrather, J., Csizmadia, E., Takigami, K., Sato, K., Grey, S. T., Colvin, R. B., Choi, A. M., Poss, K. D., and Bach, F. H. (1998). Expression of heme oxygenase-1 can determine cardiac xenograft survival. Nat Med 4, 1073-1077.
Theodosiou, A., Smith, A., Gillieron, C., Arkinstall, S., and Ashworth, A. (1999). MKP5, a new member of the MAP kinase phosphatase family, which selectively dephosphorylates stress-activated kinases. Oncogene 18, 6981-6988.
Viguier, M., Lemaitre, F., Verola, O., Cho, M. S., Gorochov, G., Dubertret, L., Bachelez, H., Kourilsky, P., and Ferradini, L. (2004). Foxp3 expressing CD4+CD25(high) regulatory T cells are overrepresented in human metastatic melanoma lymph nodes and inhibit the function of infiltrating T cells. J Immunol 173, 1444-1453.
Winoto, A., and Littman, D. R. (2002). Nuclear hormone receptors in T lymphocytes. Cell 109 Suppl, S57-66.
Woltman, A. M., van der Kooij, S. W., Coffer, P. J., Offringa, R., Daha, M. R., and van Kooten, C. (2003). Rapamycin specifically interferes with GM-CSF signaling in human dendritic cells, leading to apoptosis via increased p27KIP1 expression. Blood 101, 1439-1445.
Xu, X., Su, B., Barndt, R. J., Chen, H., Xin, H., Yan, G., Chen, L., Cheng, D., Heitman, J., Zhuang, Y., et al. (2002). FKBP12 is the only FK506 binding protein mediating T-cell inhibition by the immunosuppressant FK506. Transplantation 73, 1835-1838.

**Table 12 Module-by-module comparison of genes expression levels in patients with metastatic melanoma vs. healthy volunteers. Patients with melanoma (n=22) vs. Healthy Volunteer (n=23) - training test Mann Whitney U test p-value<0.05, no mtc**

| | | | | | | |
|---|---|---|---|---|---|---|
| **M1.1** | **Total = 69 transcripts** | | **1** | **Overexpressed** | | |
| | | | **41** | **Underexpressed** | | |

| | **Healthy Normalized** | **Raw** | | **Melanoma Normalized** | **Raw** | **p-value** |
|---|---|---|---|---|---|---|
| 221739_at | 0.95782816 | 1236.3346 | 1.1074278 | 1418.6411 | 0.0419 | **Overexpressed** |
| 217148_x_at | 1.0001118 | 1071.8698 | 0.8419552 | 919.35004 | 0.0395 | **Underexpressed** |
| 221004_s_at | 0.9632601 | 358.4826 | 0.6395204 | 275.68634 | 0.0373 | **Underexpressed** |
| 214973_x_at | 0.95571476 | 425.2174 | 0.7533219 | 331.37726 | 0.0275 | **Underexpressed** |
| 217227_x_at | 1.030817 | 252.42172 | 0.7421306 | 194.39093 | 0.0267 | **Underexpressed** |
| 217281_x_at | 0.9731019 | 195.9739 | 0.5853659 | 134.62273 | 0.0228 | **Underexpressed** |
| 221253_s_at | 1.0117456 | 1245.6392 | 0.8528783 | 1057.9501 | 0.0187 | **Underexpressed** |
| 214768_x_at | 0.9577761 | 382.38693 | 0.7833054 | 317.42725 | 0.0187 | **Underexpressed** |
| 214916_x_at | 1.0031506 | 836.6 | 0.7976999 | 648.4591 | 0.0187 | **Underexpressed** |
| 216207_x_at | 0.99609053 | 1815.187 | 0.76317847 | 1397.0773 | 0.0175 | **Underexpressed** |
| 216557_x_at | 0.976244 | 291.37393 | 0.7877697 | 237.53636 | 0.0108 | **Underexpressed** |
| 211635_x_at | 0.95895207 | 312.03043 | 0.65123487 | 220.43636 | 0.00867 | **Underexpressed** |
| 211634_x_at | 0.9670513 | 337.26523 | 0.67734057 | 237.45454 | 0.00641 | **Underexpressed** |
| 211798_x_at | 0.9414217 | 490.5 | 0.6210052 | 330.48642 | 0.00593 | **Underexpressed** |
| 205267_at | 0.9441677 | 1333.8263 | 0.59434533 | 875.75 | 0.00398 | **Underexpressed** |
| 215214_at | 1.0556614 | 210.14351 | 0.6043143 | 141.03635 | 0.00337 | **Underexpressed** |
| 216401_x_at | 0.90682054 | 343.88696 | 0.41231337 | 181.18634 | 0.00261 | **Underexpressed** |
| 214836_x_at | 1.0400113 | 1534.1781 | 0.6920298 | 1034.5046 | 0.00116 | **Underexpressed** |
| 211881_x_at | 0.96697015 | 412.35217 | 0.7305791 | 315.13635 | 0.00116 | **Underexpressed** |
| 211650_x_at | 0.9814827 | 215.1913 | 0.40998137 | 108.97729 | 0.00105 | **Underexpressed** |
| 217022_s_at | 0.83105236 | 5844.505 | 0.32260314 | 3146.8455 | 0.000867 | **Underexpressed** |
| 216853_x_at | 0.9752875 | 187.34348 | 0.56240225 | 122.90455 | 0.000384 | **Underexpressed** |
| 217179_x_at | 1.0001303 | 404.84348 | 0.61423665 | 278.5318 | 0.000384 | **Underexpressed** |
| 215121_x_at | 1.011581 | 7666.9604 | 0.5861822 | 5019.491 | 0.000345 | **Underexpressed** |
| 209138_x_at | 0.99642843 | 7415.57 | 0.58257836 | 4781.964 | 0.000223 | **Underexpressed** |
| 211645_x_at | 1.0724704 | 1021.34796 | 0.66694844 | 655.9682 | 0.000158 | **Underexpressed** |
| 211908_x_at | 1.0104389 | 154.12175 | 0.48859146 | 78.85 | 0.000158 | **Underexpressed** |
| 215176_x_at | 0.9650824 | 1707.6132 | 0.52609867 | 967.5227 | 0.000141 | **Underexpressed** |
| 221651_x_at | 1.0071738 | 13620.529 | 0.63826245 | 9160.868 | 0.000111 | **Underexpressed** |
| 216984_x_at | 0.9702421 | 631.7958 | 0.53891784 | 363.07272 | 0.000111 | **Underexpressed** |
| 217258_x_at | 0.92905986 | 192.33913 | 0.45039603 | 114.42273 | 0.00008 | **Underexpressed** |
| 212592_at | 1.0162625 | 873.5739 | 0.44719663 | 393.49088 | 0.00006 | **Underexpressed** |
| 214677_x_at | 0.95363015 | 8469.707 | 0.52562743 | 5229.05 | 0.00005 | **Underexpressed** |
| 221671_x_at | 1.0028782 | 13795.262 | 0.5931697 | 8619.091 | 0.00003 | **Underexpressed** |
| 214669_x_at | 0.9574678 | 2535.991 | 0.5320335 | 1533.1046 | 0.00002 | **Underexpressed** |
| 211644_x_at | 0.9865403 | 714.4 | 0.59137183 | 491.3318 | 0.00001 | **Underexpressed** |
| 215379_x_at | 0.94754726 | 1798.8262 | 0.60458195 | 1160.209 | 0.00001 | **Underexpressed** |
| 213502_x_at | 1.0198038 | 1779.4697 | 0.647953 | 1129.6864 | 0.00000 | **Underexpressed** |
| 215946_x_at | 1.0226241 | 968.33484 | 0.652376 | 634.5455 | 0.00000 | **Underexpressed** |
| 211430_s_at | 0.9557637 | 3228.7043 | 0.34361708 | 1392.868 | 0.00000 | **Underexpressed** |
| 215118_s_at | 1.0264945 | 666.8652 | 0.45220152 | 296.60913 | 0.00000 | **Underexpressed** |
| 214777_at | 0.9772283 | 331.58698 | 0.45065597 | 171.59091 | 0.00000 | **Underexpressed** |
| **M1.2** | **Total = 96 transcripts** | | | 27 | **Overexpressed** | |
| | | | | 0 | **Underexpressed** | |

| | **Healthy Normalized** | **Raw** | | **Melanoma Normalized** | **Raw** | **p-value** |
|---|---|---|---|---|---|---|
| 215240_at | **0.84488094** | **70.81304** | **1.357003** | **102.99999** | **0.0469** | **Overexpressed** |
| 214525_x_at | 0.9848302 | 322.36084 | 1.3282202 | 442.75455 | 0.0443 | **Overexpressed** |
| 201108_s_tt | 0.9146578 | 356.29565 | 1.3576593 | 612.44104 | 0.0395 | **Overexpressed** |
| 202555_s_at | 0.9885717 | 296.20438 | 1.3414105 | 362.7636 | 0.0373 | **Overexpressed** |
| 208792_s_at | 0.8612959 | 807.19556 | 1.2635909 | 1124.9318 | 0.0373 | **Overexpressed** |
| 34408_at | 0.96183056 | 182.31737 | 1.1373757 | 219.56363 | 0.0373 | **Overexpressed** |
| 217963_s_at | 1.1339012 | 1563.9651 | 1.5669028 | 2227.7456 | 0.0351 | **Overexpressed** |
| 216956_s_at | 0.7829213 | 204.1348 | 1.5463859 | 415.91818 | 0.0331 | **Overexpressed** |
| 201121_s_at | 1.0322056 | 1613.4347 | 1.2225119 | 1934.8318 | 0.0243 | **Overexpressed** |
| 218771_s_at | 0.99680185 | 431.49997 | 1.42558 | 581.89545 | 0.0228 | **Overexpressed** |
| 201120_s_at | 0.94010484 | 340.50867 | 1.42432 | 520.25 | 0.0214 | **Overexpressed** |
| 208546_x_at | 0.8914968 | 153.6913 | 1.3705896 | 228.33636 | 0.0153 | **Overexpressed** |
| 206390_x_at | 0.92774665 | 5643.8003 | 1.3453588 | 8165.2554 | 0.0143 | **Overexpressed** |
| 221211_s_at | 0.91162527 | 728.28687 | 1.5703968 | 1208.6091 | 0.0133 | **Overexpressed** |
| 215492_x_at | 1.0163302 | 440.34348 | 1.2563187 | 549.9545 | 0.0124 | **Overexpressed** |
| 200665_s_at | 0.88955754 | 1178.2607 | 1.3896513 | 1828.4362 | 0.0124 | **Overexpressed** |
| 204081_at | 1.0017431 | 4611.0264 | 1.3459872 | 5716.1274 | 0.01 | **Overexpressed** |
| 209301_at | 0.8715413 | 527.3521 | 1.6178412 | 996.9409 | 0.00867 | **Overexpressed** |
| 209911_x_at | 1.0457131 | 499.37827 | 1.4152079 | 703.6182 | 0.00835 | **Overexpressed** |
| 206110_at | 0.91896015 | 598.5217 | 1.5700213 | 915.52264 | 0.00692 | **Overexpressed** |
| 203585_at | 0.9754219 | 985.7871 | 1.2642365 | 1237.1998 | 0.00431 | **Overexpressed** |
| 204069_at | 0.97689235 | 183.36089 | 1.3654811 | 258.06366 | 0.00239 | **Overexpressed** |
| 203414_at | 0.91576725 | 1835.5477 | 1.4408619 | 2731.3635 | 0.00168 | **Overexpressed** |
| 204838_s_at | 1.0724757 | 356.96957 | 1.802362 | 579.2273 | 0.00168 | **Overexpressed** |
| 208527_x_at | 0.99865276 | 315.16528 | 1.2899728 | 411.1136 | 0.00153 | **Overexpressed** |
| 206655_s_at | 0.95771337 | 710.26086 | 1.7583773 | 1352.2181 | 0.00031 | **Overexpressed** |
| 202708_s_at | 1.020722 | 641.77405 | 1.6268821 | 1041.6364 | 0.00001 | **Overexpressed** |

| **Module-by-module comparison of genes expression levels in patients with metastatic melanoma vs. healthy volunteers. Patients with melanoma (n=22) vs. Healthy Volunteer (n=23) - training test Mann Whitney U test p-value<0.05, no mtc** | | | | | | |
|---|---|---|---|---|---|---|
| M1.1 | Total = 69 transcripts | | 1 | Overexpressed | | |
| | | | 41 | Underexpressed | | |

| Systematic | Common _Affy | Product | | | Genbank | LocusLink |
|---|---|---|---|---|---|---|
| 221739_at | IL27w | | | | AL524093 | 56005 |
| 217148_x_at | IGLV | immunoglobulin lambda variable region | | | AJ249377 | 28831 |
| 221004_s_at | ITM2C; E25; BRI3; E25C; ITM3; BRICD2C | integral membrane protein 3 | | | NM_030926 | 81618 |
| 214973_x_at | IGVH3 | immunoglobulin heavy chain variable region | | | AJ275469 | 3502 |
| 217227_x_at | IGL@ | immunoglobulin lambda light chain VJC region | | | X93006 | 3535 |
| 217281_x_at | IGHV | immunoglobulin heavy chain variable region | | | AJ239383 | 3502 |
| 221253_s_at | TXNDC5; ERP46; UNQ364; EndoPDI; MGC3178 | thioredoxin domain containing 5 isoform 2; thioredoxin domain containing 5 isoform 1 | | | NM_030810 | 81567 |
| 214768_x_at | IGKC | | | | BG540628 | 3514 |
| 214916_x_at | IGHM | | | | BG340548 | 3507 |
| 216207_x_at | IGKV1D-13 | | | | AW408194 | 28902 |
| 216557_x_at | A1VH3 | rearranged immunoglobulin heavy chain | | | U92706 | 3502 |
| 211635_x_at | IGH@ | | | | M24670 | 3507 |
| 211634_x_at | IGH@ | | | | M24669 | 3507 |
| 211798_x_at | IGLJ3 | single-chain antibody | | | AB001733 | 28831 |
| 205267_at | POU2AF1; BOB1; OBF1; OCAB; OBF-1 | POU domain, class 2, associating factor 1 | | | NM_006235 | 5450 |
| 215214_at | IGL@ | | | | H53689 | 3535 |
| 216401_x_at | IGKV | immunoglobulin kappa chain variable region | | | AJ408433 | |
| 214836_x_at | IGKC | | | | BG536224 | 3514 |
| 211881_x_at | IGLJ3 | VEGF single chain antibody | | | AB014341 | 28831 |
| 211650_x_at | IGHM | | | | L34164 | 3507 |
| 217022_a_at | IGHM | immunoglobulin A1-A2 lambda hybrid GAU heavy chain | | | S55735 | 3507 |
| 216853_x_at | IGLJ3 | immunoglobulin light chain variable region | | | AF234255 | 28831 |
| 217179_x_at | IGL@ | immunoglobulin lambda light chain | | | X79782 | 3535 |
| 215121_x_at | IGLJ3 | | | | AA680302 | 28831 |
| 209138_x_at | IGLJ3 | | | | M87790 | 28831 |
| 211645_x_at | IgK | immunoglobulin kappa-chain VK-1 | | | M85256 | 3514 |
| 211908_x_at | IGHM | IgM | | | M87268 | 3507 |
| 215176_x_at | IGKC | | | | AW404894 | 3514 |
| 221651_x_at | IGKC | Unknown (protein for MGC:12418) | | | BC005332 | 3514 |
| 216984_x_at | IGLJ3 | immunoglobulin light chain V-J region | | | D84143 | 28831 |
| 217258_x_at | IGL | immunoglobulin lambda chain | | | AF043583 | |
| 212592_at | IGJ | | | | AV733266 | 3512 |
| 214677_x_at | IGLJ3 | | | | X57812 | 28831 |
| 221671_x_at | IGKC | | | | M63438 | 3514 |
| 214669_x_at | IGKC | | | | BG485135 | 3514 |
| 211644_x_at | IGKC | | | | L14458 | 3514 |
| 215379_x_at | IGLJ3 | | | | AV698647 | 3535 |
| 213502_x_at | IGLL3; 16.1 | lambda L-chain C region | | | X03529 | 91316 |
| 215946_x_at | LOC91316 | | | | AL022324 | 91316 |
| 211430_s_at | IGHG3 | | | | M87789 | 3502 |
| 215118_s_at | | | | | AW519168 | |
| 214777_at | IGKC | | | | BG482805 | 3514 |
| M1.2 Total = 96 transcripts | | | 27 | Overexpressed | | |
| | | | 0 | Underexpressed | | |

| Systematic | Common _Affy | Product | | | Genbank | LocusLink |
|---|---|---|---|---|---|---|
| 215240_at | ITGB3 | | | | AI189839 | 3690 |
| 214525_x_at | MLH3; HNPCC; S240II117 | mutL homolog 3 | | | AB039667 | 27030 |
| 201108_a_at | THBS1 | | | | AI812030 | 7057 |
| 202555_s_at | MYLK; KRP; MLCK; MLCK108; MLCK210; FLJ12216 | myosin light chain kinase isoform 6; myosin light chain kinase isoform 1; myosin light chain kinase isoform 2; myosin light chain kinase isoform 3A; myosin light chain kinase isoform 3B; myosin light chain kinase isoform 4; myosin light chain kinase isofo | | | NM_005965 | 4638 |
| 208792_s_at | CLU; CLI; APOJ; SGP2; SGP-2; SP-40; TRPM2; TRPM-2; MGC24903 | clusterin isoform 1; clusterin isoform 2 | | | M25915 | 1191 |
| 34408_at | RTN2; NSP2; NSPL1 | reticulon 2 isoform A; reticulon 2 isoform B; reticulon 2 isoform C; reticulon 2 isoform D | | | AF004222 | 6253 |
| 217963_s_at | NGFRAP1; Bex; BEX3; NADE; HGR74; DXS6984E | nerve growth factor receptor (TNFRSF16) associated protein 1 isoform b; nerve growth factor receptor (TNFRSF16) associated protein 1 isoform a | | | NM_014380 | 27018 |
| 216956_s_at | ITGA2B; GTA; CD41; GP2B; CD41B; GPIIb | integrin alpha 2b precursor | | | AF098114 | 3674 |
| 201121_s_at | PGRMC1; MPR; HPR6.6 | progesterone receptor membrane component 1 | | | NM_006667 | 10857 |
| 218711_s_at | SDPR; SDR; PS-p68 | serum deprivation response protein | | | NM_004657 | 8436 |
| 201121_s_at | PGRMC1 | | | | AL547946 | 10857 |
| 208546_x_at | HIST1H2BH; H2B/j; H2BFJ | H2B histone family, member J | | | NM_003524 | 8345 |
| 206390_x_at | PF4; CXCL4; SCYB4 | platelet factor 4 (chemokine (C-X-C motif) ligand 4) | | | NM_002619 | 5196 |
| 221211_s_at | C21orf7; TAK1L | chromosome 21 open reading frame 7 | | | NM_020152 | 56911 |
| 215492_x_at | PTCRA | | | | AL035587 | 17155 |
| 200665_s_at | SPARC; ON | secreted protein, acidic, cysteine-rich (osteonectin) | | | NM_003118 | 6678 |
| 204081_at | NRGN; RC3; hng | neurogranin | | | NM_006176 | 4900 |
| 209301_at | CA2; CA-II | carbonic anhydrase II | | | M36532 | 760 |
| 209911_x_at | HIST1H2BD; H2B/b; H2BFB; H2B.1B; HIRIP2; dJ221C16.6 | H2B histone family, member B | | | BC002842 | 3017 |
| 206110_at | HIST1H3H; H3/k; H3FK; H3F1K | H3 histone family, member K | | | NM_003536 | 8357 |
| 203585_at | ZNF185 | zinc finger protein 185 (LIM domain) | | | NM_007150 | 7739 |
| 204069_at | MEIS1 | Meis1 homolog | | | NM_002398 | 4211 |
| 203414_at | MMD; MMA; PAQR11 | monocyte to macrophage differentiation-associated precursor | | | NM_012329 | 23531 |
| 204838_s_at | MLH3; HNPCC; S240II117 | mutL homolog 3 | | | NM_014381 | 27030 |
| 208527_x_at | HIST1H2BE; H2B.h; H2B/h; H2BFH; dJ221C16.8 | H2B histone family, member H | | | NM_003523 | 8344 |
| 206655_s_at | GP1BB; CD42c | glycoprotein Ib beta polypeptide precursor | | | NM_000407 | 2812 |
| 202708_s_at | HIST2H2BE; GL105; H2B.1; H2B/q; H2BFQ | H2B histone family, member Q | | | NM_003528 | 8349 |
| M1.3 | Total = 47 transcripts | | 0 | Overexpressed | | |
| | | | 38 | Underexpressed | | |

| Systematic | Common _Affy | Product | | | Genbank | LocusLink |
|---|---|---|---|---|---|---|
| 202431_s_at | MYC; c-Myc | v-myc myelocytomatosis viral oncogene homolog | | | NM_002467 | 4609 |
| 213674_x at | IGHG3 | | | | AI858004 | 3502 |
| 204208_at | RNGTT; HCE; HCE1; hCAP; CAP1A | RNA guanylyltransferase and 5'-phosphatase | | | NM_003800 | 8732 |
| 202759_s_at | AKAP2 | | | | BE879367 | 11217 |
| 215925_s_at | CD72; LYB2 | CD72 antigen | | | AF283777 | 971 |
| 39318_at | TCL1A | T-cell lymphoma-1 | | | X82240 | 8115 |
| 219471_at | C13orf18; FLJ21562 | chromosome 13 open reading frame 18 | | | NM_025113 | 80183 |
| 212827_at | IGHM; MU | | | | X17115 | 3507 |
| 206896_s_at | GNG7 | guanine nucleotide binding protein (G protein), gamma 7 | | | NM_005145 | 2788 |
| 218781_at | SMC6L1; FLJ22116 | SMC6 protein | | | NM_024624 | 79677 |
| 206398_s_at | CD19; B4; MGC12802 | CD19 antigen | | | NM_001770 | 930 |
| 201689_s_at | TPD52 | | | | BE974098 | 7163 |
| 205297_s_at | CD79B; B29; IGB | CD79B antigen isoform 1 precursor; CD79B antigen isoform 2 precursor | | | NM_000626 | 974 |
| 210889_s_at | FCGR2B; CD32; FCG2; IGFR2 | Fc fragment of IgG, low affinity IIb, receptor for (CD32) isoform 2; Fc fragment of IgG, low affinity IIb, receptor for (CD32) isoform 3; Fc fragment of IgG, low affinity IIb, receptor for (CD32) isoform 4; Fc fragment of IgG, low affinity IIb, receptor f | | | M31933 | 2213 |
| 217979_at | TM4SF13; NET-6; FLJ22934 | tetraspan NET-6 | | | NM_014399 | 27075 |
| 206478_at | KIAA0125 | KIAA0125 gene product | | | NM_014792 | 9834 |
| 204004_at | PAWR | | | | AI336206 | 5074 |
| 220068_at | VPREB3; 8HS20; N27C7-2 | pre-B lymphocyte gene 3 | | | NM_013378 | 29802 |
| 204581_at | CD22; SIGLEC-2 | CD22 antigen | | | NM_001771 | 933 |
| 206759_at | FCER2; CD23; FCE2; CD23A; IGEBF | Fc fragment of IgE, low affinity II, receptor for (CD23A) | | | NM_002002 | 2208 |
| 206255_at | BLK; MGC10442 | B lymphoid tyrosine kinase | | | NM_001715 | 640 |
| 203642_s_at | COBLL1; KIAA0977 | COBL-like 1 | | | NM_014900 | 22837 |
| 213772_s_at | GGA2 | | | | BF196572 | 23062 |
| 209583_s_at | CD200; MRC; MOX1; MOX2; OX-2 | CD200 antigen isoform b; CD200 antigen isoform c; CD200 antigen isoform a precursor | | | AF063591 | 4345 |
| 212386_at | | | | | AK021980 | |
| 219498_s_at | BCL11A; EVI9; CTIP1; BCL11A-L; BCL11A-S; FLJ10173; KIAA1809; BCL11A-XL | B-cell CLL/lymphoma 11A isoform 2; B-cell CLL/lymphoma 11A isoform 1; B-cell CLL/lymphoma 11A isoform 5; B-cell CLL/lymphoma 11A isoform 3 | | | NM_018014 | 53335 |
| 219497_s_at | BCL11A; EVI9; CTIP1; BCL11A-L; BCL11A-S; FLJ10173; KIAA1809; BCL11A-XL | B-cell CLL/lymphoma 11A isoform 2; B-cell CLL/lymphoma 11A isoform 1; B-cell CLL/lymphoma 11A isoform 5; B-cell CLL/lymphoma 11A isoform 3 | | | NM_022893 | 53335 |
| 44790_s_at | C13orf18 | | | | AI129310 | 80183 |
| 205671_s_at | HLA-DOB | major histocompatibility complex, class II, DO beta precursor | | | NM_002120 | 3112 |
| 210356_x_at | MS4A1; B1; S7; Bp35; CD20; MS4A2; LEU-16; MGC3969 | membrane-spanning 4-domains, subfamily A, member 1 | | | BC002807 | 931 |
| 207777_s_at | SP140; LYSP100-A; LYSP100-B | SP140 nuclear body protein isoform 2; SP140 nuclear body protein isoform 1 | | | NM_007237 | 11262 |
| 213891_s_at | | | | | AI927067 | |
| 220059_at | BRDG1; STAP1; STAP-1 | BCR downstream signaling 1 | | | NM_012108 | 26228 |
| 217418_x_at | MS4A1; B1; S7; Bp35; CD20; MS4A2; LEU-16; MGC3969 | membrane-spanning 4-domains, subfamily A, member 1 | | | X12530 | 931 |
| 205861_at | SPIB; SPI-B | Spi-B transcription factor (Spi-1/PU.1 related) | | | NM_003121 | 6689 |
| 207655_s_at | BLNK; Ly57; SLP65; BLNK-s; SLP-65 | B-cell linker | | | NM_013314 | 29760 |
| 219073_s_at | OSBPL10; ORP10; OSBP9; FLJ20363 | oxysterol-binding protein-like protein 10 | | | NM_017784 | 11488 |
| 219667_s_at | BANK1; FLJ20706 | B-cell scaffold protein with ankyrin repeats 1 | | | NM_017935 | 55024 |
| M1.4 | Total = 87 transcripts | | 53 | Overexpressed | | |
| | | | 0 | Underexpressed | | |

| Systematic | Common _Affy | Product | | | Genbank | LocusLink |
|---|---|---|---|---|---|---|
| 221727_at | PC4 | | | | BF209507 | 10923 |
| 218319_at | PELI1 | pellino protein | | | NM_020651 | 57162 |
| 210281_s_at | ZNF198; FIM; MYM; RAMP; SCLL | zinc finger protein 198 | | | AL136621 | 7750 |
| 212434_at | HMGE | | | | AL542571 | 80273 |
| 208881_x_at | IDI1 | isopentenyl-diphosphate delta isomerase | | | BC005247 | 3422 |
| 209967_s_at | CREM; ICER; MGC17881; MGC41893 | cAMP responsive element modulator isoform b; cAMP responsive element modulator isoform a; cAMP responsive element modulator isoform d; cAMP responsive element modulator isoform e; cAMP responsive element modulator isoform f; cAMP responsive element modula | | | D14826 | 1390 |
| 200870_at | STRAP; MAWD; PT-WD; UNRIP | serine/threonine kinase receptor associated protein | | | NM_007178 | 11171 |
| 60084_at | CYLD | | | | AI453099 | 1540 |
| 202644_s_at | TNFAIP3; A20; TNFA1P2 | tumor necrosis factor, alpha-induced protein 3 | | | NM_006290 | 7128 |
| 218399_s_at | CDCA4; HEPP; FLJ20764; | cell division cycle associated 4 | | | NM_017955 | 55038 |
| 204440_at | CD83; BL11; HB15 | CD83 antigen (activated B lymphocytes, immunoglobulin superfamily) | | | NM_004233 | 9308 |
| 203708_at | PDE4B; DPDE4; PDEIVB | phosphodiesterase 4B, cAMP-specific (phosphodiesterase E4 dunce homolog, Drosophila) | | | NM_002600 | 5142 |
| 213134_x_at | BTG3 | | | | AI765445 | 10950 |
| 212665_at | DKFZP434J214 | | | | AL556438 | 25976 |
| 211999_at | H3F3B; H3.3B | H3 histone, family 3B | | | NM_005324 | 3021 |
| 208811_s_at | HSJ2 | DnaJ-like 2 protein | | | AF080569 | 10049 |
| 208931_s_at | ILF3; MMP4; MPP4; NF90; NFAR; TCP80; DRBP76; NFAR-1; MPHOSPH4; NF-AT-90 | interleukin enhancer binding factor 3 isoform b; interleukin enhancer binding factor 3 isoform a: interleukin enhancer binding factor 3 isoform c | | | AF147209 | 3609 |
| 214714_at | ZNF394; FLJ12298 | zinc finger protein 99 | | | AK022360 | 84124 |
| 205548_s_at | BTG3; ANA; TOB5; TOFA; TOB55; MGC8928 | B-cell translocation gene 3 | | | NM_006806 | 10950 |
| 212241_at | GRINL1A | | | | AI632774 | 81488 |
| 216248_s_at | NR4A2; NOT; RNR1; HZF-3; NURR1; TINUR | nuclear receptor subfamily 4, group A, member 2 isoform a; nuclear receptor subfamily 4, group A, member 2 isoform b; nuclear receptor subfamily 4, group A, member 2 isoform c; nuclear receptor subfamily 4, group A, member 2 isoform d | | | S77154 | 4929 |
| 36711_at | MAFF | | | | AL021977 | 23764 |
| 220046_s_at | CCNL1; BM-001; ania-6a | cyclin L1 | | | NM_020307 | 57018 |
| 205281_s_at | PIGA; GPI3; PIG-A | phosphatidylinositol N-acetylglucosaminyltransferase subunit A isoform 1; phosphatidylinositol N-acetylglucosaminyltransferase subunit A isoform 2; phosphatidylinositol N-acetylglucosaminyltransferase subunit A isoform 3 | | | NM_002641 | 5277 |
| 200731_s_at | PTP4A1 | | | | BF576710 | 7803 |
| 203752_s_at | JUND | jun D proto-oncogene | | | NM_005354 | 3727 |
| 220239_at | KLHL7; KLHL6; SBBI26 | SBBI26 protein | | | NM_018846 | 55975 |
| 219228_at | ZNF331; RITA; ZNF361; ZNF463 | zinc finger protein 331 | | | NM_018555 | 55422 |
| 201751_at | KIAA0063 | KIAA0063 gene product | | | NM_014876 | 9929 |
| 209020_at | C20orf111; HSPC207; dJ1183I21.1 | chromosome 20 open reading frame 111 | | | AF217514 | 51526 |
| 202861_at | PER1; hPER; RIGUI | period 1 | | | NM_002616 | 5187 |
| 222044_at | C20orf67 | | | | AI199589 | 63935 |
| 213538_at | SON | | | | AI936458 | 6651 |
| 207332_s_at | TFRC; CD71; TRFR | transferrin receptor | | | NM_003234 | 7037 |
| 210110_x_at | HNRPH3; 2H9 | heterogeneous nuclear ribonucleoprotein H3 isoform a; heterogeneous nuclear ribonucleoprotein H3 isoform b | | | AF132363 | 3189 |
| 204622_x_at | NR4A2; NOT; RNR1; HZF-3; NURR1; TINUR | nuclear receptor subfamily 4, group A, member 2 isoform a; nuclear receptor subfamily 4, group A, member 2 isoform b; nuclear receptor subfamily 4, group A, member 2 isoform c; nuclear receptor subfamily 4, group A, member 2 isoform d | | | NM_006186 | 4929 |
| 212430_at | RNPC1 | | | | AL109955 | 55544 |
| 205214_at | STK17B; DRAK2 | serine/threonine kinase 17b (apoptosis-inducing) | | | NM_004226 | 9262 |
| 202558_s_at | STCH | stress 70 protein chaperone, microsome-associated, 60kDa precursor | | | NM_006948 | 6782 |
| 210837_s_at | PDE4D; DPDE3; STRK1 | cAMP-specific phosphodiesterase 4D | | | AF012074 | 5144 |
| 209510_at | RNF139; RCA1; TRC8; HRCA1; MGC31961 | ring finger protein 139 | | | AF064801 | 11236 |
| 208632_at | RNF10; RIE2; KIAA0262 | ring finger protein 10 | | | AL578551 | 9921 |
| 209457_at | DUSP5; HVH3 | dual specificity phosphatase 5 | | | U16996 | 1847 |
| 208078_s_at | TCF8; BZP; ZEB; ZEB1; AREB6; ZFHEP; NIL-2A; ZFHX1A; NIL-2-A | transcription factor 8 (represses interleukin 2 expression) | | | No_030751 | 6935 |
| 89948_at | C20orf67 | | | | AI743331 | 63935 |
| 202021_x_at | SUI1; A121; ISO1 | putative translation initiation factor | | | AF083441 | 10209 |
| 212130_x_at | SUI1 | | | | AL537707 | 10209 |
| 212227_x_at | SUI1 | | | | AL516854 | 10209 |
| 207630_s_at | CREM; ICER; MGC17881; MGC41893 | cAMP responsive element modulator isoform b; cAMP responsive element modulator isoform a; cAMP responsive element modulator isoform d; cAMP responsive element modulator isoform e; cAMP responsive element modulator isoform f; cAMP responsive element modula | | | NM_001881 | 1390 |
| 222045_s_at | C20orf67 | iduronate-2-sulfatase isoform a precursor; iduronate-2-sulfatase isoform b precursor | | | AI199589 | 63935 |
| 206342_x_at | IDS; MPS2; SIDS | | | | NM_006123 | 3423 |
| 200732_s_at | PTP4A1 | | | | BF576710 | 7803 |
| 200779_at | ATF4; CREB2; TXREB; CREB-2; TAXREB67 | activating transcription factor 4 | | | NM_001675 | 468 |
| M1.5 | Total = 130 transcripts | | 28 | Overexpressed | | |
| | | | 0 | Underexpressed | | |

| Systematic | Common _Affy | Product | | | Genbank | LocusLink |
|---|---|---|---|---|---|---|
| 209263_x_at | TM4SF7; NAG-2; TSPAN-4; | transmembrane 4 superfamily member 7 | | | BC000389 | 7106 |
| 211284_s_at | GRN; PEPI; PCDGF | granulin | | | BC000324 | 2896 |
| 204393_s_at | ACPP; PAP; ACP3; ACP-3 | prostatic acid phosphatase precursor | | | NM_001099 | 55 |
| 221731_x_at | CSPG2; VERSICAN | chondroitin sulfate proteoglycan 2 (versican) | | | J02814 | 1462 |
| 209949_at | NCF2; NOXA2; p67phox; P67-PHOX | neutrophil cytosolic factor 2 | | | BC001606 | 4688 |
| 208702_x_at | APLP2; APPH; APPL2; CDEBP | amyloid beta (A4) precursor-like protein 2 | | | BC000373 | 334 |
| 203508_at | TNFRSF1B; p75; TBPII; TNFBR; TNFR2; CD120b; TNFR80; TNF-R75; p75TNFR; TNF-R-II | tumor necrosis factor receptor 2 precursor | | | NM_001066 | 7133 |
| 222218_s_at | PILRA; FDF03 | paired immunoglobulin-like type 2 receptor alpha isoform 1 precursor; paired immunoglobulin-like type 2 receptor alpha isoform 2 precursor; paired immunoglobulin-like type 2 receptor alpha isoform 3 precursor | | | AJ400843 | 29992 |
| 201743_at | CD14 | CD14 antigen precursor | | | NM_000591 | 929 |
| 201360_at | CST3; AD8 | cystatin C precursor | | | NM_000099 | 1471 |
| 217865_at | RNF130; GP; G1RZFP; GOLIATH | ring finger protein 130 | | | NM_018434 | 55819 |
| 200743_s_at | CLN2; TPP1; LINCL; TPP I; TPP-I | tripeptidyl-peptidase I precursor | | | NM_000391 | 1200 |
| 217897_at | FXYD6 | FXYD domain-containing ion transport regulator 6 | | | NM_022003 | 53826 |
| 202902_s_at | CTSS; MGC3886 | cathepsin S preproprotein | | | NM_004079 | 1520 |
| 218217_at | SCPEP1; RISC; HSCP1 | serine carboxypeptidase 1 precursor protein | | | NM_021626 | 59342 |
| 217764_s_at | RAB31; Rab22B | RAB31, member RAS oncogene family | | | AF183421 | 11031 |
| 208248_x_at | APLP2; APPH; APPL2; CDEBP | amyloid beta (A4) precursor-like protein 2 | | | NM_001642 | 334 |
| 208130_s_at | TBXAS1; TS; TXS; CYP5; THAS; TXAS; CYP5A1 | thromboxane A synthase 1 (platelet, cytochrome P450, family 5, subfamily A) isoform TXS-I; thromboxane A synthase 1 (platelet, cytochrome P450, family 5, subfamily A) isoform TXS-II | | | NM_030984 | 6916 |
| 215049_x_at | CD163; M130; MM130 | CD163 antigen isoform a; CD163 antigen isoform b | | | Z22969 | 9332 |
| 200838_at | CTSB; APPS; CPSB | cathepsin B preproprotein | | | NM_001908 | 1508 |
| 202833_s_at | SERPINA1; A1A; AAT; PI1; A1AT; MGC9222; MGC23330 | serine (or cysteine) proteinase inhibitor, clade A (alpha-1 antiproteinase, antitrypsin), member 1 | | | NM_000295 | 5265 |
| 215051_x_at | AIF1 | | | | BF213829 | 199 |
| 218454_at | FLJ22662 | hypothetical protein FLJ22662 | | | NM_024829 | 79887 |
| 205237_at | FCN1; FCNM | ficolin 1 precursor | | | NM_002003 | 2219 |
| 203773_x_at | BLVRA; BVRA | biliverdin reductase A | | | NM_000712 | 644 |
| 211729_x_at | BLVRA; BVRA | biliverdin reductase A | | | BC005902 | 644 |
| 209901_x_at | AIF1; IBA1; AIF-1; IRT-1 | allograft inflammatory factor 1 isoform 3; allograft inflammatory factor 1 isoform 2; allograft inflammatory factor 1 isoform 1 | | | U19713 | 199 |
| 201995_at | EXT1 | exostosin 1 | | | NM_000127 | 2131 |
| M1.6 | Total = 28 transcripts | | 0 | Overexpressed | | |
| | | | 1 | Underexpressed | | |

| Systematic | Common _Affy | Product | | | Genbank | LocusLink |
|---|---|---|---|---|---|---|
| 215221_at | | | | | AK025064 | |
| M1.7 | Total = 127 transcripts | | 14 | Overexpressed | | |
| | | | 0 | Underexpressed | | |
| Systematic | Common _Affy | Product | | | Genbank | LocusLink |
| 214459_x_at | HLA-C; D6S204; HLA-Cw7; HLA-JY3 | major histocompatibility complex, class I, C precursor | | | M12679 | 51353 |
| 217740_x_at | RPL7A; TRUP; SURF3 | ribosomal protein L7a | | | NM_000972 | 6130 |
| 201665_x_at | RPS17; RPS17L1; RPS17L2 | ribosomal protein S17 | | | NM_001021 | 6218 |
| 200716_x_at | RPL13A | ribosomal protein L13a | | | NM_012423 | 23521 |
| 211942_x_at | | | | | BF979419 | |
| 201254_x_at | RPS6 | ribosomal protein S6 | | | NM_001010 | 6194 |
| 211296_x_at | UBC; HMG20 | ubiquitin C | | | AB009010 | 7316 |
| 212788_x_at | FTL | | | | BG537190 | 2512 |
| 221700_s_at | UBA52; CEP52; RPL40; HUBCEP52 | ubiquitin and ribosomal protein L40 precursor | | | AF348700 | 7311 |
| 208729_x_at | HLA-B | | | | D83043 | 3106 |
| 200905_x_at | HLA-E; HLA-6.2 | major histocompatibility complex, class I, E precursor | | | NM_005516 | 3133 |
| 204102_s_at | EEF2; EEF-2 | eukaryotic translation elongation factor 2 | | | NM_001961 | 1938 |
| 212581_x_at | GAPD | | | | BE561479 | 2597 |
| 211528_x_at | HLA-G2.2 | b2 microglobulin | | | M90685 | 3135 |
| M1.8 | Total = 86 transcripts | | 0 | Overexpressed | | |
| | | | 73 | Underexpressed | | |

| Systematic | Common _Affy | Product | | | Genbank | LocusLink |
|---|---|---|---|---|---|---|
| 201447_at | TIA1 | TIA1 protein isoform 1; TIA1 protein isoform 2 | | | NM_022037 | |
| 221081_s_at | FLJ22457 | hypothetical protein FLJ22457 | | | NM_024901 | 79961 |
| 213405_at | | | | | N95443 | |
| 221865_at | DKFZp547P234 | | | | BF969986 | 20319 |
| 202184_s_at | NUP133; hNUP133; FLJ10814; MGC21133 | nucleoporin 133kDa | | | NM_018230 | 55746 |
| 202453_s_at | GTF2H1; BTF2; TFIIH | general transcription factor IIH, polypeptide 1, 62kDa | | | NM_005316 | 2965 |
| 219243_at | HIMAP4; IAN1; hIAN1; MSTP062; FLJ11110 | immunity associated protein 4 | | | NM_018326 | 55303 |
| 202227_s_at | BRD8; SMAP; p120 | bromodomain containing 8 isoform 1; bromodomain containing 8 isoform 2; bromodomain containing 8 isoform 3 | | | NM_006696 | 10902 |
| 218455_at | NFS1; NIFS; HUSSY-08 | NFS1 nitrogen fixation 1 isoform a precursor; | | | NM_021100 | 9054 |
| | | NFS1 nitrogen fixation 1 isoform b precursor | | | | |
| 208121_a_at | PTPRO; PTPU2; GLEPP1; PTP-U2 | receptor-type protein tyrosine phosphatase O isoform b precursor; receptor-type protein tyrosine phosphatase O isoform a precursor; receptor-type protein tyrosine phosphatase O isoform d precursor; receptor-type protein tyrosine phosphatase O isoform c pr | | | NM_002848 | 5800 |
| 212378_at | GART | | | | BE966876 | 2618 |
| 218303_x_at | LOC51315 | hypothetical protein LOC51315 | | | NM_016618 | 51315 |
| 218614_at | FLJ10652 | hypothetical protein FLJ10652 | | | NM_018169 | 55196 |
| 209175_at | SEC23IP; P125; MSTP053 | Sec23-interacting protein p125 | | | AK001135 | 11196 |
| 213838_at | RANBP9 | | | | AA191426 | 51406 |
| 210053_at | TAF5 | | | | AW138827 | 6877 |
| 219146_at | FLJ22729 | hypothetical protein FLJ22729 | | | NM_024683 | 79736 |
| 212872_s_at | USP49; TRFP; PR00213; | TRF-proximal protein | | | AK023092 | 25862 |
| 201121_s_at | PUM2; PUMH2; PUML2; KIAA0235 | pumilio homolog 2 | | | D87078 | 23369 |
| 218371_s_at | PSPC1; PSP1; FLJ10955 | paraspeckle protein 1 | | | NM_018282 | 55269 |
| 203159_at | GLS; GLS1; KIAA0838 | glutaminase C | | | NM_014905 | 2744 |
| 210541_s_at | RFP; RNF76; TRIM27 | ret finger protein isoform alpha; ret finger protein isoform beta | | | AF230394 | 5987 |
| 212116_at | RFP; RNF76; TRIM27 | ret finger protein isoform alpha; ret finger protein isoform beta | | | NM_006510 | 5987 |
| 214129_at | PDE4DIP | | | | AI821791 | 9659 |
| 218716_x_at | MTO1; CGI-02 | mitochondrial translation optimization 1 homolog; mitochondrial translation optimization 1 homolog isoform IV | | | NM_012123 | 25821 |
| 212405_s_at | CGI-01; KIAA0859 | CGI-01 protein isoform 2; CGI-01 protein isoform 1 | | | AL049669 | 51603 |
| 209828_s_at | IL16; LCF; IL-16; prIL-16; HsT19289 | interleukin 16 isoform 1 precursor; interleukin 16 isoform 2 | | | M90391 | 3603 |
| 218588_s_at | C5orf3; 133K02 | chromosome 5 open reading frame 3 | | | NM_018691 | 10827 |
| 212170_at | RBM12; SWAN; KIAA0765; HRIHFB2091 | RNA binding motif protein 12 | | | AB018308 | 10137 |
| 207338_s_at | ZNF200 | zinc finger protein 200 | | | NM_003454 | 7752 |
| 204676_at | DKFZP564K2062 | DKFZP564K2062 protein | | | NM_015421 | 25880 |
| 219303_at | C13orf7; FLJ13449 | chromosome 13 open reading frame 7 | | | NM_024546 | 79596 |
| 64418_at | | | | | AI472320 | |
| 213626_at | CBR4; FLJ14431 | carbonic reductase 4 | | | AL049442 | 84869 |
| 212632_at | | | | | AF131808 | |
| 206734_at | JRKL; HHMJG | jerky homolog-like | | | NM_003772 | 8690 |
| 208968_s_at | ZFP64; ZNF338 | zinc finger protein 64 isoform a; zinc finger protein 64 isoform b; zinc finger protein 64 isoform c; zinc finger protein 64 isoform d | | | NM_018197 | 55734 |
| 53968 at | KIAA1698 | | | | AI869988 | 80789 |
| 212740_at | PIK3R4 | | | | BF740111 | 30849 |
| 206332_s_at | IFI16; IFNGIP1 | interferon, gamma-inducible protein 16 | | | NM_005531 | 3428 |
| 217907_at | MRPL18; HSPC071; MRP-L18 | mitochondrial ribosomal protein L18 | | | NM_014161 | 29074 |
| 203584_at | KIAA0103 | KIAA0103 | | | NM_014673 | 9694 |
| 203614_at | UTP14C; KIAA0266; 2700066J21Rik | UTP14, U3 small nucleolar ribonucleoprotein, homolog C | | | NM_021645 | 9724 |
| 218534_s_at | VG5Q; FLJ10283; HSU84971; HSU84971 | angiogenic factor VG5Q | | | NM_018046 | 55109 |
| 203143_s_at | KIAA0040 | | | | T79953 | 9674 |
| 205140_at | FPGT; GFPP | fucose-1-phosphate guanyltransferase | | | NM_003838 | 8790 |
| 219130_at | FLJ10287; FLJ11219 | hypothetical protein FLJ10287 | | | NM_019083 | 54482 |
| 219123_at | ZNF232 | zinc finger protein 232 | | | NM_014519 | 7775 |
| 214440_at | NAT1; AAC1 | N-acetyltransferase 1 | | | NM_000662 | 9 |
| 222028_at | ZNF45 | | | | AI967981 | 7596 |
| 212453_at | KIAA1279; DKFZP586B0923 | KIAA1279 | | | AB033105 | 26128 |
| 202060_at | SH2BP1; TSBP; p150TSP; | SH2 domain binding protein 1 | | | NM_014633 | 9646 |
| 218138_at | MKKS; KMS; MKS; BB56; HMCS | McKueick-Kaufman syndrome protein | | | NM_018848 | 8195 |
| 41329_at | FLJ10706 | | | | AI458463 | 57147 |
| 202983_at | SMARCA3 | | | | AI760760 | 6596 |
| 220992_s_at | Clorf25; MGC57134; bG120K12.3 | N2,N2-dimethylguanosine tRNA methyltransferase-like | | | NM_030934 | 81627 |
| 203611_at | TERF2; TRF2; TRBF2 | telomeric repeat binding factor 2 | | | NM_005652 | 7014 |
| 201142_at | EIF2S1 | | | | AA577698 | 1965 |
| 219467_at | FLJ20125 | hypothetical protein FLJ20125 | | | NM_017676 | 54826 |
| 219913_s_at | CRNKL1; HCRN; MSTP021 | crooked neck-like 1 protein | | | NM_016652 | 51340 |
| 202322_s_at | GGPS1; GGPPS; GGPPS1 | geranylgeranyl diphosphate synthase 1 | | | NM_004837 | 9453 |
| 203427_at | ASF1A; CIA; DKFZP547E2110 | ASF1 anti-silencing function 1 homolog A | | | NM_014034 | 25842 |
| 219777_at | hIAN2; FLJ22690 | human immune associated nucleotide 2 | | | NM_024711 | 79765 |
| 201832_s_at | VDP; TAP; P115 | vesicle docking protein p115 | | | NM_003715 | 8615 |
| 219128_at | FLJ20558 | hypothetical protein FLJ20558 | | | NM_017880 | 54980 |
| 200854_at | NCOR1; N-CoR; TRAC1; hN-CoR; KIAA1047; hCIT529I10 | nuclear receptor co-repressor 1 | | | NM_006311 | 9611 |
| 209662_at | CETN3; CEN3; MGC12502 | centrin 3 | | | BC005383 | 1070 |
| 211758_x_at | TXNDC9; APACD | ATP binding protein associated with cell differentiation | | | BC005968 | 10190 |
| 214988_s_at | SON; SON3; BASS1; DBP-5; NREBP; C21orf50; FLJ21099; KIAA1019 | SON DNA-binding protein isoform G; SON DNA-binding protein isoform B; SON DNA-binding protein isoform E; SON DNA-binding protein isoform A; SON DNA-binding protein isoform C; SON DNA-binding protein isoform F | | | X63071 | 6651 |
| 207513_s_at | ZNF189 | zinc finger protein 189 | | | NM_003452 | 7743 |
| 218056_at | BFAR; BAR; RNF47 | apoptosis regulator | | | NM_016561 | 51283 |
| 212402_at | KIAA0853 | | | | BE895685 | 23091 |
| 218242_s_at | SUV420H1; CGI-85; MGC703; MGC21161 | suppressor of variegation 4-20 homolog 1 isoform 2; suppressor of variegation 4-20 homolog 1 isoform 1 | | | NM_017635 | 51111 |
| M2.1 | Total = 72 transcripts | | 1 | Overexpressed | | |
| | | | 4 | Underexpressed | | |

| Systematic | Common _Affy | Product | | | Genbank | LocusLink |
|---|---|---|---|---|---|---|
| 204655_at | CCLS; SISd; SCYAS; RANTES; TCP228; D17S136E; MGC17164 | small inducible cytokine A5 precursor | | | NM_002985 | 6352 |
| 216920_s_at | TCRGC2 | | | | M27331 | 6965 |
| 209813_x_at | TRGV9; V2; TCRGV9 | | | | M16768 | 6965 |
| 215806_x_at | TRGC2; TCRGC2; TRGC2(2X); TRGC@ (3X) | | | | M13231 | 6967 |
| 207723_s_at | KLRC3; NKG2E; NKG2-E | killer cell lectin-like receptor subfamily C, member 3 isoform NKG2-E; killer cell lectin-like receptor subfamily C, member 3 isoform NKG2-H | | | NM_002261 | 3823 |
| M2.2 | Total = 44 transcripts | | 5 | Overexpressed | | |
| | | | 6 | Underexpressed | | |

| Systematic | Common _Affy | Product | | | Genbank | LocusLink |
|---|---|---|---|---|---|---|
| 201161_s_at | CSDA; DBPA; ZONAB | cold shock domain protein A | | | NM_003651 | 8531 |
| 207205_at | CEACAM4; CGM7 | carcinoembryonic antigen-related cell adhesion molecule 4 | | | NM_001817 | 1089 |
| 219281_at | MSRA | methionine sulfoxide reductase A | | | NM_012331 | 4482 |
| 212531_at | LCN2; NGAL | lipocalin 2 (oncogene 24p3) | | | NM_005564 | 3934 |
| 208650_s_at | CD24 | | | | BG327863 | 934 |
| 204881_s_at | UGCG; GCS | ceramide glucosyltransferase | | | NM_003358 | 7357 |
| 208651_x_at | CD24; CD24A | CD24 antigen | | | M58664 | 934 |
| 266_s_at | CD24; CD24A | CD24 antigen | | | L33930 | 934 |
| 204351_at | S100P | S100 calcium binding protein P | | | NM_005980 | 6286 |
| 216379_x_at | CD24 | | | | AK000168 | 934 |
| 209771_x_at | CD24 | | | | AA761181 | 934 |
| M2.3 | Total = 94 transcripts | | 12 | Overexpressed | | |
| | | | 2 | Underexpressed | | |

| Systematic | Common _Affy | Product | | | Genbank | LocusLink |
|---|---|---|---|---|---|---|
| 217748_at | ADIPOR1; PAQR1; ACDCR1; CGI-45 | adiponectin receptor 1 | | | NM_015999 | 51094 |
| 218847_at | IMP-2; IMP2; VICKZ2 | IGF-II mRNA-binding protein 2 | | | NM_006548 | 10644 |
| 202947_s_at | GYPC; GE; GPC | glycophorin C isoform 1; glycophorin C isoform 2 | | | NM_002101 | 2995 |
| 221824_s_at | MGC26766 | | | | AA770170 | 22097 |
| 204848_x_at | HBG1; HBGA; HBGR | A-gamma globin | | | NM_000559 | 3047 |
| 209018_s_at | PINK1 | | | | BF432478 | 65018 |
| 207827_x_at | SNCA; PD1; NACP; PARK1; PARK4 | alpha-synuclein isoform NACP140; alpha-synuclein isoform NACP112 | | | L36675 | 6622 |
| 200075_s_at | GUK1; GMK | guanylate kinase 1 | | | BC006249 | 2987 |
| 220757_s_at | UBXD1; UBXDC2 | UBX domain containing 1 | | | NM_025241 | 80700 |
| 219458_s_at | NSUN3; FLJ22609 | NOL1/NOP2/Sun domain family 3 | | | NM_022072 | 63899 |
| 207667_s_at | MAP2K3; MEK3; MKK3; MAPKK3; PRKMK3 | mitogen-activated protein kinase kinase 3 isoform A; mitogen-activated protein kinase kinase 3 isoform B; mitogen-activated protein kinase kinase 3 isoform C | | | NM_002756 | 5606 |
| 201178_at | FBXO7; FBX7; FBX07 | F-box only protein 7 | | | NM_012179 | 25793 |
| 214273_x_at | CGTHBA | | | | AV704353 | 8131 |
| 211475_s_at | BAG1 | BCL2-associated athanogene isoform 1L | | | AF116273 | 573 |
| M2.4 | Total = 118 transcripts | | 10 | Overexpressed | | |
| | | | 1 | Underexpressed | | |

| Systematic | Common _Affy | Product | | | Genbank | LocusLink |
|---|---|---|---|---|---|---|
| 217906_at | KLHDC2; LCP; HCLP-1 | kelch domain containing 2 | | | NM_014315 | 23588 |
| 201993_x_at | HNRPDL; HNRNP; JKTBP; JKTBP2; laAUF1 | heterogeneous nuclear ribonucleoprotein D-like | | | NM_005463 | 9987 |
| 200631_s_at | SET; 2PP2A; IGAAD; I2PP2A; PHAPII; TAF-IBETA | SET translocation (myeloid leukemia-associated) | | | NM_003011 | 6418 |
| 201258_at | RPS16 | ribosomal protein S16 | | | NM_001020 | 6217 |
| 211666_x_at | RPL3; TARBP-B | ribosomal protein L3 | | | L22453 | 6122 |
| 222099_s_at | DKFZP434D1335 | | | | AW593859 | 26065 |
| 201230_s_at | ARIH2; ARI2; TRIAD1 | ariadne homolog 2 | | | NM_006321 | 10425 |
| 221476_s_at | RPL15; EC45; RPL10; RPLY10; RPYL10 | ribosomal protein L15 | | | AF279903 | 6138 |
| 217747_s_at | RPS9 | ribosomal protein S9 | | | NM_001013 | 6203 |
| 207132_x_at | PFDN5; MM1; MM-1; PFD5; MGC5329 | prefoldin 5 isoform alpha; prefoldin 5 isoform beta; prefoldin 5 isoform gamma | | | NM_002624 | 5204 |
| 200081_s_at | RPS6 | | | | BE741754 | 6194 |
| M2.5 | Total = 242 transcripts | | 3 | Overexpressed | | |
| | | | 1 | Underexpressed | | |

| Systematic | Common _Affy | Product | | | Genbank | LocusLink |
|---|---|---|---|---|---|---|
| 216809_at | CYLC1; CYCL1 | cylicin | | | Z22780 | 1538 |
| 218692_at | FLJ20366 | hypothetical protein FLJ20366 | | | NM_017786 | 55638 |
| 220375_s_at | | | | | NM_024752 | |
| 211572_s_at | SLC23A2; NBTL1; SVCT2; YSPL2; | solute carrier family 23 (nucleobase | | | AF092511 | 9962 |
| | SLC23A1; KIAA0238 | transporters), member 2 | | | | |
| M2.6 Total = 110 transcripts | | | 33 | Overexpressed | | |
| | | | 1 | Underexpressed | | |

| Systematic | Common _Affy | Product | | | Genbank | LocusLink |
|---|---|---|---|---|---|---|
| 213590_at | SLC16A5 | | | | AA705628 | 9121 |
| 201422_at | IFI30; GILT; IP30; IFI-30; MGC32056 | interferon, gamma-inducible protein 30 preproprotein | | | NM_006332 | 10437 |
| 212041_at | ATP6V0D1 | | | | AL566172 | 9114 |
| 202917_s_at | S100A8; P8; MIF; NIF; CAGA; CFAG; CGLA; L1Ag; MRP8; CP-10; MA387; 60B8AG | S100 calcium-binding protein A8 | | | NM_002964 | 6279 |
| 208949_a_at | LGALS3; GAL3; MAC2; CBP35; GALBP; LGALS2 | galectin-3 | | | BC001120 | 3958 |
| 208704_x_at | APLP2; APPH; APPL2; CDEBP | amyloid beta (A4) precursor-like protein 2 | | | BC000373 | 334 |
| 202192_s_at | GAS7 | growth arrest-specific 7 isoform b | | | NM_005890 | 8522 |
| 204232_at | FCER1G | Fc fragment of IgE, high affinity I, receptor for, gamma polypeptide precursor | | | NM_004106 | 2207 |
| 202388_at | RGS2; G0S8 | regulator of G-protein signalling 2, 24kDa | | | NM_002923 | 5997 |
| 201425_at | ALDH2; ALDM; ALDHI; ALDH-E2; MGC1806 | mitochondrial aldehyde dehydrogenase 2 precursor | | | NM_000690 | 217 |
| 205922_at | VNN2; FOAP-4; GPI-80 | vanin 2 isoform 1 precursor; vanin 2 isoform 2 | | | NM_004665 | 8875 |
| 204122_at | TYROBP; DAP12; KARAP; PLOSL | TYRO protein tyrosine kinase binding protein isoform 1 precursor; TYRO protein tyrosine kinase binding protein isoform 2 precursor | | | NM_003332 | 7305 |
| 211474_s_at | SERPINB6 | | | | BC004948 | 5269 |
| 213187_x_at | | | | | BG538564 | |
| 209179_s_at | LENG4; BB1 | leukocyte receptor cluster (LRC) member 4 protein | | | BC003164 | 79143 |
| 202201_at | BLVRB; FLR; BVRB | biliverdin reductase B (flavin reductase (NADPH)) | | | NM_000713 | 645 |
| 211429_s_at | SERPINAL | PR02275 | | | AF119873 | 5265 |
| 203645_s_at | CD163; M130; MM130 | CD163 antigen isoform a; CD163 antigen isoform b | | | NM_004244 | 9332 |
| 200839_s_at | CTSB; APPS; CPSB | cathepsin B preproprotein | | | NM_001908 | 1508 |
| 208703_s_at | APLP2; APPH; APPL2; CDEBP | amyloid beta (A4) precursor-like protein 2 | | | BC000373 | 334 |
| 202426_s_at | RXRA; NR2B1 | retinoid X receptor, alpha | | | NM_002957 | 6256 |
| 203535_at | S100A9; MIF; NIF; P14; CAGB; CFAG; CGLB; L1AG; LIAG; MRP14; 60B8AG; MAC387 | S100 calcium-binding protein A9 | | | NM_002965 | 6280 |
| 203167_at | TIMP2; CSC-21K | tissue inhibitor of metalloproteinase 2 precursor | | | NM_003255 | 7077 |
| 204053_x_at | PTEN; BZS; MHAM; TEP1; MMAC1; PTEN1; MGC11227 | phosphatase and tensin homolog | | | U96180 | 5728 |
| 213006_at | CEBPD | | | | AV655640 | 1052 |
| 211404_s_at | APLP2; APPH; APPL2; CDEBP | amyloid beta (A4) precursor-like protein 2 | | | BC004371 | 334 |
| 214875_x_at | APLP2 | | | | AW001847 | 334 |
| 201200_at | CREG1 | cellular repressor of E1A-stimulated genes | | | NM_003851 | 8804 |
| 200645_at | GABARAP; MM46 | GABA(A) receptor-associated protein | | | NM_007278 | 11337 |
| 202803_s_at | ITGB2; LAD; CD18; MF17; MFI7; LCAMB; LFA-1 | integrin beta chain, beta 2 precursor | | | NM_000211 | 3689 |
| 209288_s_at | CDC42EP3; UB1; CEP3; BORG2 | Cdc42 effector protein 3 | | | AL136842 | 10602 |
| 200701_at | NPC2; HE1; NP-C2; MGC1333 | Niemann-Pick disease, type C2 precursor | | | NM_006432 | 10577 |
| 221541_at | DKFZP434B044 | hypothetical protein DKFZp434B044 | | | AL136861 | 83716 |
| 205068_s_at | GRAF | | | | BE671084 | 23092 |
| M2.7 | Total = 43 transcripts | | 0 | Overexpressed | | |
| | | | 0 | Underexpressed | | |
| | | | | | | |
| M2.8 | Total = 104 transcripts | | 1 | Overexpressed | | |
| | | | 29 | Underexpressed | | |

| Systematic | Common _Affy | Product | | | Genbank | LocusLink |
|---|---|---|---|---|---|---|
| 204019_s_at | SH3YL1; Ray; DKFZP586F1318 | SH3 domain containing, Ysc84-like 1 | | | NM_015677 | 26751 |
| 219315_s_at | C16orf30; CLP24; FLJ20898 | claudin-like protein 24 | | | NM_024600 | 79652 |
| 212413_at | SEPT6; SEP2; KIAA0128; MGC16619; MGC20339 | septin 6 isoform B; septin 6 isoform A; septin 6 isoform D | | | D50918 | 23157 |
| 213340_s_at | KIAA0495 | KIAA0495 | | | AB007964 | 57212 |
| 218877_s_at | C6orf75; MDS024; dJ187J11.2 | chromosome 6 open reading frame 75 | | | NM_021820 | 60487 |
| 216262_s_at | TGIF2 | | | | AL050318 | 60436 |
| 213971_s_at | JJAZ1 | | | | AI924660 | 23512 |
| 212549_at | | | | | AL080218 | |
| 221558_s_at | LEF1; TCF1ALPHA | lymphoid enhancer binding factor-1 | | | AF288571 | 51176 |
| 214482_at | ZNF46; KUP; ZBTB25 | zinc finger protein 46 (KUP) | | | NM_006977 | 7597 |
| 218259_at | MKL2; MRTF-B; NPD001 | megakaryoblastic leukemia 2 protein | | | NM_014048 | 57496 |
| 202969_at | DYRK2 | dual-specificity tyrosine-(Y)-phosphorylation regulated kinase 2 isoform 1; dual-specificity tyrosine-(Y)-phosphorylation regulated kinase 2 isoform 2 | | | Y09216 | |
| 218918_at | MAN1C1; HMIC; MAN1A3 | mannosidase, alpha, class 1C, member 1 | | | NM_020379 | 57134 |
| 219765_at | FLJ12586 | hypothetical protein FLJ12586 | | | NM_024620 | 79673 |
| 212771_at | LOC221061 | | | | AU150943 | |
| 216945_x_at | PASK; STK37; PASKIN; KIAA0135; DKFZP434O051 | PAS domain containing serine/threonine kinase | | | U79240 | 23178 |
| 218510_x_at | FLJ20152 | | | | AI816291 | 54463 |
| 212642_s_at | HIVEP2 | | | | AL023584 | 3097 |
| 212313_at | MGC29816 | hypothetical protein MGC29816 | | | BC004344 | 91782 |
| 219724_s_at | KIAA0748 | | | | NM_014796 | 9840 |
| 212400_at | | | | | AL043266 | |
| 213039_at | ARHGEF18; KIAA0521; MGC15913 | Rho-specific guanine nucleotide exchange factor p114 | | | AB011093 | 23370 |
| 218437_s_at | LZTFL1 | leucine zipper transcription factor-like 1 | | | NM_020347 | 54585 |
| 221601_s_at | TOSO | | | | AI084226 | 9214 |
| 221602_s_at | TOSO | regulator of Fas-induced apoptosis | | | AF057557 | 9214 |
| 219734_at | SIDT1; SID1; SID-1; FLJ20174; B830021E24Rik | SID1 transmembrane family, member 1 | | | NM_017699 | 54847 |
| 206337_at | CCR7; BLR2; EBI1; CDw197; | chemokine (C-C motif) receptor 7 precursor | | | NM_001838 | 1236 |
| 218932_at | FLJ20729; FLJ20760 | hypothetical protein FLJ20729 | | | NM_017953 | 54680 |
| 215967_s_at | LY9 | | | | AL582804 | 4063 |
| 57082_at | ARH | | | | AA169780 | 26119 |
| M2.9 | Total = 122 transcripts | | 2 | Overexpressed | | |
| | | | 5 | Underexpressed | | |

| Systematic | Common _Affy | Product | | | Genbank | LocusLink |
|---|---|---|---|---|---|---|
| 211537_x_at | MAP3K7; TAK1; TGF1a | mitogen-activated protein kinase kinase kinase 7 isoform A; mitogen-activated protein kinase kinase kinase 7 isoform B; mitogen-activated | | | AF218074 | 6885 |
| | | protein kinase kinase kinase 7 isoform C; mitogen-activated protein kinase kinase kinase 7 isoform D | | | | |
| 210235_s_at | PPFIA1; LIP1; LIP.1; LIPRIN; MGC26800 | PTPRF interacting protein alpha 1 isoform b; PTPRF interacting protein alpha 1 isoform a | | | U22815 | 8500 |
| 200839_s_at | SMAP-5; SB140; FLJ30014 | golgi membrane protein SB140 | | | NM_030799 | 81555 |
| 200839_s_at | ENO1; NNE; PPH; MPB1; MBP-1; ENO1 | enolase 1 | | | U88968 | |
| 207724_s_at | SPG4; FSP2; ADPSP; SPAST; | spastin isoform 1; spastin isoform 2 | | | NM_014946 | 6683 |
| 221268_s_at | SGPP1; SPPasel | sphingosine-1-phosphatase | | | NM_030791 | 81537 |
| 201044_x_at | DUSP1 | | | | AA530892 | 1843 |
| M2.10 | Total = 44 transcripts | | 2 | Overexpressed | | |
| | | | 4 | Underexpressed | | |

| Systematic | Common _Affy | Product | | | Genbank | LocusLink |
|---|---|---|---|---|---|---|
| 221656_s_at | FLJ10521 | hypothetical protein FLJ10521 | | | BC003073 | 55160 |
| 208056_s_at | CBFA2T3; MTG16; MTGR2; ZMYND4 | myeloid translocation gene-related protein 2 isoform MTG16a; myeloid translocation gene-related protein 2 isoform MTG16b | | | NM_005187 | 863 |
| 207761_s_at | DKFZP586A0522 | DKFZP586A0522 protein | | | NM_014033 | 25840 |
| 201944_at | HEXB | hexosaminidase B peproprotein | | | NM_000521 | 3074 |
| 214030_at | DKFZp667G2110 | | | | BE501352 | 13154 |
| 221539_at | EIF4EBP1; PHAS-I | eukaryotic translation initiation factor 4E binding protein 1 | | | AB044548 | 1978 |
| M2.11 | Total = 77 transcripts | | 0 | Overexpressed | | |
| | | | 15 | Underexpressed | | |

| Systematic | Common _Affy | Product | | | Genbank | LocusLink |
|---|---|---|---|---|---|---|
| 203627_at | IGF1R; JTK13 | insulin-like growth factor 1 receptor precursor | | | NM_000875 | |
| 221830_at | RAP2A | | | | AI302106 | 5911 |
| 200839_s_at | TPR | | | | BF110993 | 7175 |
| 200839_s_at | YY1; DELTA; NF-E1; UCRBP; YIN-YANG-1 | YY1 transcription factor | | | NM_003403 | 7528 |
| 207700_s_at | NCOA3; ACTR; AIB1; RAC3; SRC3; pCIP; CTG26; CAGH16; TNRC14; TNRC16; TRAM-1 | nuclear receptor coactivator 3 isoform b; nuclear receptor coactivator 3 isoform a | | | NM_006534 | 8202 |
| 200839_s_at | SNX27; MY014; KIAA0488; MGC20471 | sorting nexin family member 27 | | | NM_030918 | 81609 |
| 211503_s_at | RAB14 | ras-related protein rab-14 | | | AF112206 | 51552 |
| 200839_s_at | ZNF148; BERF-1; BFCOL1; ZBP-89; ZFP148; pHZ-52; HT-BETA | zinc finger protein 148 (pHZ-52) | | | L04282 | 7707 |
| 202971_s_at | DYRK2 | dual-specificity tyrosine-(Y)-phosphorylation regulated kinase 2 isoform 1; dual-specificity tyrosine-(Y)-phosphorylation regulated kinase 2 isoform 2 | | | NM_006482 | 8445 |
| 200839_s_at | PRKCI; PKCI; DXS1179E; MGC26534; nPKC-iota | protein kinase C, iota | | | L18964 | 5584 |
| 209750 at | NR1D2 | | | | N32859 | 9975 |
| 200839_s_at | ALS2CR3 | | | | AV705253 | 66008 |
| 219757_s_at | C14orf101; FLJ20392 | chromosome 14 open reading frame 101 | | | NM_017799 | 54916 |
| 205798 at | IL7R; CD127; CDW127; IL-7R-alpha | interleukin 7 receptor precursor | | | NM_002185 | 3575 |
| 215342_s_at | RABGAP1L; HHL; TBC1D18; | RAB GTPase activating protein 1-like | | | AB019490 | 9910 |
| M3.1 | Total = 80 transcripts | | 0 | Overexpressed | | |
| | | | 21 | Underexpressed | | |

| Systematic | Common _Affy | Product | | | Genbank | LocusLink |
|---|---|---|---|---|---|---|
| 204747_at | IFIT3; IRG2; IFI60; IFIT4; ISG60; RIG- G; CIG-49; GARG-49 | interferon-induced repeats 3 protein with tetratricopeptide | | | NM_001549 | 3437 |
| 203882_at | ISGF3G; p48; IRF9; IRF-9 | interferon-stimulated gamma 48kDa transcription factor 3, | | | NM_006084 | 10379 |
| 211429_s_at | TRIM5; RNF88 | tripartite motif tripartite motif tripartite motif protein TRIM5 isoform alpha; protein TRIM5 isoform gamma; protein TRIM5 isoform delta | | | AF220028 | 85363 |
| 209761_s_at | SP110; IFI41; IFI75; FLJ22835 | SP110 nuclear body nuclear body protein body protein isoform protein isoform a; SP110 isoform b; SP110 nuclear c | | | AF280094 | 3431 |
| 202270_at | GBP1 | guanylate binding inducible, 67kD protein 1, interferon- | | | NM_002053 | 2633 |
| 219014_at | PLAC8; C15 | placenta-specific 8 | | | NM_016619 | 51316 |
| 211429_s_at | OAS1; OIAS; IFI-4; OIASI | 2',5'-oligoadenylate 2',5'-oligoadenylate synthetase 1 isoform E16; synthetase 1 isoform E18 | | | NM_002534 | 4938 |
| 219209_at | IFIH1; Hlcd; MDA5; MDA-5 | melanoma differentiation associated protein-5 | | | NM_022168 | 64135 |
| 202869_at | OAS1; OIAS; IFI-4; OIASI | 2',5'-oligoadenylate synthetase 1 isoform E16; 2',5'-oligoadenylate synthetase 1 isoform E18 | | | NM_016816 | 4938 |
| 213361_at | PCTAIRE2BP | | | | AW129593 | 23424 |
| 204972_at | OAS2; P69 | 2'-5'oligoadenylate synthetase 2 isoform p69; 2'-5'oligoadenylate synthetase 2 isoform p71 | | | NM_016817 | 4939 |
| 219439_at | C1GALT1 | core 1 alpha-R beta UDP-galactose:N-acetylgalactosamine-1,3-galactosyltransferase | | | NM_020156 | 56913 |
| 211429_s_at | STAT1; ISGF-3; STAT91 | signal transducer 1 isoform alpha; of transcription 1 and activator of transcription signal transducer and activator isoform beta | | | NM_007315 | 6772 |
| 218085_at | SNF7DC2 | SNF7 domain containing 2 | | | NM_015961 | 51510 |
| 211429_s_at | FLJ20035; FLJ10787 | hypothetical protein FLJ20035 | | | NM_017631 | 55601 |
| 206133_at | HSXIAPAF1; XAF1 | XIAP associated factor-1 isoform 1; XIAP associated factor-1 isoform 2 | | | NM_017523 | 54739 |
| 202687_s_at | TNFSF10; TL2; APO2L; TRAIL; Apo-2L | tumor necrosis factor member 10 (ligand) superfamily, | | | U57059 | 8743 |
| 213293_s_at | TRIM22 | | | | AA083478 | 10346 |
| 218943_s_at | DDX58; RIG-I; FLJ13599 | DEAD/H (Asp-Glu-Ala-Asp/His) polypeptide RIG-I box | | | NM_014314 | 23586 |
| 202269_x_at | GBP1 | guanylate binding inducible, 67kD protein 1, interferon- | | | BC002666 | 2633 |
| 219691_at | SAMD9; C7orf5; FLJ20073; KIAA2004 | sterile alpha motif domain containing 9 | | | NM_017654 | 54809 |
| M3.2 | Total = 230 transcripts | | 99 | Overexpressed | | |
| | | | 0 | Underexpressed | | |

| Systematic | Common _Affy | Product | | | Genbank | LocusLink |
|---|---|---|---|---|---|---|
| 218032_at | SNN | Stannin | | | AF070673 | 8303 |
| 211429_s_at | LMNA; FPL; LFP; EMD2; FPLD; HGPS; LDP1; LMN1; LMNC; PRO1; CMD1A; CMT2B1; LGMD1B | lamin A/C isoform 2; precursor; lamin A/C lamin A/C isoform 1 isoform 3 | | | NM_005572 | 4000 |
| 203140 at | BCL6; BCL5; LAZ3; BCL6A; ZNF51; ZBTB27 | B-cell lymphoma 6 protein | | | NM_001706 | 604 |
| 206115_at | EGR3; PILOT | early growth response 3 | | | NM_004430 | 1960 |
| 207993_s_at | CHP; SLC9A1BP | calcium binding protein P22 | | | NM_007236 | 11261 |
| 213191_at | TRIF; TICAM1; PRVTIRB; MGC35334 | TIR domain containing interferon-beta adaptor inducing | | | AF070530 | 14802 |
| 200868_s_at | ZNF313; RNF114 | zinc finger protein 313 | | | NM_018683 | 55905 |
| 201055_s_at | HNRPAO; hnRNPA0 | heterogeneous nuclear ribonucleoprotein A0 | | | NM_006805 | 10949 |
| 91682_at | FLJ20591 | | | | AI571298 | 54512 |
| 203003_at | MEF2D | | | | AL530331 | 4209 |
| 201329_s_at | ETS2 | v-ets erythroblastosis virus E26 oncogene | | | NM_005239 | 2114 |
| | | homolog 2 | | | | |
| 201739_at | SGK; SGK1 | serum/glucocorticoid regulated kinase | | | NM_005627 | 6446 |
| 211506_s_at | IL8 | interleukin 8 C-terminal variant | | | AF043337 | 3576 |
| 217996_at | PHLDA1 | | | | AA576961 | 22822 |
| 216268_s_at | JAG1; AGS; AHD; AWS; HJ1; JAGL1 | jagged 1 precursor | | | U77914 | 182 |
| 216260_at | DICER1; Dicer; HERNA; KIAA0928 | dicerl | | | AK001827 | 23405 |
| 202498_s_at | SLC2A3; GLUT3 | solute carrier family 2 (facilitated glucose transporter), member 3 | | | NM_006931 | 6515 |
| 203888_at | THBD; TM; THRM; CD141 | thrombomodulin precursor | | | NM_000361 | 7056 |
| 208092_s_at | FAM49A; FLJ11080; DKFZP566A1524 | family with sequence similarity 49, member A | | | NM_030797 | 81553 |
| 219257_s_at | SPHK1 | sphingosine kinase 1 | | | NM_021972 | 8877 |
| 212432_at | HMGE | | | | AL542571 | 80273 |
| 203887_s_at | THBD; TM; THRM; CD141 | thrombomodulin precursor | | | NM_000361 | 7056 |
| 219382_at | SERTAD3; RBT1 | RPA-binding trans-activator | | | NM_013368 | 29946 |
| 221654_s_at | USP3 | SIH003 | | | AF077040 | 9960 |
| 201858_s_at | PRG1; PPG; MGC9289; SERGLYCIN | proteoglycan 1, secretory granule precursor | | | J03223 | 5552 |
| 202672_s_at | ATF3 | activating transcription factor 3 long isoform; activating transcription factor 3 delta Zip isoform | | | NM_001674 | 467 |
| 201531_at | ZFP36; TTP; GOS24; TIS11; NUP475; RNF162A | zinc finger protein 36, C3H type, homolog | | | NM_003407 | 7538 |
| 202657_s_at | TRIP-Br2 | | | | NM_014755 | 9792 |
| 209808_x_at | ING1 | | | | AW193656 | 3621 |
| 201490_s_at | PPIF; CYP3 | peptidylprolyl isomerase F precursor | | | NM_005729 | 10105 |
| 203370_s_at | PDLIM7 | enigma protein isoform 1; enigma protein isoform 2; enigma protein isoform 3; enigma protein isoform 4 | | | NM_005451 | 9260 |
| 209545_s_at | RIPK2; RICK; RIP2; CARD3; CARDIAK | receptor-interacting serine-threonine kinase 2 | | | AF064824 | 8767 |
| 220088_at | C5R1; C5A; C5AR; CD88 | complement component 5 receptor 1 (C5a ligand) | | | NM_001736 | 728 |
| 217739_s_at | PBEF1 | pre-B-cell colony enhancing factor 1 isoform a; pre-B-cell colony enhancing factor 1 isoform b | | | NM_005746 | 10135 |
| 203045_at | NINJ1; NIN1; NINJURIN | ninjurin 1 | | | NM_004148 | 4814 |
| 208786_s_at | MAP1LC3B; MAP1A/1BLC3 | microtubule-associated proteins 1A/1B light chain 3 | | | AF183417 | 81631 |
| 217738_at | PBEF | | | | BF575514 | 10135 |
| 212769_at | TLE3 | | | | AI567426 | 7090 |
| 216236_s_at | SLC2A3; GLUT3 | solute carrier family 2 (facilitated glucose transporter), member 3 | | | AL110298 | 6515 |
| 201236_s_at | BTG2; PC3; TIS21 | B-cell translocation gene 2 | | | NM_006763 | 7832 |
| 220712_at | | | | | NM_024984 | |
| 205114_s_at | CCL3; MIP1A; SCYA3; G0S19-1; LD78ALPHA; MIP-1-alpha | chemokine (C-C motif) ligand 3 | | | NM_002983 | 6348 |
| 201170_s_at | BHLHB2; DEC1; STRA13; Stra14 | differentiated embryo chondrocyte expressed gene 1 | | | NM_003670 | 8553 |
| 210190_at | STX11 | syntaxin 11 | | | AF071504 | 8676 |
| 200797_s_at | MCL1; TM; EAT; MCL1L; MCL1S; MGC1839 | myeloid cell leukemia sequence 1 isoform 1; myeloid cell leukemia sequence 1 isoform 2 | | | NM_021960 | 4170 |
| 202284_s_at | CDKN1A; P21; CIP1; SDI1; WAF1; CAP20; MDA-6 | cyclin-dependent kinase inhibitor 1A | | | NM_000389 | 1026 |
| 36994_at | ATP6V0C; ATPL; VATL; Vma3; ATP6C; ATP6L | ATPase, H+ transporting, lysosomal, V0 subunit c | | | M62762 | 527 |
| 203094_at | MAD2L1BP; CMT2; KIAA0110; MGC11282 | MAD2L1 binding protein isoform 1; MAD2L1 binding protein isoform 2 | | | NM_014628 | 9587 |
| 201460_at | MAPKAPK2 | | | | AI141802 | 9261 |
| 217202_s_at | | glutamine synthetase | | | U08626 | |
| 204908_s_at | BCL3; BCL4; D19S37 | B-cell CLL/lymphoma 3 | | | NM_005178 | 602 |
| 200711_s_at | SKP1A | | | | NM_003197 | 6500 |
| 219434_at | TREM1; TREM-1 | triggering receptor expressed on myeloid cells 1 | | | NM_018643 | 54210 |
| 206472_s_at | TLE3; ESG; ESG3; HsT18976; KIAA1547 | transducin-like enhancer protein 3 | | | NM_005078 | 7090 |
| 213138_at | ARID5A; MRF-1 | AT rich interactive domain 5A isoform 2; AT rich interactive domain 5A isoform 1 | | | M62324 | 10865 |
| 37028_at | PPP1R15A; GADD34 | protein phosphatase 1, regulatory subunit 15A | | | U83981 | 23645 |
| 212146_at | PLEKHM2; KIAA0842 | KIAA0842 protein | | | AB020649 | 23207 |
| 38037_at | DTR; DTS; HBEGF; HEGFL | diphtheria toxin receptor (heparin-binding epidermal growth factor-like growth factor) | | | M60278 | 1839 |
| 212171_x_at | VEGF | | | | H95344 | 7422 |
| 204095_s_at | ELL | | | | AL521391 | 8178 |
| 202638_s_at | ICAM1; BB2; CD54 | intercellular adhesion molecule 1 precursor | | | NM_000201 | 3383 |
| 216015_s_at | CIAS1; FCU; MWS; FCAS; NALP3; Clorf7; PYPAF1; AII/AVP; AGTAVPRL | cryopyrin isoform a; cryopyrin isoform b | | | AK027194 | 11454 |
| 212723_at | PTDSR; PSR; PTDSR1; KIAA0585 | phosphatidylserine receptor | | | AK021780 | 23210 |
| 205409_at | FOSL2; FRA2; FLJ23306 | FOS-like antigen 2 | | | NM_005253 | 2355 |
| 203574_at | NFIL3; E4BP4; IL3BP1; NFIL3A; NF-IL3A | nuclear factor, interleukin 3 regulated | | | NM_005384 | 4783 |
| 213300_at | KIAA0404 | | | | AW168132 | 23130 |
| 204370_at | HEAB; CLP1; hClp1 | ATP/GTP-binding protein | | | NM_006831 | 10978 |
| 211458_s_at | GABARAPL3 | GABA-A receptor-associated protein | | | AF180519 | 23766 |
| 202497_x_at | SLC2A3; GLUT3 | solute carrier family 2 (facilitated glucose transporter), member 3 | | | NM_006931 | 6515 |
| 215501_s_at | DUSP10; MKP5; MKP-5 | dual specificity phosphatase 10 isoform a; dual specificity phosphatase 10 isoform b | | | AK022513 | 11221 |
| 218033_s_at | SNN | Stannin | | | NM_003498 | 8303 |
| 212770_at | TLE3 | | | | AI567426 | 7090 |
| 202014_at | PPP1R15A; GADD34 | protein phosphatase 1, regulatory subunit 15A | | | NM_014330 | 23645 |
| 210845_s_at | PLAUR; CD87; UPAR; URKR | plasminogen activator, urokinase receptor isoform 2 precursor; plasminogen activator, urokinase receptor isoform 3 precursor; plasminogen activator, urokinase receptor isoform 1 precursor | | | U08839 | 5329 |
| 203821_at | DTR; DTS; HBEGF; HEGFL | diphtheria toxin receptor (heparin-binding epidermal growth factor-like growth factor) | | | NM_001945 | 1839 |
| 200663_at | CD63; MLA1; ME491; LAMP-3; OMA81h | CD63 antigen | | | NM_001780 | 967 |
| 208785_s_at | MAP1LC3B | | | | BE893893 | 81631 |
| 201489_at | PPIF; CYP3 | peptidylprolyl isomerase F precursor | | | BC005020 | 10105 |
| 210365_at | RUNX1; AML1; CBFA2; AMLCR1; PEBP2A2; PEBP2aB | runt-related transcription factor 1 isoform b; runt-related transcription factor 1 isoform a | | | D43967 | 861 |
| 205349_at | GNA15; GNA16 | guanine nucleotide binding protein (G protein), alpha 15 (Gq class) | | | NM_002068 | 2769 |
| 222088_s_at | SLC2A14, | | | | AA778684 | 14419 |
| 218023_s_at | C5orf6 | putative nuclear protein | | | NM_016605 | 51307 |
| 202859_x_at | IL8; K60; NAF; GCP1; IL-8; LECT; LUCT; NAP1; 3-10C; CXCL8; GCP-1; LYNAP; MDNCF; MONAP; NAP-1; SCYB8; TSG-1; AMCF-I; b-ENAP | interleukin 8 precursor | | | NM_000584 | 3576 |
| 209304_x_at | GADD45B; MYD118; GADD45BETA; DKFZP566B133 | growth arrest and DNA-damage-inducible, beta | | | AF087853 | 4616 |
| 218881_s_at | FOSL2 | FOS-like antigen 2 | | | NM_024530 | 79579 |
| 200919_at | PHC2; EDR2; HPH2 | polyhomeotic 2-like isoform b; polyhomeotic 2-like isoform a | | | NM_004427 | 1912 |
| 203234_at | UPP1; UPASE; UDRPASE | uridine phosphorylase 1 | | | NM_003364 | 7378 |
| 214696_at | MGC14376; DKFZp686006159 | hypothetical protein MGC14376 | | | AF070569 | 84981 |
| 209345_s_at | PI4KII | | | | AL561930 | 55361 |
| 211924_s_at | PLAUR; CD87; UPAR; URKR | plasminogen activator, urokinase receptor isoform 2 precursor; plasminogen activator, urokinase receptor isoform 3 precursor; plasminogen activator, urokinase receptor isoform 1 precursor | | | AY029180 | 5329 |
| 210264_at | GPR35 | G protein-coupled receptor 35 | | | AF089087 | 2859 |
| 200730_s_at | PTP4A1 | | | | BF576710 | 7803 |
| 208937_s_at | ID1 | inhibitor of DNA binding 1 isoform a; inhibitor of DNA binding 1 isoform b | | | D13889 | 3397 |
| 218880_at | FOSL2 | | | | N36408 | 2355 |
| 212722_s_at | PTDSR; PSR; PTDSR1; KIAA0585 | phosphatidylserine receptor | | | AK021780 | 23210 |
| 219622_at | RAB20; FLJ20429 | RAB20, member RAS oncogene family | | | NM_017817 | 55647 |
| 208869_s_at | GABARAPL1; GEC1; APG8L | GABA(A) receptor-associated protein like 1 | | | AF087847 | 23710 |
| 213524_s_at | G0S2 | putative lymphocyte G0/G1 switch gene | | | NM_015714 | 50486 |
| 201631_s_at | IER3; DIF2; IEX1; PRG1; DIF-2; GLY96; IEX-1; IEX-1L | immediate early response 3 immediate early response 3 isoform short; isoform long | | | NM_003897 | 8870 |
| M3.3 | Total = 230 transcripts | | 54 | Overexpressed | | |
| | | | 6 | Underexpressed | | |

| Systematic | Common _Affy | Product | | | Genbank | LocusLink |
|---|---|---|---|---|---|---|
| 214500_at | H2AFY; H2A.y; H2A/y; H2AFJ; mH2A1; H2AF12M; MACROH2A1.1; MACROH2A1.2 | H2A histone family, member Y isoform 2; H2A histone family, member Y isoform 1; H2A histone family, member Y isoform 3 | | | AF044286 | 9555 |
| 216338_s_at | C6orf109; KLIP1; dJ337H4.3; DKFZP566C243 | chromosome 6 open reading frame 109 | | | AK021433 | 25844 |
| 217995_at | SQRDL; CGI-44 | sulfide dehydrogenase like | | | NM_021199 | 58472 |
| 211742_s_at | EVI2B; EVDB; D17S376 | ecotropic viral integration site 2B | | | BC005926 | 2124 |
| 218388_at | PGLS; 6PGL | 6-phosphogluconolactonase | | | NM_012088 | 25796 |
| 218017_s_at | FLJ22242 | | | | NM_025070 | 80140 |
| 207168_s_at | H2AFY; H2A.y; H2A/y; H2AFJ; mH2A1; H2AF12M; MACROH2A1.1; MACROH2A1.2 | H2A histone family, member Y isoform 2; H2A histone family, member Y isoform 1; H2A histone family, member Y isoform 3 | | | NM_004893 | 9555 |
| 220990_s_at | VMP1; DKFZP566I133 | hypothetical protein DKFZp566I133 | | | NM_030938 | 81671 |
| 205639_at | AOAH | acyloxyacyl hydrolase precursor | | | NM_001637 | 313 |
| 206488_s_at | CD36; FAT; GP4; GP3B; GPIV; PASIV; SCARB3 | CD36 antigen | | | NM_000072 | 948 |
| 201463_s_at | TALDO1; TAL-H; TALDOR | transaldolase 1 | | | NM_006755 | 6888 |
| 204620_s_at | CSPG2; VERSICAN | chondroitin sulfate proteoglycan 2 (versican) | | | NM_004385 | 1462 |
| 213733_at | MYO1F | | | | BF740152 | 4542 |
| 214152_at | PIGB | | | | AU144243 | 9488 |
| 210715_s_at | SPINT2; Kop; HAI2; HAI-2 | serine protease inhibitor, Kunitz type, 2 | | | AF027205 | 10653 |
| 212807_s_at | SORT1 | | | | BE742268 | 6272 |
| 217837_s_at | VPS24; NEDF; CGI-149 | vacuolar protein sorting 24 | | | NM_016079 | 51652 |
| 200808_s_at | ZYX | zyxin | | | NM_003461 | 7791 |
| 209575_at | IL10RB; CRFB4; CRF2-4; D21S58; D21S66; CDW210B; IL-10R2 | interleukin 10 receptor, beta precursor | | | BC001903 | 3588 |
| 220034_at | IRAK3; IRAK-M | interleukin-1 receptor-associated kinase 3 | | | NM_007199 | 11213 |
| 202788_at | MAPKAPK3; 3PK; MAPKAP3 | mitogen-activated protein kinase-activated protein kinase 3 | | | NM_004635 | 7867 |
| 205147_x_at | NCF4; MGC3810; P40PHOX | neutrophil cytosolic factor 4 (40kD) isoform 1; neutrophil cytosolic factor 4 (40kD) isoform 2 | | | NM_000631 | 4689 |
| 209473_at | ENTPD1 | | | | AV717590 | 953 |
| 212268_at | SERPINB1; EI; LEI; PI2; MNEI; M/NEI; ELANH2 | serine (or cysteine) proteinase inhibitor, clade B (ovalbumin), member 1 | | | NM_030666 | 1992 |
| 209616_s_at | CES1; CEH; CES2; HMSE; SES1; HMSE1 | carboxylesterase 1 (monocyte/macrophage serine esterase 1) | | | S73751 | 1066 |
| 202888_s_at | ANPEP; CD13; LAP1; PEPN; gp150 | membrane alanine aminopeptidase precursor | | | NM_001150 | 290 |
| 208270_s_at | RNPEP; DKFZP547H084 | arginyl aminopeptidase (aminopeptidase B) | | | NM_020216 | 6051 |
| 201118_at | PGD; 6PGD | phosphogluconate dehydrogenase | | | NM_002631 | 5226 |
| 200824_at | GSTP1; PI; DFN7; GST3; FAEES3 | glutathione transferase | | | NM_000852 | 2950 |
| 212657_s_at | IL1RN | | | | AW083357 | 3557 |
| 89476_r_at | NPEPL1 | | | | AA398062 | 79716 |
| 201095_at | DAP | death-associated protein | | | NM_004394 | 1611 |
| 208700_s_at | TKT; TKT1 | transketolase | | | L12711 | 7086 |
| 201483_s_at | SUPT4H1; SPT4H | suppressor of Ty 4 homolog 1 | | | BC002802 | 6827 |
| 203175_at | RHOG; ARHG | ras homolog gene family, member G | | | NM_001665 | 391 |
| 201470_at | GST01; P28; GSTTLp28 | glutathione-S-transferase omega 1 | | | NM_004832 | 9446 |
| 215706_x_at | ZYX | zyxin | | | BC002323 | 7791 |
| 215489_x_at | HOMER3 | | | | AI871287 | 9454 |
| 208699_x_at | TKT | | | | BF696840 | 7086 |
| 218945_at | MGC2654; FLJ12433 | hypothetical protein MGC2654 | | | NM_024109 | 79091 |
| 200941_at | HSBP1 | heat shock factor binding protein 1 | | | AK026575 | 3281 |
| 202096_s_at | BZRP; MBR; PBR; PBR-S | peripheral benzodiazapine receptor isoform PBR; peripheral benzodiazapine receptor isoform PBR-S | | | NM_000714 | 706 |
| 212043_at | TGOLN2; TGN38; TGN46; TGN48; TGN51; TTGN2; MGC14722 | trans-golgi network protein 2 | | | AK025557 | |
| 208074_s_at | AP2S1; AP17; CLAPS2; AP17-DELTA | adaptor-related protein complex 2, sigma 1 subunit isoform AP17; adaptor-related protein complex 2, sigma 1 subunit isoform AP17delta | | | NM_021575 | 1175 |
| 219256_s_at | SH3TC1; FLJ20356 | SH3 domain and tetratricopeptide repeats 1 | | | NM_018986 | 54436 |
| 202671_s_at | PDXK; PKH; PNK; C21orf97 | pyridoxal kinase | | | NM_003681 | 8566 |
| 211047_x_at | AP2S1; AP17; CLAPS2; AP17-DELTA | adaptor-related protein complex 2, sigma 1 subunit isoform AP17; adaptor-related protein complex 2, sigma 1 subunit isoform AP17delta | | | BC006337 | 1175 |
| 218606_at | ZDHHC7; ZNF370; FLJ10792; FLJ20279 | zinc finger, DHHC domain containing 7 | | | NM_017740 | 55625 |
| 204099_at | SMARCD3; Rsc6p; BAF60C; CRACD3 | SWI/SNF related, matrix associated, actin dependent regulator of chromatin, subfamily d, member 3 isoform 2; SWI/SNF related, matrix associated, actin dependent regulator of chromatin, subfamily d, member 3 isoform 1 | | | NM_003078 | 6604 |
| 207809_s_at | ATP6AP1; 16A; CF2; ORF; Ac45; XAP3; XAP-3; ATP6S1; VATPS1; ATP6IP1 | ATPase, H+ transporting, lysosomal accessory protein 1 precursor | | | NM_001183 | 537 |
| 221059_s_at | CHST6; MCDC1 | carbohydrate (N-acetylglucosamine 6-O) sulfotransferase 6 | | | NM_021615 | 4166 |
| 205863_at | S100A12; p6; CGRP; MRP6; CAAF1; ENRAGE | S100 calcium-binding protein A12 | | | NM_005621 | 6283 |
| 201379_s_at | TPD52L2; D54; hD54 | tumor protein D52-like 2 isoform e; tumor protein D52-like 2 isoform f; tumor protein D52-like 2 isoform a; tumor protein D52-like 2 isoform b; tumor protein D52-like 2 isoform c; tumor protein D52-like 2 isoform d | | | NM_003288 | 7165 |
| 213902_at | ASAH1 | | | | AI379338 | 427 |
| 206130_s_at | ASGR2; L-H2; ASGP-R; Hs.1259 | asialoglycoprotein receptor 2 isoform a; asialoglycoprotein receptor 2 isoform b; asialoglycoprotein receptor 2 isoform c | | | NM_001181 | 433 |
| 219549_s_at | RTN3; ASYIP; NSPL2; NSPLII | reticulon 3 isoform a; reticulon 3 isoform b; reticulon 3 isoform c; reticulon 3 isoform d | | | NM_006054 | 10313 |
| 204214_s_at | RAB32 | RAB32, member RAS oncogene family | | | NM_006834 | 10981 |
| 203081_at | CTNNBIP1; ICAT | beta-catenin-interacting protein ICAT | | | NM_020248 | 56998 |
| 200736_s_at | GPX1; GSHPX1; MGC14399 | glutathione peroxidase 1 isoform 1; glutathione peroxidase 1 isoform 2 | | | NM_000581 | 2876 |
| 200866_s_at | PSAP; GLBA; SAP1 | prosaposin (variant Gaucher disease and variant metachromatic leukodystrophy) | | | M32221 | 5660 |
| M3.4 | Total = 323 transcripts | | 1 | Overexpressed | | |
| | | | 107 | Underexpressed | | |

| Systematic | Common _Affy | Product | | | Genbank | LocusLink |
|---|---|---|---|---|---|---|
| 208841_s_at | G3BP2 | Ras-GTPase activating protein SH3 domain-binding protein 2 isoform a; Ras-GTPase activating protein SH3 domain-binding protein 2 isoform b | | | AB014560 | 9908 |
| 201385_at | DHX15; DBP1; HRH2; DDX15; PRP43; PrPp43p | DEAH (Asp-Glu-Ala-His) box polypeptide 15 | | | NM_001358 | 1665 |
| 221502_at | KPNA3 | | | | AL120704 | 3839 |
| 212200_at | KIAA0692 | KIAA0692 protein | | | AB014592 | 5451 |
| 212180_at | CRKL | v-crk sarcoma virus CT10 oncogene homolog (avian)-like | | | AK000311 | |
| 209689_at | FLJ10996; MGC13033 | hypothetical protein FLJ10996 | | | BC005078 | 83609 |
| 221493_at | TSPYL1; SIDDT | hypothetical protein | | | AL136629 | 7259 |
| 214467_at | GPR65; TDAG8; hTDAG8 | G protein-coupled receptor 65 | | | NM_003608 | 8477 |
| 218263_s_at | LOC58486 | Buster1 transposase-like protein | | | NM_021211 | 58486 |
| 201934_at | PR02730 | | | | N92524 | 80335 |
| 215716_s_at | ATP2B1; PMCA1 | plasma membrane calcium ATPase 1 isoform 1a; plasma membrane calcium ATPase 1 isoform 1b | | | L14561 | 490 |
| 202265_at | BMI1; RNF51; MGC12685 | B lymphoma Mo-MLV insertion region | | | NM_005180 | 648 |
| 203303_at | TCTE1L; TCTEX1L | t-complex-associated-testis-expressed 1-like | | | NM_006520 | 8971 |
| 201031_s_at | HNRPH1; hnRNPH | heterogeneous nuclear ribonucleoprotein H1 | | | NM_005520 | 3187 |
| 209654_at | KIAA0947 | KIAA0947 protein | | | BC004902 | 23379 |
| 210346_s_at | | CLK4 | | | AF212224 | |
| 204512_at | HIVEP1; MBP-1; ZNF40; PRDII-BF1 | human immunodeficiency virus type I enhancer binding protein 1 | | | NM_002114 | 3096 |
| 204009_s_at | KRAS2 | | | | W80678 | 3845 |
| 55692_at | ELM02 | | | | W22924 | 63916 |
| 208896_at | DDX18; MrDb | DEAD (Asp-Glu-Ala-Asp) box polypeptide 18 | | | BC003360 | 8886 |
| 207956_x_at | APRIN; AS3; CG008; FLJ23236; KIAA0979 | androgen-induced prostate proliferative shutoff associated protein | | | NM_015928 | 23047 |
| 207996_s_at | C18orf1 | chromosome 18 open reading frame 1 isoform gamma 1; chromosome 18 open reading frame 1 isoform gamma 2; chromosome 18 open reading frame 1 isoform beta 1; chromosome 18 open reading frame 1 isoform alpha 1; chromosome 18 open reading frame 1 isoform alpha | | | NM_004338 | 753 |
| 221596_s_at | DKFZP564O0523 | hypothetical protein DKFZp56400523 | | | AL136619 | 84060 |
| 212706_at | RASA4; GAPL; CAPRI; KIAA0538 | RAS p21 protein activator 4 | | | AB011110 | 10156 |
| 202653_s_at | AXOT; MARCH-VII; DKFZP586F1122 | axotrophin | | | BC003404 | 64844 |
| 208661_s_at | TTC3; DCRR1; RNF105; TPRDIII | tetratricopeptide repeat domain 3 | | | D84294 | 7267 |
| 212366_at | ZNF292 | | | | AA972711 | 23036 |
| 212984_at | | | | | BE786164 | |
| 212569_at | KIAA0650 | | | | AA868754 | 23347 |
| 214855_s_at | GARNL1; GRIPE; TULIP1; KIAA0884; DKFZp566D133; | GTPase activating Rap/RanGAP domain-like 1 isoform 1; GTPase activating Rap/RanGAP domain-like 1 isoform 2 | | | AL050050 | 26134 |
| 214363_s_at | MATR3 | | | | AA129420 | 9782 |
| 202853_s_at | RYK; RYK1; D3S3195 | RYK receptor-like tyrosine kinase precursor | | | NM_002958 | 6259 |
| 212855_at | KIAA0276 | KIAA0276 protein | | | D87466 | 23142 |
| 202386_s_at | LKAP; KIAA0430; A-362G6.1 | limkain b1 isoform 1; limkain b1 isoform 2 | | | NM_019081 | |
| 213372_at | LOC152559 | | | | AW173157 | |
| 221020_s_at | MFTC | mitochondrial folate transporter/carrier | | | NM_030780 | 81034 |
| 217812_at | YTHDF2; HGRG8; NY-REN-2 | high glucose-regulated protein 8 | | | NM_016258 | 51441 |
| 221559_s_at | MIS12; hMis12; MGC2488; 2510025F08Rik | MIS12 homolog | | | BC000229 | 79003 |
| 208127_s_at | SOCS5; CIS6; CISH6; Cish5; SOCS-5; KIAA0671 | suppressor of cytokine signaling 5 | | | NM_014011 | 9655 |
| 209187_at | DR1 | | | | AW516932 | 1810 |
| 202918_s_at | PREI3; 2C4D; MOB1; MOB3; CGI-95; MGC12264 | preimplantation protein 3 isoform 1; preimplantation protein 3 isoform 2 | | | AF151853 | 25843 |
| 209451_at | TANK; TRAF2; I-TRAF | TRAF interacting protein TANK isoform a; TRAF interacting protein TANK isoform b | | | U59863 | 10010 |
| 217863_at | PIAS1; GBP; DDXBP1; GU/RH-II | protein inhibitor of activated STAT, 1 | | | NM_016166 | |
| 215696_s_at | KIAA0310 | KIAA0310 protein | | | BC001404 | 9919 |
| 221905_at | CYLD; EAC; CDMT; CYLD1; HSPC057; KIAA0849 | cylindromatosis (turban tumor syndrome) | | | AJ250014 | 1540 |
| 209666_s_at | CHUK; IKK1; IKKA; IKBKA; TCF16; NFKBIKA; IKK-alpha | conserved helix-loop-helix ubiquitous kinase | | | AF080157 | 1147 |
| 212920_at | | | | | AV682285 | |
| 218172_s_at | DER1 | derlin-1 | | | NM_018630 | 55493 |
| 213212_x_at | | | | | AI632181 | |
| 213227_at | PGRMC2 | | | | BE879873 | 10424 |
| 203250_at | RBM16; KIAA1116 | RNA-binding motif protein 16 | | | NM_014892 | 22828 |
| 218352_at | RCBTB1; GLP; CLLD7; CLLL7; FLJ10716 | regulator of chromosome condensation (RCC1) and BTB (POZ) domain containing protein 1 | | | NM_018191 | 55213 |
| 212927_at | SMC5L1; KIAA0594 | SMC5 protein | | | AB011166 | 23137 |
| 201713_s_at | RANBP2; NUP358 | RAN binding protein 2 | | | D42063 | 5903 |
| 221257_x_at | FBX038; MOKA; Fbx38; SP329 | F-box protein 38 isoform a; F-box protein 38 isoform b | | | NM_030793 | 81545 |
| 212006_at | UBXD2; UBXDC1; KIAA0242 | UBX domain containing 2 | | | D87684 | 23190 |
| 218386_x_at | USP16; UBP-M | ubiquitin specific protease 16 isoform b; ubiquitin specific protease 16 isoform a | | | NM_006447 | 10600 |
| 211375_s_at | ILF3; MMP4; MPP4; NF90; NFAR; TCP80; DRBP76; NFAR-1; MPHOSPH4; NF-AT-90 | interleukin enhancer binding factor 3 isoform b; interleukin enhancer binding factor 3 isoform a; interleukin enhancer binding factor 3 isoform c | | | AF141870 | 3609 |
| 203403_s_at | RNF6 | ring finger protein 6 isoform 1; ring finger protein 6 isoform 2 | | | NM_005977 | 6049 |
| 218649_x_at | SDCCAG1; NY-CO-1; FLJ10051 | serologically defined colon cancer antigen 1 | | | NM_004713 | 9147 |
| 209724_s_at | ZFP161 | | | | AL534416 | 7541 |
| 214709_s_at | KTN1; CG1; KNT; KIAA0004 | kinectin 1 | | | Z22551 | 3895 |
| 212588_at | PTPRC | | | | AI809341 | 5788 |
| 219031_s_at | CGI-37; HSPC031 | Saccharomyces cerevisiae Nip7p homolog | | | NM_016101 | 51388 |
| 218499_at | MST4; MASK | serine/threonine protein kinase MASK | | | NM_016542 | 51765 |
| 218674_at | FLJ13611 | hypothetical protein FLJ13611 | | | NM_024941 | 80006 |
| 216944_s_at | ITPR1; IP3R; IP3R1; Insp3r1 | inositol 1,4,5-triphosphate receptor, type 1 | | | U23850 | 3708 |
| 221568_s_at | LIN7C; VELI3; LIN-7C; MALS-3; LIN-7-C; FLJ11215 | lin-7 homolog C | | | AF090900 | 55327 |
| 217833_at | SYNCRIP; pp68; NSAP1; GRY-RBP; dJ3J17.2 | synaptotagmin binding, cytoplasmic RNA interacting protein | | | NM_006372 | |
| 212231_at | FBXO21; FBX21; KIAA0875; DKFZp434G058 | F-box only protein 21 isoform 2; F-box only protein 21 isoform 1 | | | AK001699 | 23014 |
| 213070_at | | | | | AV682436 | |
| 208671_at | TDE2; TDE1L; TMS-2; KIAA1253 | tumor differentially expressed 2 | | | AF164794 | 57515 |
| 201486_at | RCN2; E6BP; ERC55; ERC-55 | reticulocalbin 2, EF-hand calcium binding domain | | | NM_002902 | 5955 |
| 201437_s_at | EIF4E; CBP; EIF-4E; EIF4EL1 | eukaryotic translation initiation factor 4E | | | NM_001968 | 1977 |
| 221970_s_at | DKFZP586L0724 | | | | AU158148 | 25926 |
| 218098_at | ARFGEF2; BIG2; dJ1164I10.1 | ADP-ribosylation factor guanine nucleotide-exchange factor 2 | | | NM_006420 | |
| 212536_at | ATP11B; ATPIF; ATPIR; KIAA0956; MGC46576; DKFZP434J238; DKFZP434N1615 | KIAA0956 protein | | | AB023173 | 23200 |
| 206695_x_at | ZNF43; HTF6; KOX27; ZNF39L1 | zinc finger protein 43 (HTF6) | | | NM_003423 | 7594 |
| 203301_s_at | DMTF1; DMP1; hDMP1 | cyclin D binding myb-like transcription factor 1 | | | NM_021145 | 9988 |
| 202673_at | DPM1; MPDS; CDGIE | dolichyl-phosphate mannosyltransferase polypeptide 1 | | | NM_003859 | 8813 |
| 211946_s_at | XTP2; BAT2-iso; KIAA1096 | HBxAg transactivated protein 2 | | | AL096857 | 23215 |
| 201769_at | ENTH; EPN4; EPNR; Clint; EPSINR; KIAA0171 | enthoprotin | | | NM_014666 | 9685 |
| 221751_at | | | | | AL565516 | |
| 201603_at | PPP1R12A; MBS; MYPT1 | protein phosphatase 1, regulatory (inhibitor) subunit 12A | | | NM_002480 | 4659 |
| 213025_at | FLJ20274 | | | | AL134904 | 55623 |
| 218566_s_at | CHORDC1; CHP1 | cysteine and histidine-rich domain (CHORD)-containing, zinc binding protein 1 | | | NM_012124 | 26973 |
| 209022_at | STAG2 | | | | AK026678 | 10735 |
| 222204_s_at | RRN3; DKFZp566E104 | RRN3 RNA polymerase I transcription factor homolog | | | AL110238 | 54700 |
| 200899_s_at | MGEA5; MEA5; KIAA0679 | meningioma expressed antigen 5 (hyaluronidase) | | | NM_012215 | 10724 |
| 209115_at | UBE1C; UBA3; hUba3; MGC22384; DKFZp566J164 | ubiquitin-activating enzyme E1C isoform 1; ubiquitin-activating enzyme E1C isoform 2; ubiquitin-activating enzyme E1C isoform 3 | | | AL117566 | 9039 |
| 218432_at | FBX03; FBA; FBX3; DKFZp564B092 | F-box only protein 3 isoform 1; F-box only protein 3 isoform 2 | | | NM_012175 | 26273 |
| 201260_s_at | SYPL; H-SP1 | synaptophysin-like protein isoform a; synaptophysin-like protein isoform b | | | NM_006754 | 6856 |
| 209028_s_at | ABI1; E3B1; NAP1; ABI-1; SSH3BP1 | abl-interactor 1 | | | AF006516 | 10006 |
| 201672_s_at | USP14; TGT | ubiquitin specific protease 14 | | | NM_005151 | 9097 |
| 216321_s_at | NR3C1; GR; GCR; GRL | nuclear receptor subfamily 3, group C, member 1 (glucocorticoid receptor) | | | X03348 | 2908 |
| 205269_at | LCP2 | | | | AI123251 | 3937 |
| 221229_s_at | FLJ20628; DKFZp564I2178 | hypothetical protein FLJ20628 | | | NM_017910 | 55006 |
| 213000_at | ZCWCC3; NXP2; ZCW5; KIAA0136 | zinc finger, CW-type with coiled-coil domain 3 | | | AP000693 | 23515 |
| 203531_at | | | | | BF435809 | |
| 218096_at | LPAAT-e; FLJ11210 | acid acyltransferase-epsilon | | | NM_018361 | 55326 |
| 203378_at | PCF11; KIAA0824 | pre-mRNA cleavage complex II protein Pcf11 | | | AB020631 | 51585 |
| 212450_at | KIAA0256 | KIAA0256 gene product | | | D87445 | 9728 |
| 203347_s_at | M96; MTF2 | putative DNA binding protein | | | NM_007358 | 22823 |
| 201699_at | PSMC6; P44; p42; SUG2; CADP44; MGC12520 | proteasome 26S ATPase subunit 6 | | | NM_002806 | 5706 |
| 215245_x_at | FMR1 | | | | AA830884 | 2332 |
| 212633_at | KIAA0776 | | | | AW298092 | 23376 |
| 218577_at | FLJ20331 | hypothetical protein FLJ20331 | | | NM_017768 | 55631 |
| 218878_s_at | SIRT1; SIR2L1 | sirtuin 1 | | | NM_012238 | 23411 |
| M3.5 | Total = 19 transcripts | | 3 | Overexpressed | | |
| | | | 0 | Underexpressed | | |

| Systematic | Common _Affy | Product | | | Genbank | LocusLink |
|---|---|---|---|---|---|---|
| 217052_x_at | | | | | AK024108 | |
| 208749_x_at | FLOT1 | flotillin 1 | | | AF085357 | 10211 |
| 200630_x_at | SET | | | | AV702810 | 6418 |
| M3.6 | Total = 233 transcripts | | 2 | Overexpressed | | |
| | | | 76 | Underexpressed | | |

| Systematic | Commo | Product | | | Genbank | Locus |
|---|---|---|---|---|---|---|
| 202603_at | ADAM10 | | | | N51370 | 102 |
| 214895_s_at | ADAM10 | | | | AU135154 | 102 |
| 200723_s_at | M11S1; GPIAP1 | membrane component, chromosome 11, surface marker 1 isoform 1; membrane component, chromosome 11, surface marker 1 | | | NM_005898 | 4076 |
| 218973_at | EFTUD1; FAM42A; FLJ13119; HsT19294 | elongation factor Tu GTP binding domain containing 1 | | | NM_024580 | 79631 |
| 205078_at | PIGF; MGC32646; MGC33136 | phosphatidylinositol glycan, class F isoform 1; phosphatidylinositol glycan, class F isoform 2 | | | NM_002643 | 5281 |
| 217815_at | SUPT16H; FACT; CDC68; FACTP140; FLJ10857; FLJ14010; | chromatin-specific transcription elongation factor large subunit | | | NM_007192 | 11198 |
| 204215_at | C7orf23; MGC4175; MM-TRAG SPT16/CDC68 | chromosome 7 open reading frame 23 | | | NM_024315 | 79161 |
| 213149_at | DLAT | | | | AW299740 | 1737 |
| 202970_at | DYRK2 | dual-specificity tyrosine-(Y)-phosphorylation regulated kinase 2 isoform 1; dual-specificity tyrosine-(Y)-phosphorylation regulated kinase 2 isoform 2 | | | Y09216 | |
| 201633_s_at | CYB5-M | | | | AW235051 | 80777 |
| 203073_at | COG2; LDLC | component of oligomeric golgi complex 2 | | | NM_007357 | 22796 |
| 209600_s_at | ACOX1; MGC1198; PALMCOX | acyl-Coenzyme A oxidase isoform a; acyl-Coenzyme A oxidase isoform b | | | S69189 | 51 |
| 200687_s_at | SF3B3; RSE1; SAP130; SF3b130; STAF130; KIAA0017 | splicing factor 3b, subunit 3, 130kDa | | | NM_012426 | 23450 |
| 206572_x_at | ZNF85; HPF4; HTF1 | zinc finger protein 85 (HPF4, HTF1) | | | NM_003429 | 7639 |
| 217939_s_at | AFTIPHILIN; FLJ20080; FLJ23793; MGC33965 | aftiphilin protein isoform c; aftiphilin protein isoform b; aftiphilin protein isoform a | | | NM_017657 | 54812 |
| 203201_at | PMM2; CDG1; CDGS | phosphomannomutase 2 | | | NM_000303 | 5373 |
| 208716_s_at | LOC54499 | putative membrane protein | | | AB020980 | 54499 |
| 204185_x_at | PPID; CYPD; CYP-40; MGC33096 | peptidylprolyl isomerase D | | | NM_005038 | 5481 |
| 212216_at | KIAA0436; FLJ16627 | putative prolyl oligopeptidase | | | AB007896 | 9581 |
| 211337_s_at | 76P | gamma tubulin ring complex protein (76p gene) | | | BC000966 | 27229 |
| 204172_at | CPOX; CPX; HCP | coproporphyrinogen oxidase | | | NM_000097 | 1371 |
| 205055_at | ITGAE; CD103; HUMINAE | integrin, alpha E (antigen CD103, human mucosal lymphocyte antigen 1; alpha polypeptide) | | | NM_002208 | 3682 |
| 203306_s_at | SLC35A1; CST; HCST; CMPST; inactive | solute carrier family 35 (CMP-sialic acid transporter), member A1 | | | NM_006416 | 10559 |
| 204683_at | ICAM2; CD102 | intercellular adhesion molecule 2 precursor | | | NM_000873 | 3384 |
| 202654_x_at | AXOT; MARCH-VII; DKFZP586F1122 | axotrophin | | | NM_022826 | 64844 |
| 207627_s_at | TFCP2; LSF; LBP-1C; TFCP2C | transcription factor CP2 | | | NM_005653 | 7024 |
| 209366_x_at | CYB5 | cytochrome b-5 | | | M22865 | 1528 |
| 202663_at | | | | | AI005043 | |
| 214179_s_at | NFE2L1 | | | | H93013 | 4779 |
| 217921_at | | | | | H97940 | |
| 219017_at | ETNK1; EKI; EKI1 | ethanolamine kinase 1 | | | NM_018638 | 55500 |
| 201128_s_at | ACLY; ATPCL; CLATP | ATP citrate lyase isoform 1; ATP citrate lyase isoform 2 | | | NM_001096 | 47 |
| 212264_s_at | KIAA0261; FOE | KIAA0261 | | | D87450 | 23063 |
| 204642_at | EDG1; ECGF1; EDG-1; S1PR1; CHEDG1; D1S3362 | endothelial differentiation, sphingolipid G-protein-coupled receptor, 1 | | | NM_001400 | 1901 |
| 200683_s_at | UBE2L3; E2-F1; L-UBC; UBCH7; UbcM4 | ubiquitin-conjugating enzyme E2L 3 isoform 1; ubiquitin-conjugating enzyme E2L 3 isoform 2 | | | NM_003347 | 7332 |
| 201500_s_at | PPP1R11; HCGV; HCG-V; TCTE5; TCTEX5 | protein phosphatase 1, regulatory (inhibitor) subunit 11 isoform 1; protein phosphatase 1, regulatory (inhibitor) subunit 11 isoform 2 | | | NM_021959 | 6992 |
| 218313_s_at | GALNT7; GalNAcT7; GALNAC-T7 | polypeptide N-acetylgalactosaminyltransferase 7 | | | NM_017423 | 51809 |
| 207490_at | TUBA4; FLJ13940 | tubulin, alpha 4 | | | NM_025019 | 80086 |
| 200979_at | | | | | BF739979 | |
| 211547_s_at | PAFAH1B1; LIS1; MDCR | platelet-activating factor acetylhydrolase, isoform Ib, alpha subunit (45kD) | | | L13387 | 5048 |
| 214582_at | PDE3B | phosphodiesterase 3B, cGMP-inhibited | | | NM_000753 | 5140 |
| 204634_at | NEK4; NRK2; STK2 | NIMA (never in mitosis gene a)-related kinase 4 | | | NM_003157 | 6787 |
| 219283_at | C1GALT2 | core 1 UDP-galactose:N-acetylgalactosamine-alpha-R beta 1,3-galactosyltransferase 2 | | | NM_014158 | 29071 |
| 205263_at | BCL10; CLAP; mE10; CIPER; c-E10; CARMEN | B-cell CLL/lymphoma 10 | | | AF082283 | 8915 |
| 205202_at | PCMT1 | protein-L-isoaspartate (D-aspartate) O-methyltransferase | | | NM_005389 | 5110 |
| 201746_at | TP53; p53; TRP53 | tumor protein p53 | | | NM_000546 | 7157 |
| 206989_s_at | SFRS2IP; SIP1; CASP11; SRRP129 | splicing factor, arginine/serine-rich 2, interacting protein | | | NM_004719 | 9169 |
| 218538_s_at | MRS2L; HPT | MRS2-like, magnesium homeostasis factor | | | NM_020662 | 57380 |
| 218646_at | FLJ20534 | hypothetical protein FLJ20534 | | | NM_017867 | 54969 |
| 200703_at | DNCL1; LC8; PIN; DLC1; DLC8; hdlc1 | cytoplasmic dynein light polypeptide | | | NM_003746 | 8655 |
| 213246_at | C14orf109 | | | | AI346504 | 26175 |
| 213063_at | FLJ11806 | | | | N64802 | 79882 |
| 203284_s_at | HS2ST1; KIAA0448 | heparan sulfate 2-O-sulfotransferase 1 | | | NM_012262 | 9653 |
| 202451_at | GTF2H1; BTF2; TFIIH | general transcription factor IIH, polypeptide 1, 62kDa | | | BC000365 | 2965 |
| 201359_at | COPB; DKFZp761K102 | coatomer protein complex, subunit beta | | | NM_016451 | 1315 |
| 209384_at | PROSC | | | | AA176833 | 11212 |
| 213853_at | ZCSL3 | zinc finger, CSL domain containing 3 | | | AL050199 | |
| 202297_s_at | RER1 | RER1 homolog | | | AF157324 | 11079 |
| 212507_at | RW1; PRO1048; KIAA0257 | | | | D87446 | 23505 |
| 204613_at | PLCG2 | phospholipase C, gamma 2 (phosphatidylinositol-specific) | | | NM_002661 | 5336 |
| 201634_s_at | CYBS-M | cytochrome b5 outer mitochondrial membrane precursor | | | NM_030579 | 80777 |
| 201240_s_at | KIAA0102 | KIAA0102 gene product | | | NM_014752 | 9789 |
| 201295_s_at | WSB1; SWIP1; WSB-1 | WD SOCS-box protein 1 isoform 1; WD SOCS-box protein 1 isoform 3; WD SOCS-box protein 1 isoform 2 | | | NM_015626 | 26118 |
| 200996_at | ACTR3; ARP3 | ARP3 actin-related protein 3 homolog | | | NM_005721 | 10096 |
| 211760_s_at | VAMP4; VAMP24 | vesicle-associated membrane protein 4 | | | BC005974 | 8674 |
| 200994_at | IPO7; RANBP7 | importin 7 | | | AL137335 | 10527 |
| 201985_at | KIAA0196 | KIAA0196 gene product | | | NM_014846 | 9897 |
| 201518_at | CBX1; M31; MOD1; HP1-BETA; HP1Hs-beta | chromobox homolog 1 (HP1 beta homolog Drosophila ) | | | NM_006807 | 10951 |
| 201098_at | COPB2; beta'-COP | coatomer protein complex, subunit beta 2 (beta prime) | | | NM_004766 | 9276 |
| 201479_at | DKC1; NAP57; NOLA4; XAP101; dyskerin | dyskerin | | | NM_001363 | 1736 |
| 202811_at | STAMBP; AMSH | STAM binding protein | | | NM_006463 | 10617 |
| 200050_at | ZNF146; OZF | zinc finger protein 146 | | | NM_007145 | 7705 |
| 201472_at | VBP1; PFD3; PFDN3; VBP-1 | von Hippel-Lindau binding protein 1 | | | NM_003372 | 7411 |
| 205898_at | CX3CR1; V28; CCRL1; GPR13; CMKDR1; GPRV28; CMKBRL1 | chemokine (C-X3-C motif) receptor 1 | | | U20350 | 1524 |
| 213102_at | ACTR3 | | | | Z78330 | 10096 |
| 201661_s_at | ACSL3; ACS3; FACL3; PR02194 | acyl-CoA synthetase long-chain family member 3 | | | NM_004457 | 2181 |
| 212412_at | | | | | AV715767 | |
| 203008_x_at | TXNDC9; APACD | ATP binding protein associated with cell differentiation | | | NM_005783 | 10190 |
| M3.7 | Total = 80 transcripts | | 2 | Overexpressed | | |
| | | | 16 | Underexpressed | | |

| Systematic | Common _Affy | Product | | | Genbank | LocusLink |
|---|---|---|---|---|---|---|
| 202266_at | TTRAP; EAP2; AD022; MGC9099; dJ30M3.3 | TRAF and TNF receptor-associated protein | | | NM_016614 | 51567 |
| 214096_s_at | SHMT2 | | | | AW190316 | 6472 |
| 201966_at | NDUFS2 | NADH dehydrogenase (ubiquinone) Fe-S protein 2, 49kDa (NADH-coenzyme Q reductase) | | | NM_004550 | 4720 |
| 203721_s_at | CGI-48 | CGI-48 protein | | | NM_016001 | 51096 |
| 211061_s_at | MGAT2; GNT2; CDGS2; GNT-II; GLCNACTII | alpha-1,6-mannosyl-glycoprotein beta-1,2-N-acetylglucosaminyltransferase | | | BC006390 | 4247 |
| 200045_at | ABCF1; ABC27; ABC50; EST123147 | ATP-binding cassette, sub-family F, member 1 | | | NM_001090 | 23 |
| 203576_at | BCAT2; BCAM; BCT2 | branched chain aminotransferase 2, mitochondrial | | | NM_001190 | 587 |
| 221622_s_at | HT007 | uncharacterized hypothalamus protein HT007 | | | AF246240 | 55863 |
| 205644_s_at | SNRPG; SMG | small nuclear ribonucleoprotein polypeptide G | | | NM_003096 | 6637 |
| 218288_s_at | MDS025; MDS011 | hypothetical protein MDS025 | | | NM_021825 | 60492 |
| 201697_s_at | DNMT1; MCMT; CXXC9 | DNA (cytosine-5-)-methyltransferase 1 | | | NM_001379 | 1786 |
| 201176_s_at | ARCN1; COPD | archain | | | NM_001655 | 372 |
| 219220_x_at | MRPS22; GIBT; GK002; C3orf5; RPMS22; MRP-S22 | mitochondrial ribosomal protein S22 | | | NM_020191 | 56945 |
| 202520_s_at | MLH1; FCC2; COCA2; HNPCC; hMLH1; HNPCC2; MGC5172 | MutL protein homolog 1 | | | NM_000249 | 4292 |
| 211070_x_at | DBI; ACBP; ACBD1 | diazepam binding inhibitor | | | BC006466 | 1622 |
| 201241_at | DDX1; DBP-RB | DEAD (Asp-Glu-Ala-Asp) box polypeptide 1 | | | NM_004939 | 1653 |
| 208985_s_at | EIF3S1; eIF3-p35; eIF3-alpha | eukaryotic translation initiation factor 3, subunit 1 alpha, 35kDa | | | BC002719 | 8669 |
| 202911_at | MSH6; GTBP; HNPCC5 | mutS homolog 6 | | | NM_000179 | 2956 |
| M3.8 | Total = 182 transcripts | | 1 | Overexpressed | | |
| | | | 33 | Underexpressed | | |

| Systematic | Common _Affy | Product | | | Genbank | LocusLink |
|---|---|---|---|---|---|---|
| 213670_x_at | WBSCR20C | | | | AI768378 | 15540 |
| 203551_s_at | COX11; COX11P | COX11 homolog | | | NM_004375 | 1353 |
| 215743_at | RPP38 | | | | AL134489 | 10557 |
| 217902_s_at | HERC2; jdf2; p528; D15F37S1; KIAA0393 | hect domain and RLD 2 | | | NM_004667 | 8924 |
| 212944_at | | | | | AK024896 | |
| 212126_at | | | | | BG391282 | |
| 201837_s_at | STAF65 (gamma); ART1; KIAA0764 | SPTF-associated factor 65 gamma | | | AF197954 | 9913 |
| 204142_at | HSRTSBETA | rTS beta protein isoform 2; rTS beta protein isoform 1 | | | NM_017512 | 55556 |
| 203135_at | TBP; GTF2D; SCA17; TFIID; GTF2D1 | TATA box binding protein | | | NM_003194 | 6908 |
| 212037_at | PNN; DRS; SDK3 | pinin, desmosome associated protein | | | Y09703 | 5411 |
| 212454_x_at | HNRPDL | | | | AI762552 | 9987 |
| 206150_at | TNFRSF7; T14; CD27; S152; Tp55; MGC20393 | tumor necrosis factor receptor superfamily, member 7 precursor | | | NM_001242 | 939 |
| 202979_s_at | ZF | HCF-binding transcription factor Zhangfei | | | NM_021212 | 58487 |
| 215307_at | | | | | AL109722 | |
| 213703_at | LOC150759 | | | | W95043 | |
| 212914_at | CBX7 | | | | AV648364 | |
| 203944_x_at | BTN2A1; BTF1; BT2.1 | butyrophilin, subfamily 2, member A1 isoform 1 precursor; butyrophilin, subfamily 2, member A1 isoform 2 precursor | | | NM_007049 | 11120 |
| 221264_s_at | TARDBP | TAR DNA binding protein | | | NM_031214 | 81927 |
| 218557_at | NIT2 | nitrilase family, member 2 | | | NM_020202 | 56954 |
| 221626_at | ZNF506; DKFZp761G1812 | | | | AL136548 | |
| 213213_at | DATF1 | | | | AL035669 | 11083 |
| 219698_s_at | METTL4; HsT661; FLJ23017 | methyltransferase like 4 | | | NM_022840 | 64863 |
| 213653_at | | | | | AW069290 | |
| 212179_at | C6orf111; FLJ14752; FLJ14992; bA98I9.2; DKFZp564B0769 | chromosome 6 open reading frame 111 | | | AL080186 | 25957 |
| 219169_s_at | TFB1M; CGI75; mtTFB; CGI-75 | transcription factor B1, mitochondrial | | | NM_016020 | 51106 |
| 40569_at | ZNF42; MZF1; MZF-1; MZF1B | zinc finger protein 42 isoform 1; zinc finger protein 42 isoform 2 | | | M58297 | 7593 |
| 220081_x_at | HSD17B7; PRAP; MGC12523; MGC75018 | hydroxysteroid (17-beta) dehydrogenase 7 | | | NM_016371 | 51478 |
| 218373_at | FTS; FT1; FLJ13258 | fused toes homolog | | | NM_022476 | 64400 |
| 202220_at | KIAA0907 | | | | NM_014949 | 22889 |
| 220035_at | NUP210; GP210; POM210; FLJ22389; KIAA0906 | nucleoporin 210 | | | NM_024923 | 23225 |
| 209007_s_at | NPD014; DJ465N24.2.1 | NPD014 protein isoform 2; NPD014 protein isoform 1 | | | AF267856 | 57035 |
| 218343_s_at | GTF3C3; TFIIIC102; TFIIICgamma; TFiiiC2-102 | general transcription factor IIIC, polypeptide 3, 102kDa | | | NM_012086 | 9330 |
| 213483_at | KIAA0073 | KIAA0073 protein | | | AK025679 | 23398 |
| 204352_at | TRAF5; RNF84; MGC:39780 | TNF receptor-associated factor 5 | | | NM_004619 | 7188 |
| M3.9 | Total = 261 transcripts | | 0 | Overexpressed | | |
| | | | 135 | Underexpressed | | |

| Systematic | Common _Affy | Product | | | Genbank | LocusLink |
|---|---|---|---|---|---|---|
| 204236_at | FLI1; EWSR2; SIC-1 | Friend leukemia virus integration 1 | | | NM_002017 | 2313 |
| 201501_s_at | GRSF1 | G-rich RNA sequence binding factor 1 | | | NM_002092 | 2926 |
| 213883_s_at | BBP | | | | AA012917 | 83941 |
| 203629_s_at | COG5 | | | | AU152134 | 10466 |
| 209199_s_at | MEF2C | | | | N22468 | 4208 |
| 208289_s_at | EI24; PIG8; TP53I8 | etoposide induced 2.4 | | | NM_004879 | 9538 |
| 204333_s_at | AGA; AGU | aspartylglucosaminidase precursor | | | NM_000027 | 175 |
| 209382_at | POLR3C; RPC3; RPC62 | polymerase (RNA) III (DNA directed) polypeptide C (62kD) | | | U93867 | 10623 |
| 211987_at | TOP2B; TOPIIB | DNA topoisomerase II, beta isozyme | | | NM_001068 | 7155 |
| 204274_at | EBAG9 | | | | AA812215 | 9166 |
| 212585_at | OSBPL8; ORP8; OSBP10 | oxysterol-binding protein-like protein 8 isoform b; oxysterol-binding protein-like protein 8 isoform a | | | AB040884 | 11488 |
| 217724_at | PAI-RBP1 | | | | AF131807 | 26135 |
| 218452_at | SMARCAL1; HARP; HHARP | SWI/SNF-related matrix-associated actin-dependent regulator of chromatin a-like 1 | | | NM_014140 | 50485 |
| 212648_at | DHX29; DDX29 | DEAH (Asp-Glu-Ala-His) box polypeptide 29 | | | AL079292 | 54505 |
| 200988_s_at | PSME3; Ki; PA28G; REG-GAMMA; PA28-gamma | proteasome activator subunit 3 isoform 1; proteasome activator subunit 3 isoform 2 | | | NM_005789 | 10197 |
| 209786_at | HMGN4; NHC; HMG17L3; MGC5145 | high mobility group nucleosomal binding domain 4 | | | BC001282 | 10473 |
| 209943_at | FBXL4; FBL4; FBL5 | F-box and leucine-rich repeat protein 4 | | | AF176699 | 26235 |
| 201990_s_at | CREBL2 | cAMP responsive element binding protein-like 2 | | | NM_001310 | 1389 |
| 218460_at | FLJ20397 | hypothetical protein FLJ20397 | | | NM_017802 | 54919 |
| 217858_s_at | ARMCX3; ALEX3; MGC12199 | ALEX3 protein | | | NM_016607 | 51566 |
| 221744_at | HAN11 | WD-repeat protein | | | AK026008 | 10238 |
| 203983_at | TSNAX; TRAX | translin-associated factor X | | | NM_005999 | 7257 |
| 201034_at | ADD3 | | | | BE545756 | 120 |
| 205771_s_at | AKAP7; AKAP18 | A-kinase anchor protein 7 isoform alpha; A-kinase anchor protein 7 isoform gamma; A-kinase anchor protein 7 isoform beta | | | AL137063 | 9465 |
| 218536_at | | | | | AF052167 | |
| 210111_s_at | | PNAS-138 | | | AF277175 | |
| 219485_s_at | PSMD10; p28 | proteasome 26S non-ATPase subunit 10 isoform 1; proteasome 26S non-ATPase subunit 10 isform 2 | | | NM_002814 | 5716 |
| 219007_at | NUP43; p42; FLJ13287; bA350J20.1 | nucleoporin 43kDa | | | NM_024647 | 79700 |
| 201517_at | NCBP2; NIP1; CBP20 | nuclear cap binding protein subunit 2, 20kDa | | | BC001255 | 22916 |
| 209206_at | SEC22L1 | | | | AV701283 | 9554 |
| 213154_s_at | BICD2; KIAA0699 | bicaudal D homolog 2 isoform 1; bicaudal D homolog 2 isoform 2 | | | AB014599 | 23299 |
| 207305_s_at | KIAA1012; HsT2706 | KIAA1012 | | | NM_014939 | 22878 |
| 209247_s_at | ABCF2; ABC28; M-ABC1; HUSSY-18; EST133090; DKFZp586K1823 | ATP-binding cassette, sub-family F, member 2 isoform b; ATP-binding cassette, sub-family F, member 2 isoform a | | | BC001661 | 10061 |
| 212476_at | CENTB2; ACAP2; CNT-B2; KIAA0041 | centaurin, beta 2 | | | D26069 | 23527 |
| 65472_at | | | | | AI161338 | |
| 201833_at | HDAC2; RPD3; YAF1 | histone deacetylase 2 | | | NM_001527 | 3066 |
| 219296_at | ZDHHC13; HIP14L; HIP3RP; FLJ10852; FLJ10941; MGC64994 | zinc finger, DHHC domain containing 13 isoform 2; zinc finger, DHHC domain containing 13 isoform 1 | | | NM_019028 | 54503 |
| 218699_at | RAB7L1 | | | | BG338251 | 8934 |
| 222103_at | ATF1 | | | | AI434345 | 466 |
| 200902_at | 15-Sep | 15 kDa selenoprotein isoform 1 precursor; 15 kDa selenoprotein isoform 2 precursor | | | NM_004261 | 9403 |
| 213390_at | C19orf7; KIAA1064 | KIAA1064 protein | | | AB028987 | 23211 |
| 218396_at | VPS13C | vacuolar protein sorting 13C protein | | | NM_017684 | 54832 |
| 203048_s_at | KIAA0372 | | | | BE566023 | 9652 |
| 203537_at | PRPSAP2; PAP41 | phosphoribosyl pyrophosphate synthetase-associated protein 2 | | | NM_002767 | 5636 |
| 201036_s_at | HADHSC; SCHAD | L-3-hydroxyacyl-Coenzyme A dehydrogenase, short chain | | | NM_005327 | 3033 |
| 203566_s_at | AGL; GDE | amylo-1,6-glucosidase, 4-alpha-glucanotransferase isoform 1; amylo-1,6-glucosidase, 4-alpha-glucanotransferase 4-alphisoform 1; amylo-1,6-glucosidase, 4-alpha-glucanotransferase isoform 2; amylo-1,6-glucosidase, 4-alpha-glucanotransferase isoform 3 | | | NM_000645 | 178 |
| 218593_at | RBM28; FLJ10377 | RNA binding motif protein 28 | | | NM_018077 | 55131 |
| 202318_s_at | SENP6; SSP1; SUSP1; KIAA0797 | SUMO1/sentrin specific protease 6 | | | AF306508 | 26054 |
| 218838_s_at | FLJ12788 | hypothetical protein FLJ12788 | | | NM_022492 | 64427 |
| 212150_at | KIAA0143 | | | | AA805651 | 23167 |
| 218147_s_at | AD-017; FLJ14611 | glycosyltransferase AD-017 | | | NM_018446 | 55830 |
| 200760_s_at | JWA | | | | N92494 | 10550 |
| 211971_s_at | LRPPRC; LSFC; GP130; LRP130; CLONE-23970 | leucine-rich PPR motif-containing protein | | | AF052133 | 10128 |
| 218684_at | LRRC5; FLJ10470 | leucine rich repeat containing 5 | | | NM_018103 | 55144 |
| 201624_at | DARS | aspartyl-tRNA synthetase | | | NM_001349 | 1615 |
| 214198_s_at | DGCR2 | | | | AU150824 | 9993 |
| 218989_x_at | SLC30A5; ZNT5; ZTL1; ZNTL1; ZnT-5; MGC5499; FLJ12496; FLJ12756 | zinc transporter ZTL1 | | | NM_022902 | 64924 |
| 200860_s_at | KIAA1007; AD-005 | KIAA1007 protein isoform a; KIAA1007 protein isoform b | | | BC000779 | 23019 |
| 217886_at | EPS15 | | | | BF213575 | 2060 |
| 208953_at | KIAA0217 | KIAA0217 protein | | | BC003381 | 23185 |
| 205052_at | AUH | AU RNA-binding protein/enoyl-Coenzyme A hydratase precursor | | | NM_001698 | 549 |
| 202215_s_at | NFYC; HSM; CBFC; HAP5; CBF-C; NF-YC; H1TF2A | nuclear transcription factor Y, gamma | | | NM_014223 | 4802 |
| 202038_at | UBE4A; UFD2; KIAA0126 | ubiquitination factor E4A | | | NM_004788 | 9354 |
| 212530_at | NEK7 | NIMA (never in mitosis gene a)-related kinase 7 | | | AL080111 | 14060 |
| 212499_s_at | C14orf32; MISS; MGC23138; c14_5346 | MAPK-interacting and spindle-stabilizing protein | | | AK025580 | |
| 201375_s_at | PPP2CB | protein phosphatase 2 (formerly 2A), catalytic subunit, beta isoform | | | NM_004156 | 5516 |
| 209486_at | SAS10 | disrupter of silencing 10 | | | BC004546 | 57050 |
| 218491_s_at | THY28; MY105; MDS012; HSPC144; MGC12187 | thymocyte protein thy28 isoform 1; thymocyte protein thy28 isoform 2 | | | NM_014174 | 29087 |
| 218212_s_at | MOCS2; MPTS; MCBPE; MOCO1 | molybdopterin synthase large subunit MOCS2B; molybdopterin synthase small subunit MOCS2A | | | NM_004531 | 4338 |
| 210962_s_at | AKAP9; PRKA9; CG-NAP; YOTIAO; AKAP350; AKAP450; HYPERION; KIAA0803 | A-kinase anchor protein 9 isoform 2; A-kinase anchor protein 9 isoform 4; A-kinase anchor protein 9 isoform 1; A-kinase anchor protein 9 isoform 3 | | | AB019691 | 10142 |
| 203513_at | FLJ21439; KIAA1840 | hypothetical protein FLJ21439 | | | NM_025137 | 80208 |
| 213027_at | | | | | AU146655 | |
| 38892_at | KIAA0240 | | | | D87077 | 23506 |
| 203690_at | TUBGCP3; Spc98p | spindle pole body protein | | | NM_006322 | 10426 |
| 209551_at | MGC11061 | hypothetical protein MGC11061 | | | BC004875 | 84272 |
| 202850_at | ABCD3; ABC43; PMP70; PXMP1 | ATP-binding cassette, sub-family D, member 3 | | | NM_002858 | 5825 |
| 203738_at | FLJ11193 | | | | AI421192 | 55322 |
| 209200_at | MEF2C | | | | N22468 | 4208 |
| 201326_at | CCT6A | | | | BE737030 | 908 |
| 218128_at | NFYB | | | | AI804118 | 4801 |
| 218962_s_at | FLJ13576 | hypothetical protein FLJ13576 | | | NM_022484 | 64418 |
| 209143_s_at | CLNS1A; CLCI; ICln; CLNS1B | chloride channel, nucleotide-sensitive, 1A | | | AF005422 | 1207 |
| 204314_s_at | CREB1; MGC9284 | cAMP responsive element binding protein 1 isoform A; cAMP responsive element binding protein 1 isoform B | | | NM_004379 | 1385 |
| 201778_s_at | KIAA0494 | | | | NM_014774 | 9813 |
| 202664_at | | | | | AI005043 | |
| 204354_at | POT1; hPot1; DKFZP586D211 | POT1 protection of telomeres 1 homolog | | | NM_015450 | 25913 |
| 212640_at | LOC201562 | | | | AV712602 | |
| 203787_at | SSBP2; HSPC116 | single-stranded DNA binding protein 2 | | | NM_012446 | 23635 |
| 202346_at | HIP2; LIG; HYPG | huntingtin interacting protein 2 | | | NM_005339 | 3093 |
| 215548_s_at | SCFD1; RA410; KIAA0917; STXBP1L2; C14orf163 | vesicle transport-related protein isoform a; vesicle transport-related protein isoform b | | | AB020724 | 23256 |
| 212513_s_at | USP33; VDU1; KIAA1097; MGC16868 | ubiquitin specific protease 33 isoform 1; ubiquitin specific protease 33 isoform 2; ubiquitin specific protease 33 isoform 3 | | | AB029020 | 23032 |
| 212486_s_at | | | | | N20923 | |
| 212904_at | KIAA1185 | KIAA1185 protein | | | AB033011 | 57470 |
| 204020_at | PURA | | | | BF739943 | 5813 |
| 203465_at | MRPL19; RLX1; RPML15; MRP-L15; KIAA0104; MGC20675 | mitochondrial ribosomal protein L19 | | | NM_014763 | 9801 |
| 212943_at | KIAA0528 | KIAA0528 gene product | | | AB011100 | 9847 |
| 201296_s_at | WSB1; SWIP1; WSB-1 | WD SOCS-box protein 1 isoform 1; WD SOCS-box protein 1 isoform 3; WD SOCS-box protein 1 isoform 2 | | | NM_015626 | 26118 |
| 202214_s_at | CUL4B; KIAA0695 | cullin 4B | | | NM_003588 | 8450 |
| 218179_s_at | FLJ12716 | FLJ12716 protein isoform a; FLJ12716 protein isoform b | | | NM_021942 | 60684 |
| 214670_at | ZNF36 | | | | AA653300 | 7586 |
| 222201_s_at | CASP8AP2; CED-4; FLASH; RIP25; FLJ11208; KIAA1315 | CASP8 associated protein 2 | | | AB037736 | 9994 |
| 201218_at | CTBP2 | C-terminal binding protein 2 isoform 1; C-terminal binding protein 2 isoform 2 | | | NM_001329 | 1488 |
| 204980_at | CLOCK; KIAA0334 | clock | | | NM_004898 | 9575 |
| 208882_s_at | DD5 | | | | U69567 | 51366 |
| 201817_at | KIAA0010 | | | | NM_014671 | 9690 |
| 221196_x_at | C6.1A | c6.1A | | | NM_024332 | 79184 |
| 201493_s_at | PUM2 | | | | BE778078 | 23369 |
| 202956_at | ARFGEF1; BIG1; P200; ARFGEP1; D730028018Rik | brefeldin A-inhibited guanine nucleotide-exchange protein 1 | | | NM_006421 | 10565 |
| 218842_at | FLJ21908 | hypothetical protein FLJ21908 | | | NM_024604 | 79657 |
| 203253_s_at | KIAA0433 | KIAA0433 protein | | | NM_015216 | 23262 |
| 214113_s_at | RBM8A | | | | AI738479 | 9939 |
| 212299_at | NEK9; Nek8; NERCC; NERCC1; MGC16714; DKFZp434D0935 | NIMA related kinase | 9 | | AL117502 | 91754 |
| 200992_at | IPO7; RANBP7 | importin 7 | | | AL137335 | 10527 |
| 203689_s_at | FMR1 | | | | AI743037 | 2332 |
| 209323_at | PRKRIR; DAP4; P52rIPK | protein-kinase, interferon-inducible double stranded RNA dependent inhibitor, repressor of (P58 repressor) | | | AF081567 | 5612 |
| 218322_s_at | ACSL5; ACS2; ACS5; FACL5 | acyl-CoA synthetase long-chain family member 5 isoform a; acyl-CoA synthetase long-chain family member 5 isoform b | | | NM_016234 | 51703 |
| 38290_at | RGS14 | regulator of G-protein signalling 14 | | | AF037195 | 10636 |
| 34031_i_at | CCM1; CAM; KRIT1 | krev interaction trapped 1 | | | U90268 | 889 |
| 213090_s_at | TAF4 | | | | AI744029 | 6874 |
| 218545_at | FLJ11088; p56 | hypothetical protein FLJ11088 | | | NM_018318 | 55297 |
| 209268_at | VPS45A; H1; VSP45; VPS45B; VPS54A; VSP45A; H1VPS45 | vacuolar protein sorting 45A | | | AF165513 | 11311 |
| 203743_s_at | TDG | thymine-DNA glycosylase | | | NM_003211 | 6996 |
| 218047_at | OSBPL9; ORP9; FLJ12492; | oxysterol-binding protein-like protein 9 isoform | | | NM_024586 | 11488 |
| | FLJ14629; FLJ14801; FLJ32055; FLJ34384; MGC15035 | e; oxysterol-binding protein-like protein 9 isoform a; oxysterol-binding protein-like protein 9 isoform b; oxysterol-binding protein-like protein 9 isoform c; oxysterol-binding protein-like protein 9 isofor | | | | |
| 221873 at | ZNF143 | | | | AW162015 | 7702 |
| 211784_s_at | | | | | BC006181 | |
| 201448 at | TIA1 | TIA1 protein isoform 1; TIA1 protein isoform 2 | | | NM_022037 | |
| 221931_s_at | SEC13L | | | | AV701173 | 81929 |
| 221931_s_at | KIAA0372 | KIAA0372 | | | NM_014639 | 9652 |
| 201503 at | G3BP | | | | BG500067 | 10146 |
| 203791 at | DMXL1 | Dmx-like 1 | | | NM_005509 | 1657 |
| 219363_s_at | CGI-12 | CGI-12 protein | | | NM_015942 | 51001 |
| 202491_s_at | IKBKAP: FD; DYS; IKAP | inhibitor of kappa light polypeptide gene enhancer in B-cells, kinase complex-associated protein | | | NM_003640 | 8518 |
| 201589_at | SMC1L1; SMCB; SB1.8; DXS423E; KIAA0178; SMC1alpha | SMC1 structural maintenance of chromosomes 1-like 1 | | | D80000 | 8243 |
| 213238_at | ATP10D | | | | AI478147 | 57205 |
| 209523_at | TAF2; TAF2B; CIF150; TAFII150 | TBP-associated factor 2 | | | AK001618 | 6873 |

**Table 13: Module-by-module comparison of genes expression levels in liver transplant recipients vs. healthy volunteers. Liver transplant recipients (n=22) vs. Healthy Volunteer (n=27) - training set Mann Whitney U test p-value<0.05, no mtc**

| | | | | | | |
|---|---|---|---|---|---|---|
| M1.1 | Total = 69 transcripts | | p<0.05 | 1 | **Overexpressed** | |
| | | | | 42 | **Underexpressed** | |

| | **Healthy Normalized** | **Raw** | | **Liver Transplant Normalized** | **Raw** | **p-value** |
|---|---|---|---|---|---|---|
| 216510_x_at | 0.9535944 | 293.8852 | 0.68922555 | 230.9773 | 0.037 | **Underexpressed** |
| 211908_x_at | 0.90837705 | 178.65926 | 0.48615247 | 121.38636 | 0.0333 | **Underexpressed** |
| 214916_x_at | 1.0085313 | 918.9667 | 0.7224307 | 673.1591 | 0.0316 | **Underexpressed** |
| 216853_x_at | 0.9532237 | 192.05556 | 0.65257263 | 144.11365 | 0.0284 | **Underexpressed** |
| 211881_x_at | 1.0114585 | 395.59628 | 0.8215793 | 334.8909 | 0.0215 | **Underexpressed** |
| 217148_x_at | 0.9987921 | 1299.385 | 0.76691246 | 1122.8818 | 0.0191 | **Underexpressed** |
| 211641_x_at | 0.93511134 | 363.24442 | 0.6844703 | 278.39545 | 0.016 | **Underexpressed** |
| 216576_x_at | 0.9978868 | 505.27032 | 0.610412 | 395.1182 | 0.0134 | **Underexpressed** |
| 217480_x_at | 0.97234654 | 896.04816 | 0.7454202 | 709.15 | 0.00755 | **Underexpressed** |
| 214768_x_at | 0.98825425 | 465.96667 | 0.7120667 | 329.78638 | 0.00661 | **Underexpressed** |
| 211650_x_at | 0.78916377 | 269.76294 | 0.31672192 | 166.3909 | 0.00577 | **Underexpressed** |
| 217227_x_at | 1.0972255 | 284.1741 | 0.84450734 | 249.20454 | 0.00378 | **Underexpressed** |
| 217179_x_at | 0.92833304 | 451.8037 | 0.578116 | 359.58636 | 0.00352 | **Underexpressed** |
| 211798_x_at | 1.0213583 | 531.3816 | 0.56347245 | 331.82272 | 0.00085 | **Underexpressed** |
| 221931_s_at | 1.0176613 | 8300.245 | 0.24382864 | 4918.7866 | 0.000849 | **Underexpressed** |
| 215121_x_at | 0.98634666 | 9623.081 | 0.48235297 | 6273.741 | 0.000715 | **Underexpressed** |
| 215118_s_at | 1.0840191 | 703.87775 | 0.5814518 | 406.17273 | 0.000459 | **Underexpressed** |
| 215777_at | 0.9363759 | 87.022224 | 0.5081718 | 52.359093 | 0.000459 | **Underexpressed** |
| 211644_x_at | 1.0106673 | 871.6556 | 0.5211471 | 570.0091 | 0.000382 | **Underexpressed** |
| 221253_s_at | 1.035757 | 1441.9109 | 0.7921406 | 1241.8682 | 0.000348 | **Underexpressed** |
| 215379_x_at | 1.0907915 | 2435.1445 | 0.6510944 | 2274.6091 | 0.000262 | **Underexpressed** |
| 221931_s_at | 0.93837446 | 385.98148 | 0.6499519 | 293.54544 | 0.000216 | **Underexpressed** |
| 209138_x_at | 1.0315566 | 8949.182 | 0.5179802 | 5852.959 | 0.000178 | **Underexpressed** |
| 215214_at | 1.0720766 | 222.12222 | 0.620685 | 140.14091 | 0.000146 | **Underexpressed** |
| 216984_x_at | 1.025168 | 841.889 | 0.5098904 | 503.11816 | 0.000146 | **Underexpressed** |
| 217281_x_at | 1.0657202 | 226.53333 | 0.35082975 | 118.431816 | 0.000146 | **Underexpressed** |
| 221739_at | 0.97063583 | 1171.1519 | 1.1927835 | 1448.1772 | 0.000146 | **Overexpressed** |
| 216401_x_at | 0.93514395 | 426.41113 | 0.35121965 | 219.93636 | 0.000107 | **Underexpressed** |
| 214777_at | 0.8662704 | 399.81854 | 0.3215993 | 206.45456 | 0.000107 | **Underexpressed** |
| 215176_x_at | 1.0185648 | 1924.6481 | 0.52285767 | 1201.3545 | 0.00009 | **Underexpressed** |
| 209374_s_at | 0.9966258 | 5095.5405 | 0.59940046 | 3293.0908 | 0.000063 | **Underexpressed** |
| 217258_x_at | 0.9936845 | 222.2 | 0.43583792 | 126.109085 | 0.00005 | **Underexpressed** |
| 215946_x_at | 0.9841579 | 1175.8036 | 0.53852904 | 764.5227 | 0.00005 | **Underexpressed** |
| 214677_x_at | 1.0062306 | 9875.494 | 0.46147978 | 5883.8677 | 0.00004 | **Underexpressed** |
| 213502_x_at | 0.989173 | 2057.2258 | 0.58787936 | 1328.241 | 0.00001 | **Underexpressed** |
| 214669_x_at | 0.9705455 | 2949.4036 | 0.49041447 | 1899.9272 | 0.00000 | **Underexpressed** |
| 211645_x_at | 1.0296928 | 1275.4926 | 0.5159448 | 875.50006 | 0.00000 | **Underexpressed** |
| 205267_at | 1.0090047 | 1391.5444 | 0.55226755 | 816.43634 | 0.00000 | **Underexpressed** |
| 214836_x_at | 1.0482535 | 1661.7258 | 0.61028546 | 1019.04095 | 0.00000 | **Underexpressed** |
| 221651_x_at | 1.0553 | 14163.74 | 0.58122855 | 8642.6045 | 0.00000 | **Underexpressed** |
| 221671_x_at | 1.0026699 | 14210.46 | 0.52689755 | 8358.081 | 0.00000 | **Underexpressed** |
| 221931_s_at | 1.0306439 | 4548.656 | 0.25486428 | 2239.9727 | 0.00000 | **Underexpressed** |
| 212592_at | 1.0268843 | 1194.1445 | 0.35290387 | 647.8092 | 0.00000 | **Underexpressed** |
| M1.2 | Total = 96 transcripts | | | 1 | Overexpressed | |
| | | | | 25 | Underexpressed | |

| | Healthy Normalized | Raw | | Liver Transplant Normalized | Raw | p-value |
|---|---|---|---|---|---|---|
| 202708_x_at | 1.0291246 | 703.4631 | 1.293131 | 928.1227 | 0.037 | Overexpressed |
| 215492_x_at | 1.0371836 | 505.05554 | 0.84501696 | 420.0091 | 0.0333 | Underexpressed |
| 220496_at | 0.93682235 | 459.94073 | 0.72206074 | 372.5818 | 0.0333 | Underexpressed |
| 209806_at | 0.9521103 | 2293.4187 | 0.81702846 | 2045.5999 | 0.0299 | Underexpressed |
| 207815_at | 1.1267686 | 381.02594 | 0.6693667 | 246.11362 | 0.0247 | Underexpressed |
| 221931_s_at | 0.9734153 | 322.73703 | 0.818861 | 272.37726 | 0.0227 | Underexpressed |
| 221931_s_at | 0.9424713 | 1687.637 | 0.7506878 | 1352.9364 | 0.0203 | Underexpressed |
| 203680_at | 1.0543469 | 1530.6296 | 0.7015139 | 1100.2819 | 0.0181 | Underexpressed |
| 221931_s_at | 0.971758 | 136.51112 | 0.7027385 | 106.4591 | 0.0181 | Underexpressed |
| 221931_s_at | 0.9768981 | 2615.9995 | 0.62159055 | 1791.5591 | 0.0151 | Underexpressed |
| 220336_s_at | 0.9005469 | 229.16667 | 0.70483 | 178.05453 | 0.0134 | Underexpressed |
| 204115_at | 1.0053754 | 1191.0223 | 0.5847472 | 852.1319 | 0.0134 | Underexpressed |
| 201278_at | 0.9849579 | 355.89264 | 0.8393757 | 310.1182 | 0.0134 | Underexpressed |
| 206167_s_at | 0.9658439 | 181.8778 | 0.6749217 | 132.58638 | 0.00707 | Underexpressed |
| 221931_s_at | 0.99204427 | 153.80742 | 0.71427935 | 116.05909 | 0.00661 | Underexpressed |
| 221931_s_at | 1.0480876 | 1498.8186 | 0.7536483 | 1140.0544 | 0.00557 | Underexpressed |
| 205442_at | 0.9410932 | 403.21854 | 0.53875446 | 268.2682 | 0.00468 | Underexpressed |
| 203817_at | 0.9738554 | 329.04446 | 0.6397933 | 240.97728 | 0.00406 | Underexpressed |
| 221931_s_at | 1.0526993 | 317.59625 | 0.76514447 | 233.68182 | 0.00178 | Underexpressed |
| 212813_at | 1.0291963 | 436.27036 | 0.7281337 | 334.1091 | 0.00164 | Underexpressed |
| 206283_s_at | 0.97842056 | 356.47775 | 0.68749416 | 263.33636 | 0.00129 | Underexpressed |
| 217963_s_at | 0.9497207 | 2299.615 | 0.5692195 | 1455.3591 | 0.00129 | Underexpressed |
| 221211_s_at | 1.0132638 | 973.77783 | 0.60756016 | 625.14105 | 0.00085 | Underexpressed |
| 218999_at | 0.9999457 | 590.4556 | 0.6729006 | 404.76367 | 0.000119 | Underexpressed |
| 221931_s_at | 1.0227504 | 1134.2667 | 0.78785646 | 880.1727 | 0.00007 | Underexpressed |
| 221556_at | 0.9153534 | 344.37778 | 0.6053534 | 225.53183 | 0.00002 | Underexpressed |

| Module-by-module comparison of genes expression levels in liver transplant recipients vs. healthy volunteers. Liver transplant recipients (n=22) vs. Healthy Volunteer (n=27) - training set Mann Whitney U test p-value<0.05, no mtc | | | | | | |
|---|---|---|---|---|---|---|
| M1.1 Total = 69 transcripts | | p<0.05 | 1 | Overexpressed | | |
| | | | 42 | Underexpressed | | |

| LocusLink | Common | Product | | | Genbank | |
|---|---|---|---|---|---|---|
| 216510_x_at | IgH VH | immunoglobulin heavy chain | | | AB035175 | 3507 |
| 211908_x_at | IGHM | IgM | | | M87268 | 3507 |
| 214916_x_at | IGHM | | | | BG340548 | 3507 |
| 216853_x_at | IGLJ3 | immunoglobulin light chain variable region | | | AF234255 | 28831 |
| 211881_x_at | IGLJ3 | VEGF single chain antibody | | | AB014341 | 28831 |
| 217148_x_at | IGLV | immunoglobulin lambda variable region | | | AJ249377 | 28831 |
| 211641_x_at | IGH@ | immunoglobulin heavy chain V-region | | | L06101 | 3507 |
| 216576_x_at | | immunoglobulin kappa light chain variable region | | | AF103529 | |
| 217480_x_at | IGKV1oR15-118; IGKVP2; IGKV1oR118 | | | | M20812 | |
| 214768_x_at | IGKC | | | | BG540628 | 3514 |
| 211650_x_at | IGHM | | | | L34164 | 3507 |
| 217227_x_at | IGL@ | immunoglobulin lambda light chain VJC region | | | X93006 | 3535 |
| 217179_x_at | IGL@ | immunoglobulin lambda light chain | | | X79782 | 3535 |
| 211798_x_at | IGLJ3 | single-chain antibody | | | AB001733 | 28831 |
| 221931_s_at | IGHM | immunoglobulin A1-A2 lambda hybrid GAU heavy chain | | | S55735 | 3507 |
| 215121_x_at | IGLJ3 | | | | AA680302 | 28831 |
| 221931_s_at | | | | | AW519168 | |
| 215777_at_ | | | | | AW405975 | |
| 211644_x_at | IGKC | | | | L14458 | 3514 |
| 221253_s_at | TXNDC5; ERP46; UNQ364; EndoPDI; MGC3178 | thioredoxin domain containing 5 isoform 2; thioredoxin domain containing 5 isoform 1 | | | NM_030810 | 81567 |
| 215379_x_at | IGLJ3 | | | | AV698647 | 3535 |
| 221931_s_at | ITM2C; E25; BRI3; E25C; ITM3; BRICD2C | integral membrane protein 3 | | | NM_030926 | 81618 |
| 209138_x_at | IGLJ3 | | | | M87790 | 28831 |
| 215214_at | IGL@ | | | | H53689 | 3535 |
| 216984_x_at | IGLJ3 | immunoglobulin light chain V-J region | | | D84143 | 28831 |
| 217281_x_at | IGHV | immunoglobulin heavy chain variable region | | | AJ239383 | 3502 |
| 221739_at | IL27w | | | | AL524093 | 56005 |
| 216401_x_at | IGKV | immunoglobulin kappa chain variable region | | | AJ408433 | |
| 214777_at | IGKC | | | | BG482805 | 3514 |
| 215176_x_at | IGKC | | | | AW404894 | 3514 |
| 221931_s_at | IGHM; MU | IGHM protein | | | BC001872 | 3507 |
| 217258_x_at | IGL | immunoglobulin lambda chain | | | AF043583 | |
| 215946_x_at | LOC91316 | | | | AL022324 | 91316 |
| 214677_x_at | IGLJ3 | | | | X57812 | 28831 |
| 213502_x_at | IGLL3; 16.1 | lambda L-chain C region | | | X03529 | 91316 |
| 214669_x_at | IGKC | | | | BG485135 | 3514 |
| 211645_x_at | IgK | immunoglobulin kappa-chain VK-1 | | | M85256 | 3514 |
| 205267_at_ | POU2AF1; BOB1; OBF1; OCAB; OBF-1 | POU domain, class 2, associating factor 1 | | | NM_006235 | 5450 |
| 214836_x_at | IGKC | | | | BG536224 | 3514 |
| 221651_x_at | IGKC | Unknown (protein for MGC:12418) | | | BC005332 | 3514 |
| 221671_x_at | IGKC | | | | M63438 | 3514 |
| 211430_s_at | IGHG3 | | | | M87789 | 3502 |
| 212592_at | IGJ | | | | AV733266 | 3512 |
| M1.2 | Total = 96 transcripts | | 1 | Overexpressed | | |
| | | | 25 | Underexpressed | | |

| LocusLink | Common | Product | | | Genbank | |
|---|---|---|---|---|---|---|
| 202708_s_at | HIST2H2BE; GL105; H2B.1; H2B/q; H2BFQ | H2B histone family, member Q | | | NM_003528 | 8349 |
| 215492_x_at | PTCRA | | | | AL035587 | 17155 |
| 220496_at | CLEC2 | C-type lectin-like receptor-2 | | | NM_016509 | 51266 |
| 209806_at | HIST1H2BK; H2B/S; H2BFT; H2BFAiii | H2B histone family, member T | | | BC000893 | 85236 |
| 207815_at | PF4V1; PF4A; CXCL4V1; PF4-ALT; SCYB4V1 | platelet factor 4 variant 1 | | | NM_002620 | 5197 |
| 214039_s_at | LAPTM4B | | | | T15777 | 55353 |
| 208690_s_at | PDLIM1; CLIM1; CLP36; ELFIN; CLP-36; hCLIM1 | PDZ and LIM domain 1 (elfin) | | | BC000915 | 9124 |
| 203680_at | PRKAR2B; RII-BETA | cAMP-dependent protein kinase, regulatory subunit beta 2 | | | NM_002736 | 5577 |
| 207808_s_at | PROS1; PSA | protein S (alpha) | | | NM_000313 | 5627 |
| 215071_s_at | H2AFL | | | | AL353759 | 8334 |
| 220336_s_at | GP6; GPIV; GPVI | glycoprotein VI (platelet) | | | AB043821 | 51206 |
| 204115_at | GNG11 | guanine nucleotide binding protein (G protein), gamma 11 | | | NM_004126 | 2791 |
| 201278_at | | | | | N21202 | |
| 206167_s_at | ARHGAP6; RHOGAP6; rhoGAPX-1 | Rho GTPase activating protein 6 isoform 2; Rho GTPase activating protein 6 isoform 3; Rho GTPase activating protein 6 isoform 5; Rho GTPase activating protein 6 isoform 4; Rho GTPase activating protein 6 isoform 1 | | | NM_001174 | 395 |
| 221027_s_at | PLA2G12A | phospholipase A2, group XIIA | | | NM_030821 | 81579 |
| 215111_s_at | TGFB1I4; TSC22; MGC17597 | transforming growth factor beta 1 induced transcript 4 isoform 2; transforming growth factor beta 1 induced transcript 4 isoform 1 | | | AK027071 | 8848 |
| 205442_at_ | KIAA0626 | | | | NM_021647 | 9848 |
| 203817_at | GUCY1B3 | | | | W93728 | 2983 |
| 201280_s_at | DAB2; DOC2; DOC-2 | disabled homolog 2 | | | NM_001343 | 1601 |
| 212813_at | JAM3 | | | | AA149644 | 83700 |
| 206283_s_at | TAL1; SCL; TCL5 | T-cell acute lymphocytic leukemia 1 | | | NM_003189 | 6886 |
| 217963_s_at | NGFRAP1; Bex; BEX3; NADE; HGR74; DXS6984E | nerve growth factor receptor (TNFRSF16) associated protein 1 isoform b; nerve growth factor receptor (TNFRSF16) associated protein 1 isoform a | | | NM_014380 | 27018 |
| 221211_s_at | C21orf7; TAK1L | chromosome 21 open reading frame 7 | | | NM_020152 | 56911 |
| 218999_at | FLJ11000 | hypothetical protein FLJ11000 | | | NM_018295 | 55281 |
| 209586_s_at | HTCD37 | TcD37 homolog | | | AF123539 | 58497 |
| 221556_at | CDC14B | | | | AU145941 | 8555 |

## Claims

1. A method of identifying a subject with melanoma comprising:
determining two or more melanoma vectors of expression, wherein the two or more melanoma vectors of expression comprise the expression level of six or more genes over-expressed, under-expressed or combinations thereof selected from module M1.1 and module M1.2 of Table 12; and
displaying each of the melanoma expression vectors with a separate identifier.

2. The method of claim 1, further comprising the step of detecting one or more polymorphisms in the six or more genes.

3. The method of claim 1 further comprising a method for displaying melanoma transcriptome vector data comprising:
separating one or more genes into one or more modules to visually display an aggregate gene expression vector value for each of the modules; and
displaying the aggregate gene expression vector value for overexpression, underexpression or equal expression of the aggregate gene expression vector value in each module.

4. A method of identifying a subject with immunosuppression associated with transplants comprising:
determining two or more immunosuppression vectors of expression; and
displaying each of the immunosuppression vectors of expression with a separate identifier, wherein each immunosuppression vector of expression comprises levels of expression of six or more genes selected from module M1.1 and module M1.2 of Table 13.

5. The method of claim 4, further comprising the step of detecting one or more polymorphisms in the one or more immunosuppression vector of expression.

6. The method claim 4 or 5, wherein the one or more immunosuppression vectors of expression are derived from a leukocyte.

7. The method of any one of claims 4 to 6 comprising:
separating six or more genes into two or more modules to visually display, as an aggregate, a vector of expression for each of the modules; and
displaying the vector of expression for overexpression, underexpression or equal expression of the vector of expression in each module.

8. The method of any one of claims 1 to 7, wherein the six or more genes comprise genes related to platelets, platelet glycoproteins, platelet-derived immune mediators, MHC/ribosomal proteins, MHC class I molecules, Beta 2-microglobulin, ribosomal proteins, hemoglobin genes or combinations thereof and/or genes related to interferon-inducible genes, signaling molecules, kinases, RAS family members or combinations thereof.

9. The method of any one of claims 1 to 8, wherein the expression level comprises mRNA expression level, protein expression level or both mRNA expression level and protein expression level.

10. The method of any one of claims 1 to 9, wherein the expression level-comprises a mRNA expression level and is quantitated by a method selected from the group consisting of polymerase chain reaction, real time polymerase chain reaction, reverse transcriptase polymerase chain reaction, hybridization, probe hybridization and gene expression array.

11. The method of any one of claims 1 to 10, wherein the expression level is determined using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing.

12. The method of claim 3 or 8, wherein overexpression is identified with a first identifier and underexpression is identified with a second identifier.

13. The method of claim 3 or 8, wherein overexpression is identified with a first identifier and underexpression is identified with a second identifier, wherein the first identifier is a first color and the second identifier is a second color, wherein first and second identifiers are superimposed to provide a combined color.

14. A computer readable medium comprising computer-executable instructions for performing the method of claim 1.

## Patentansprüche

1. Verfahren zur Identifikation eines Subjekts mit einem Melanom, umfassend:das Bestimmen von zwei oder mehr Melanom-Expressionsvektoren, wobei die zwei oder mehr Melanom-Expressionsvektoren das Expressionsniveau von sechs oder mehr Genen mit Überexpression, Unterexpression oder einer aus diesen beiden kombinierten Expression umfassen, die ausgewählt sind aus den Modulen M1.1 und M1.2 der Tabelle 12; und das Anzeigen eines jeden der Melanom-Expressionsvektoren mittels eines separaten Identifikators.

2. Verfahren nach Anspruch 1, des Weiteren umfassend den Schritt des Detektierens eines oder mehrerer Polymorphismen in den sechs oder mehr Genen.

3. Verfahren nach Anspruch 1, des Weiteren umfassend ein Verfahren zum Anzeigen von Melanom-Transkriptom-Vektordaten, umfassend:
das Aufteilen eines oder mehrerer Gene in eines oder mehrere Module zur visuellen Anzeige eines Aggregat-Genexpressionsvektorwerts für jedes der Module; und das Anzeigen des Aggregat-Genexpressionsvektorwerts zur Überexpression, Unterexpression oder gleichmä-βigen Expression des Aggregat-Genexpressionsvektorwerts in jedem der Module.

4. Verfahren zur Identifikation eines Subjekts mit einer mit Transplantaten assoziierten Immunsuppression, umfassend: das Bestimmen von zwei oder mehr Immunsuppressions-Expressionsvektoren; und das Anzeigen jedes der Immunsuppressions-Expressionsvektoren mittels eines separaten Identifikators, wobei jeder der Immunsuppressions-Expressionsvektoren das Expressionsniveau von sechs oder mehr Genen umfasst, die aus den Modulen M1.1 und M1.2 der Tabelle 13 ausgewählt sind.

5. Verfahren nach Anspruch 4, des Weiteren umfassend den Schritt des Detektierens eines oder mehrerer Polymorphismen in dem einen oder den mehreren Immunsuppressions-Expressionsvektoren.

6. Verfahren nach Anspruch 4 oder 5, wobei der eine oder die mehreren Immunsuppressions-Expressionsvektoren von einem Leukozyten abgeleitet sind.

7. Verfahren nach einem der Ansprüche 4 bis 6, umfassend: das Aufteilen von sechs oder mehr Genen in zwei oder mehr Module zur visuellen Anzeige eines Expressionsvektors für jedes der Module in Form eines Aggregats; und das Anzeigen des Expressionsvektors zur Überexpression, Unterexpression oder gleichmäßigen Expression des Expressionsvektors in jedem der Module.

8. Verfahren nach einem der Ansprüche 1 bis 7, wobei die sechs oder mehr Gene Gene umfassen, die mit Blutplättchen, Blutplättchen-Glykoproteinen, von Blutplättchen abgeleiteten Immunmediatoren, MHC-/ribosomalen Proteinen, Molekülen der MHC-Klasse I, Beta-2-Mikroglobulin, ribosomalen Proteinen, Hämoglobingenen oder Kombinationen aus diesen in Bezug stehen, und/oder Gene, die mit durch Interferon induzierbaren Genen, Signalmolekülen, Kinasen, Vertretern der RAS-Familie oder Kombinationen aus diesen in Bezug stehen.

9. Verfahren nach einem der Ansprüche 1 bis 8, wobei das Expressionsniveau das mRNA-Expressionsniveau, das Protein-Expressionsniveau oder sowohl das mRNA-Expressionsniveau als auch das Protein-Expressionsniveau umfasst.

10. Verfahren nach einem der Ansprüche 1 bis 9, wobei das Expressionsniveau ein mRNA-Expressionsniveau umfasst und durch ein Verfahren quantifiziert ist, das ausgewählt ist aus der Gruppe bestehend aus Polymerase-Kettenreation, Echtzeit-Polymerase-Kettenreaktion, Reverse-Transkriptase-Polymerase-Kettenreaktion, Hybridisierung, Sondenhybridisierung und einem Genexpressions-Array.

11. Verfahren nach einem der Ansprüche 1 bis 10, wobei die Bestimmung des Expressionsniveaus unter Verwendung mindestens einer Technik erfolgt, die ausgewählt ist aus der Gruppe bestehend aus Polymerase-Kettenreaktion, Heteroduplex-Analyse, Einzelstrang-Konformations-Polymorphismus-Analyse, Ligase-Kettenreaktion, komparativer Genomhybridisierung, Southern-Blotting, Northern-Blottin, Western-Blotting, ELISA (*enzyme-linked immunosorbent assay*), Fluoreszenz-Resonanz-Energietransfer und Sequenzierung.

12. Verfahren nach Anspruch 3 oder 8, wobei jeweils eine Überexpression mittels eines ersten Identifikators und eine Unterexpression mittels eines zweiten Identifikators identifiziert wird.

13. Verfahren nach Anspruch 3 oder 8, wobei jeweils eine Überexpression mittels eines ersten Identifikators und eine Unterexpression mittels eines zweiten Identifikators identifiziert wird, wobei jeweils der erste Identifikator eine erste Farbe und der zweite Identifikator eine zweite Farbe aufweist, wobei der erste und der zweite Identifikator zur Bereitstellung einer kombinierten Farbe überlagert sind.

14. Computerlesbares Medium, das Anweisungen umfasst, die von einem Computer zur Durchführung des Verfahrens nach Anspruch 1 ausgeführt werden können.

## Revendications

1. Procédé permettant d'identifier un sujet souffrant d'un mélanome comprenant :
la détermination de deux vecteurs d'expression de mélanome ou plus, dans lequel les deux vecteurs d'expression de mélanome ou plus comprennent le taux d'expression de six gènes ou plus surexprimés, sous-exprimés, ou des combinaisons de ceux-ci choisis dans le module M1.1 et le module M1.2 du tableau 12 ; et
l'affichage de chacun des vecteurs d'expression de
mélanome avec un identifiant distinct.

2. Procédé selon la revendication 1, comprenant en outre l'étape consistant à détecter un ou plusieurs polymorphismes dans les six gènes ou plus.

3. Procédé selon la revendication 1 comprenant en outre un procédé d'affichage des données des vecteurs du transcriptome de mélanome comprenant :
la séparation d'un ou de plusieurs gènes en un ou plusieurs modules pour afficher visuellement une valeur de vecteur d'expression d'agrégat de gènes pour chacun des modules ; et
l'affichage de la valeur de vecteur d'expression d'agrégat de gènes pour la surexpression, la sous-expression ou une expression égale de la valeur de vecteur d'expression d'agrégat de gènes dans chaque module.

4. Procédé permettant d'identifier un sujet souffrant d'une immunosuppression associée à des transplants comprenant :
la détermination de deux vecteurs d'expression d'immunosuppression ou plus ; et
l'affichage de chacun des vecteurs d'expression d'immunosuppression avec un identifiant distinct, dans lequel chaque vecteur d'expression d'immunosuppression comprend des taux d'expression de six gènes ou plus choisis dans le module M1.1 et le module M1.2 du tableau 13.

5. Procédé selon la revendication 4, comprenant en outre l'étape consistant à détecter un ou plusieurs polymorphismes dans les un ou plusieurs vecteurs d'expression d'immunosuppression.

6. Procédé selon la revendication 4 ou 5, dans lequel les un ou plusieurs vecteurs d'expression d'immunosuppression sont dérivés d'un leucocyte.

7. Procédé selon l'une quelconque des revendications 4 à 6 comprenant :
la séparation de six gènes ou plus en deux modules ou plus pour afficher visuellement, sous la forme d'un agrégat, un vecteur d'expression pour chacun des modules ; et
l'affichage du vecteur d'expression pour la surexpression, la sous-expression ou une expression égale du vecteur d'expression dans chaque module.

8. Procédé selon l'une quelconque des revendications 1 à 7, dans lequel les six gènes ou plus comprennent des gènes associés aux plaquettes, à des glycoprotéines plaquettaires, à des médiateurs immunitaires d'origine plaquettaire, à des protéines du CMH/ribosomiales, à des molécules du CMH de classe I, à la bêta-2-microglobuline, à des protéines ribosomiales, aux gènes de l'hémoglobine ou à des combinaisons de ceux-ci et/ou des gènes associés à des gènes inductibles par l'interféron, à des molécules de signalisation, à des kinase, à des membres de la famille RAS ou à des combinaisons de ceux-ci.

9. Procédé selon l'une quelconque des revendications 1 à 8, dans lequel le taux d'expression comprend le taux d'expression d'ARNm, le taux d'expression de protéines, dans lequel à la fois le taux d'expression d'ARNm et le taux d'expression de protéines.

10. Procédé selon l'une quelconque des revendications 1 à 9, dans lequel le taux d'expression comprend un taux d'expression d'ARNm et est quantifié par un procédé choisi dans le groupe constitué par la réaction en chaîne de la polymérase, la réaction en chaîne de la polymérase en temps réel, une transcription inverse suivie d'une réaction en chaîne de la polymérase, l'hybridation, l'hybridation de sondes et une puce d'expression de gènes.

11. Procédé selon l'une quelconque des revendications 1 à 10, dans lequel le taux d'expression est déterminé en utilisant au moins une technique choisie dans le groupe constitué par la réaction en chaîne de la polymérase, l'analyse d'hétéroduplex, l'analyse de polymorphismes conformationnels d'un simple brin, la réaction en chaîne de la ligase, l'hybridation génomique comparative, l'analyse Southern blot, l'analyse Northern blot, l'analyse Western blot, la technique ELISA (enzyme-linked immunosorbent assay), le transfert d'énergie de fluorescence par résonance et le séquençage.

12. Procédé selon la revendication 3 ou 8, dans lequel la surexpression est identifiée par un premier identifiant et la sous-expression est identifiée par un second identifiant.

13. Procédé selon la revendication 3 ou 8, dans lequel la surexpression est identifiée par un premier identifiant et la sous-expression est identifiée par un second identifiant, dans lequel le premier identifiant est une première couleur et le second identifiant est une seconde couleur, dans lequel les premier et second identifiants sont superposés et fournissent une couleur combinée.

14. Support lisible par ordinateur comprenant des instructions exécutables par ordinateur pour réaliser le procédé selon la revendication 1.
